# EUROPEAN PATENT SPECIFICATION

(11) **EP 4 426 888 B1**
(45) Date of publication and mention of the grant of the patent: **11.03.2026**
(21) Application number: 22822742.7
(22) Date of filing: 04.11.2022
(51) Int. Cl.: D06M 16/00, D06M 15/03, A01N 33/12, A01N 43/16, A61L 15/28, A61L 27/34, C11D 3/48, C11D 3/22, A61L 31/16

(54) **COMPOSITIONS COMPRISING ONE CATIONIC ALPHA-1,6-GLUCAN DERIVATIVE AND ONE ALPHA-1,3-GLUCAN**
ZUSAMMENSETZUNGEN, UMFASSEND EIN KATIONISCHES ALPHA-1,6-GLUCAN-DERIVAT UND EIN ALPHA-1,3-GLUCAN
COMPOSITIONS COMPRENANT UN DÉRIVÉ CATIONIQUE D'ALPHA-1,6-GLUCAN ET UN ALPHA-1,3-GLUCAN

(30) Priority: 05.11.2021 US 202163276163 P; 22.06.2022 US 202263354501 P; 22.06.2022 US 202263354505 P
(43) Date of publication of application: 11.09.2024
(73) Proprietor: Nutrition & Biosciences USA 4, Inc., Rochester NY 14623 (US)
(72) Inventor: LI, Ziang, Wilmington, Delaware 19803 (US); RAJAN, Jana, Wilmington, Delaware 19803 (US); FRATTARELLI, David L., Wilmington, Delaware 19803 (US); ARUMUGAM, Selvanathan, Wilmington, Delaware 19803 (US)
(74) Representative: Dehns
(86) International application number: PCT/US2022/048939
(87) International publication number: WO 2023/081341

(56) References cited:
- JP-A- H11 322 555
- US-A- 4 411 891
- US-A1- 2015 232 785
- US-A1- 2021 253 977

## Description

### FIELD

The present disclosure is in the field of polysaccharides and derivatives thereof. For example, the disclosure pertains to compositions having at least a cationic alpha-glucan derivative with alpha-1,6 glycosidic linkages and an insoluble alpha-glucan with alpha-1,3 glycosidic linkages, and use of such compositions in various applications.

### BACKGROUND

Driven by a desire to find new structural polysaccharides using enzymatic syntheses or genetic engineering of microorganisms, researchers have discovered oligosaccharides and polysaccharides that are biodegradable and that can be made economically from renewably-sourced feedstocks. Further work has shown that such polysaccharides can be chemically modified (derivatized) to have additional utilities in areas such as personal care, household care, industrial care, pharmaceuticals and food. For example, ethers and esters of alpha-glucan comprising alpha-1,3 glycosidic linkages have been disclosed to have various applications (e.g., U.S. Patent Appl. Publ. Nos. 2016/0304629, 2016/0311935, 2017/0204232, 2014/0187767, 2020/0308371). Various derivatives of alpha-glucan comprising alpha-1,6 glycosidic linkages, and applications for use thereof, have also been disclosed (e.g., U.S. Patent Appl. Publ. Nos. 2018/0312781, 2018/0237816, 2018/0282385).

US2021/253977 discloses a composition comprising a polysaccharide derivative, wherein the polysaccharide derivative comprises a polysaccharide substituted with at least one sulfate group, at least one sulfonate group, at least one thiosulfate group, or a combination thereof. The polysaccharide is poly alpha-1,3-glucan, poly alpha-1,6-glucan, poly alpha-1,3-1,6-glucan, or a mixture thereof; and the polysaccharide derivative has a degree of substitution of about 0.001 to about 3. The composition can be useful as anti-deposition and/or anti-graying agents in laundry detergents, and in home, personal care, and industrial applications.

JPH11322555 discloses a composition for oral cavities contains a cationic dextran in which the cationic group introduced into the dextran molecule is a substituted or unsubstituted ammonium group.

US2015/232785 discloses compositions comprising poly alpha-1,3-1,6-glucan with a weight average degree of polymerization (DPw) of at least 1000. This glucan polymer comprises at least 30% alpha-1,3 linkages and at least 30% alpha-1,6 linkages.

US4411891A discloses cationized dextrans resulting from the introduction of a quaternary nitrogen-containing group into dextran, which is a glucan having an alpha-1,6' linkage structure, and their salts, which can be incorporated into hair or skin cosmetic bases.

Polymers having microbial control activity have been known since 1965, but their applicability in controlling microbially derived textile odor is very limited. As compared to conventional biocides, which suffer from being easily washed off fabric during laundry washing cycles, polymers have relatively low antimicrobial activity and thus require application at elevated concentrations (e.g., >1 wt%) in fabric treatment applications. However, when used at high levels, such polymers can negatively impact desired textile properties such as fabric feel, color, mechanical properties, moisture-wicking and anti-redeposition, for example. Any modification to a polymer attempting to improve its antimicrobial activity could negatively affect the polymer synthesis process and the ease of formulating it into aqueous media, both of which features are desireable by the textile industry. The backbone of a polymer, which can impact hydrophobic/hydrophilic balance, for example, can play a role in preventing excess exhaustion/precipitation of the polymer on fabric surfaces. Thus, there is an unmet need for polymers having microbial control properties that can be cost effectively produced, easily formulated into aqueous media, durably partition to fabric surfaces, and work at concentrations more comparable to those used with conventional biocides (e.g., <1 wt%). Alpha-glucan derivatives are disclosed herein to address this need. Also, while the presently disclosed alpha-glucan derivatives can partition to fabric surfaces, enhancing this partitioning would be beneficial for realizing even better antimicrobial effects (as well as other effects) on fabrics by the derivatives.

### SUMMARY

In one embodiment, the present disclosure concerns a composition comprising at least: (a) a derivative of an alpha-glucan, wherein (i) at least about 40% of the glycosidic linkages of the alpha-glucan are alpha-1,6 linkages, and (ii) the alpha-glucan has a degree of substitution (DoS) of about 0.001 to about 3.0 with at least one positively charged organic group; and (b) an insoluble alpha-glucan, wherein at least about 50% of the glycosidic linkages of the insoluble alpha-glucan are alpha-1,3 glycosidic linkages, and the weight-average degree of polymerization (DPw) of the insoluble alpha-glucan is at least 10.

In another embodiment, the present disclosure concerns a method of treating a fiber-containing material/article, the method comprising: (a) providing a composition herein comprising a positively charged alpha-glucan derivative and an insoluble alpha-glucan, and (b) contacting the composition with a fiber-containing material/article, wherein at least the alpha-glucan derivative and the insoluble alpha-glucan are adsorbed (or otherwise incorporated into/onto) to the fiber-containing material/article.

### BRIEF DESCRIPTION OF THE DRAWINGS

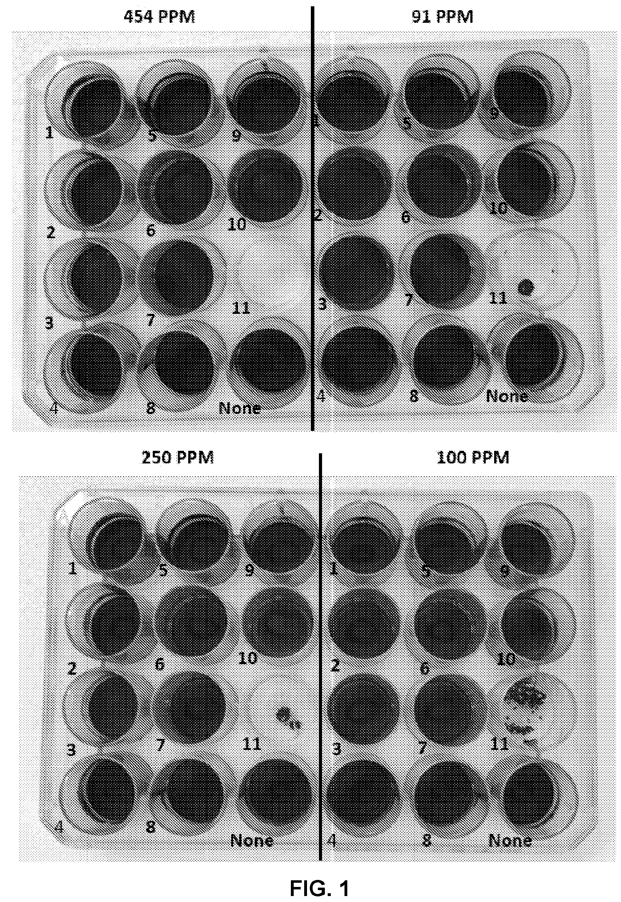

FIG. 1: Crystal violet-stained bacterial cell cultures are shown, with stain (dark) indicating the presence of biofilm. Prior to staining, the cells had been incubated for 48 hours in medium with 91, 100, 250 or 454 parts-per-million (ppm) of an alpha-glucan or alpha-glucan derivative (Sample Nos. 1-11), or no glucan (None). All of the wells were darkly stained, except for the four wells containing Sample No. 11 (Glucan Quat C12). Refer to Example 21.

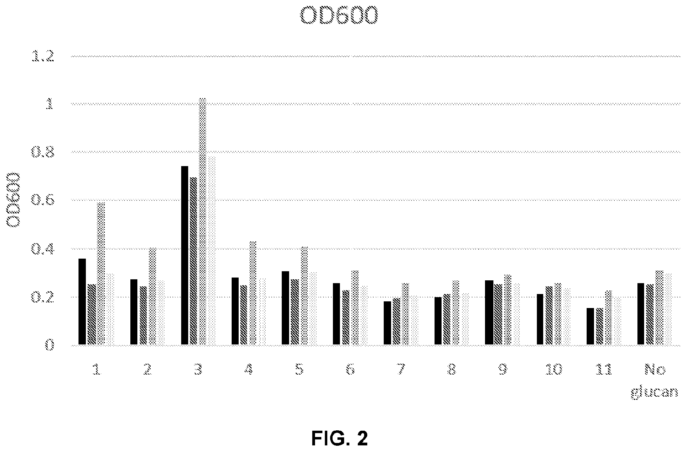

FIG. 2: The density (OD₆₀₀) of the planktonic (non-biofilm) cells of the cultures described above for FIG. 1. For each culture receiving an alpha-glucan or alpha-glucan derivative (Sample Nos. 1-11), the bars from darker to lighter indicate the 454 ppm (leftmost bar), 91 ppm, 250 ppm, and 100 ppm (right-most bar) treatments, respectively. Refer to Example 21.

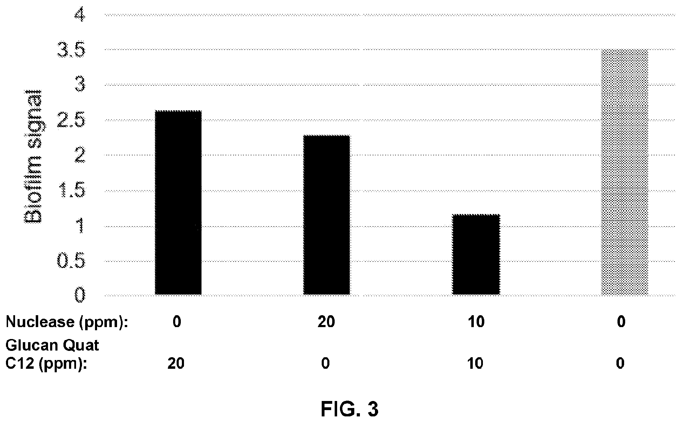

FIG. 3: The effects of nuclease alone (20 ppm), Sample No. 11 (Glucan Quat C12) alone (20 ppm), or a combination of both of these at 10 ppm each on biofilm formation was measured. Refer to Example 22.

### DETAILED DESCRIPTION

Unless otherwise disclosed, the terms "a" and "an" as used herein are intended to encompass one or more (i.e., at least one) of a referenced feature.

Where present, all ranges are inclusive and combinable, except as otherwise noted. For example, when a range of "1 to 5" (i.e., 1-5) is recited, the recited range should be construed as including ranges "1 to 4", "1 to 3", "1-2", "1-2 & 4-5", "1-3 & 5", and the like. The numerical values of the various ranges in the present disclosure, unless expressly indicated otherwise, are stated as approximations as though the minimum and maximum values within the stated ranges were both proceeded by the word "about". In this manner, slight variations above and below the stated ranges can typically be used to achieve substantially the same results as values within the ranges. Also, the disclosure of these ranges is intended as a continuous range including each and every value between the minimum and maximum values.

It is intended that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

It is to be appreciated that certain features of the present disclosure, which are, for clarity, described above and below in the context of aspects/embodiments, may also be provided in combination in a single element. Conversely, various features of the disclosure that are, for brevity, described in the context of a single aspect/embodiment, can also be provided separately or in any sub-combination.

A "glucan" herein is a type of polysaccharide that is a polymer of glucose (polyglucose). A glucan can be comprised of, for example, about, or at least about, 90%, 91%, 92%, 93%, 94%, 95%, 96%, 97%, 98%, 99%, or 100% by weight glucose monomeric units. An example of a glucan herein is alpha-glucan.

The terms "alpha-glucan", "alpha-glucan polymer" and the like are used interchangeably herein. An alpha-glucan is a polymer comprising glucose monomeric units linked together by alpha-glycosidic linkages. In typical aspects, the glycosidic linkages of an alpha-glucan herein are about, or at least about, 80%, 81%, 82%, 83%, 84%, 85%, 86%, 87%, 88%, 89%, 90%, 91%, 92%, 93%, 94%, 95%, 96%, 97%, 98%, 99%, or 100% alpha-glycosidic linkages. An example of an alpha-glucan polymer herein is alpha-1,6-glucan.

The term "saccharide" and other like terms herein refer to monosaccharides and/or disaccharides/oligosaccharides, unless otherwise noted. A "disaccharide" herein refers to a carbohydrate having two monosaccharides joined by a glycosidic linkage. An "oligosaccharide" herein can refer to a carbohydrate having 3 to 15 monosaccharides, for example, joined by glycosidic linkages. An oligosaccharide can also be referred to as an "oligomer". Monosaccharides (e.g., glucose and/or fructose) comprised within disaccharides/oligosaccharides can be referred to as "monomeric units", "monosaccharide units", or other like terms.

The terms "alpha-1,6-glucan", "poly alpha-1,6-glucan", "alpha-1,6-glucan polymer", "dextran", and the like herein refer to a water-soluble alpha-glucan comprising glucose monomeric units linked together by glycosidic linkages, wherein at least about 40% of the glycosidic linkages are alpha-1,6. Alpha-1,6-glucan in some aspects comprises about, or at least about, 90%, 95%, or 100% alpha-1,6 glycosidic linkages. Other linkages that can be present in alpha-1,6-glucan include alpha-1,2, alpha-1,3, and/or alpha-1,4 linkages.

The terms "alpha-1,3-glucan", "poly alpha-1,3-glucan", "alpha-1,3-glucan polymer" and the like are used interchangeably herein. Alpha-1,3-glucan is an alpha-glucan comprising glucose monomeric units linked together by glycosidic linkages, wherein at least about 50% of the glycosidic linkages are alpha-1,3. Alpha-1,3-glucan in some aspects comprises about, or at least about, 90%, 95%, or 100% alpha-1,3 glycosidic linkages. Most or all of the other linkages, if present, in alpha-1,3-glucan herein typically are alpha-1,6, though some linkages may also be alpha-1,2 and/or alpha-1,4. Alpha-1,3-glucan herein is typically water-insoluble.

An "alpha-1,2 branch" (and like terms) as referred to herein typically comprises a glucose that is alpha-1,2-linked to a dextran backbone; thus, an alpha-1,2 branch herein can also be referred to as an alpha-1,2,6 linkage. An alpha-1,2 branch herein typically has one glucose group (can optionally be referred to as a pendant glucose).

An "alpha-1,3 branch" (and like terms) as referred to herein typically comprises a glucose that is alpha-1,3-linked to a dextran backbone; thus, an alpha-1,3 branch herein can also be referred to as an alpha-1,3,6 linkage. An alpha-1,3 branch herein typically has one glucose group (can optionally be referred to as a pendant glucose).

The percent branching in an alpha-glucan herein refers to that percentage of all the linkages in the alpha-glucan that represent branch points. For example, the percent of alpha-1,2 branching in an alpha-glucan herein refers to that percentage of all the linkages in the glucan that represent alpha-1,2 branch points. Except as otherwise noted, linkage percentages disclosed herein are based on the total linkages of an alpha-glucan, or the portion of an alpha-glucan for which a disclosure specifically regards.

The terms "linkage", "glycosidic linkage", "glycosidic bond" and the like refer to the covalent bonds connecting the sugar monomers within a saccharide compound (oligosaccharides and/or polysaccharides). Examples of glycosidic linkages include 1,6-alpha-D-glycosidic linkages (herein also referred to as "alpha-1,6" linkages), 1,3-alpha-D-glycosidic linkages (herein also referred to as "alpha-1,3" linkages), 1,4-alpha-D-glycosidic linkages (herein also referred to as "alpha-1,4" linkages), and 1,2-alpha-D-glycosidic linkages (herein also referred to as "alpha-1,2" linkages).

The glycosidic linkage profile of an alpha-glucan or derivative thereof can be determined using any method known in the art. For example, a linkage profile can be determined using methods using nuclear magnetic resonance (NMR) spectroscopy (e.g., ¹³C NMR and/or ¹H NMR). These and other methods that can be used are disclosed in, for example, Food Carbohydrates: Chemistry, Physical Properties, and Applications (S. W. Cui, Ed., Chapter 3, S. W. Cui, Structural Analysis of Polysaccharides, Taylor & Francis Group LLC, Boca Raton, FL, 2005).

The "molecular weight" of an alpha-glucan or alpha-glucan derivative herein can be represented as weight-average molecular weight (Mw) or number-average molecular weight (Mn), the units of which are in Daltons (Da) or grams/mole. Alternatively, molecular weight can be represented as DPw (weight average degree of polymerization) or DPn (number average degree of polymerization). The molecular weight of smaller alpha-glucan polymers such as oligosaccharides can optionally be provided as "DP" (degree of polymerization), which simply refers to the number of monomers comprised within the alpha-glucan; "DP" can also characterize the molecular weight of a polymer on an individual molecule basis. Various means are known in the art for calculating these various molecular weight measurements such as with high-pressure liquid chromatography (HPLC), size exclusion chromatography (SEC), or gel permeation chromatography (GPC).

As used herein, Mw can be calculated as Mw = ΣNiMi² / ΣNiMi; where Mi is the molecular weight of an individual chain i and Ni is the number of chains of that molecular weight. Besides SEC, the Mw of a polymer can be determined by other techniques such as static light scattering, mass spectrometry, MALDI-TOF (matrix-assisted laser desorption/ionization time-of-flight), small angle X-ray or neutron scattering, or ultracentrifugation. As used herein, Mn can be calculated as Mn = ΣNiMi / ΣNi where Mi is the molecular weight of a chain i and Ni is the number of chains of that molecular weight. Besides SEC, the Mn of a polymer can be determined by various colligative property methods such as vapor pressure osmometry, end-group determination by spectroscopic methods such as proton NMR, proton FTIR, or UV-Vis. As used herein, DPn and DPw can be calculated from Mw and Mn, respectively, by dividing them by molar mass of the one monomer unit M₁. In the case of unsubstituted glucan polymer, M₁ = 162. In the case of a substituted (derivatized) glucan polymer, M₁ = 162 + M_{f} x DoS, where M_{f} is molar mass of the substituting group, and DoS is degree of substitution (average number of substituted groups per one glucose unit of the glucan polymer).

An "alpha-glucan derivative" (and like terms) herein typically refers to an alpha-glucan that has been substituted with at least one type of organic group. The degree of substitution (DoS) of an alpha-glucan derivative can be up to about 3.0 (e.g., about 0.001 to about 3.0) in some aspects. An organic group herein that is an ether group is linked to an alpha-glucan derivative via ether linkage. A precursor of an alpha-glucan derivative herein typically refers to the non-derivatized alpha-glucan used to make the derivative (can also be referred to as the alpha-glucan portion of the derivative). An organic group herein typically is positively charged (cationic); generally, such charge can be as it exists when the organic group is in an aqueous composition herein, further taking into account the pH of the aqueous composition (in some aspects, the pH can be 4-10, 5-9, 6-8, or any pH as disclosed herein).

The term "degree of substitution" (DoS, or DS) as used herein refers to the average number of hydroxyl groups that are substituted with one or more types of organic group (e.g., via an ether linkage) in each monomeric unit of an alpha-glucan derivative. The DoS of an alpha-glucan derivative herein can be stated with reference to the DoS of a specific substituent, or the overall DoS, which is the sum of the DoS values of different substituent types (e.g., if a mixed ether). Unless otherwise disclosed, when DoS is not stated with reference to a specific substituent type, the overall DoS is meant.

Terms used herein regarding "ethers" (e.g., alpha-glucan ether derivative) can be as disclosed, for example, in U.S. Patent Appl. Publ. Nos. 2016/0311935, 2018/0237816, or 2020/0002646, or Int. Pat. Appl. Publ. No. WO2021/257786 (Int. Pat. Appl. No. PCT/US2021/37756). The terms "alpha-glucan ether derivative", "alpha-glucan ether compound", "alpha-glucan ether", and the like are used interchangeably herein. An alpha-glucan ether derivative herein is alpha-glucan that has been etherified with one or more organic groups (e.g., charged organic group such as cationic group) such that the derivative has a DoS with one or more organic groups of up to about 3.0. An alpha-glucan ether derivative is termed an "ether" herein by virtue of comprising the substructure -C_{G}-O-C-, where "-C_{G}-" represents a carbon atom of a monomeric unit (typically glucose) of the alpha-glucan ether derivative (where such carbon atom was bonded to a hydroxyl group [-OH] in the alpha-glucan precursor of the ether), and where "-C-" is a carbon atom of an organic group.

An organic group can refer to a "positively charged organic group". A positively charged organic group as used herein refers to one or more carbons (e.g., "carbon chain") that has one or more hydrogens substituted with another atom or functional group (i.e., a "substituted alkyl group"), where one or more of the substitutions is with a positively charged group. Where a positively charged organic group has a substitution in addition to a substitution with a positively charged group, such additional substitution may be with one or more hydroxyl groups, oxygen atoms (thereby forming an aldehyde or ketone group), alkyl groups, and/or additional positively charged groups. A positively charged organic group has a net positive charge since it comprises one or more positively charged groups. The terms "positively charged group", "positively charged ionic group", "cationic group" and the like are used interchangeably herein. A positively charged group comprises a cation (a positively charged ion). Examples of positively charged groups include substituted ammonium groups, carbocation groups and acyl cation groups.

The terms "substituted ammonium", "substituted ammonium group", "substituted ammonium ion", "substituted ammonium cation" and the like are used interchangeably herein. A substituted ammonium group herein comprises Structure I: 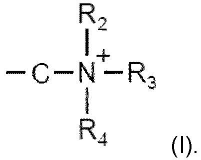 R₂, R₃ and R₄ in Structure I each independently represent a hydrogen atom or an alkyl, aryl, cycloalkyl, aralkyl, or alkaryl group. The positioning of R₂, R₃ and R₄ in Structure I is generally of no particular importance and not intended to invoke any particular stereochemistry. The carbon atom (C) in Structure I is part of one or more carbons (e.g., "carbon chain") of the positively charged organic group. The carbon atom is either directly linked (e.g., ether-linked) to a glucose monomeric unit of an alpha-glucan herein, or is part of a chain of two or more carbon atoms that is linked (e.g., ether-linked) to the glucose monomeric unit. The carbon atom in Structure I can be -CH₂-, -CH- (where an H is substituted with another group such as a hydroxy group), or -C- (where both H's are substituted).

A substituted ammonium group herein can be a "tertiary ammonium group", or "quaternary ammonium" group, depending on the composition of R₂, R₃ and R₄ in Structure I. A tertiary ammonium group herein refers to Structure I in which R₂ is a hydrogen atom and each of R₃ and R₄ is independently an alkyl, aryl, cycloalkyl, aralkyl, or alkaryl group. Assignment here of R₂, R₃ and R₄ is completely arbitrary. A quaternary ammonium group herein refers to Structure I in which each of R₂, R₃ and R₄ is independently an alkyl, aryl, cycloalkyl, aralkyl, or alkaryl group (i.e., none of R₂, R₃ and R₄ is a hydrogen atom). It would be understood that a fourth member (i.e., R₁) implied by the above nomenclature is the one or more carbons (e.g., chain) of the positively charged organic group that is linked (e.g., ether-linked) to a glucose monomeric unit of the alpha-glucan.

Examples of tertiary and quaternary ammonium alpha-glucan derivatives herein comprise a hydroxypropyl group that links the ammonium group to the alpha-glucan. The positively charged organic group of such a derivative compound can be represented as Structure II: 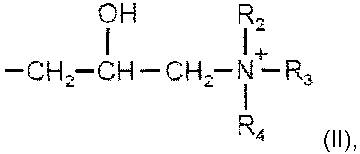 where each of R₂, R₃ and R₄ is as described above for either a tertiary or quaternary ammonium group.

The terms "aqueous liquid", "aqueous fluid", "aqueous conditions", "aqueous setting", "aqueous system" and the like as used herein can refer to water or an aqueous solution. An "aqueous solution" herein can comprise one or more dissolved salts, where the maximal total salt concentration can be about 3.5 wt% in some embodiments. Although aqueous liquids herein typically comprise water as the only solvent in the liquid, an aqueous liquid can optionally comprise one or more other solvents (e.g., polar organic solvent) that are miscible in water. Thus, an aqueous solution can comprise a solvent having at least about 10 wt% water.

An "aqueous composition" herein has a liquid component that comprises about, or at least about, 10, 20, 30, 40, 50, 60, 70, 80, 90, 95, 99, or 100 wt% water, for example. Examples of aqueous compositions include mixtures, solutions, dispersions (e.g., suspensions, colloidal dispersions) and emulsions, for example. In some embodiments, the pH of an aqueous composition is between ~2 and ~11 (e.g., between ~4 and ~9).

As used herein, the term "colloidal dispersion" refers to a heterogeneous system having a dispersed phase and a dispersion medium, i.e., microscopically dispersed insoluble particles are suspended throughout another substance (e.g., an aqueous composition such as water or aqueous solution). An example of a colloidal dispersion herein is a hydrocolloid. The terms "dispersant" and "dispersion agent" are used interchangeably herein to refer to a material that promotes the formation and/or stabilization of a dispersion. "Dispersing" herein refers to the act of preparing a dispersion of a material in an aqueous liquid. As used herein, the term "latex" (and like terms) refers to a dispersion of one or more types of polymer particles in water or aqueous solution. In some aspects, a latex is an emulsion that comprises dispersed particles. An "emulsion" herein is a dispersion of minute droplets of one liquid in another liquid in which the droplets are not soluble or miscible (e.g., a non-polar substance such as oil or other organic liquid such as an alkane, in a polar liquid such as water or aqueous solution).

An alpha-glucan derivative herein (e.g., ether), or a non-derivatized alpha-glucan, that is "soluble", "aqueous-soluble", or "water-soluble" (and like terms) dissolves (or appreciably dissolves) in water or other aqueous conditions, optionally where the aqueous conditions are further characterized to have a pH of 4-9 (e.g., pH 6-8) and/or temperature of about 1 to 130 °C (e.g., 20-25 °C). In contrast, an alpha-glucan derivative, or a non-derivatized alpha-glucan, that is "insoluble", "aqueous-insoluble", or "water-insoluble" (and like terms) does not dissolve under these conditions. Alpha-glucan derivatives herein (e.g., dextran derivatives such as dextran ethers) typically are aqueous-soluble. Alpha-1,3-glucan herein (non-derivatized) typically is aqueous-insoluble.

The term "viscosity" as used herein refers to the measure of the extent to which a fluid (aqueous or non-aqueous) resists a force tending to cause it to flow. Various units of viscosity that can be used herein include centipoise (cP, cps) and Pascal-second (Pa·s), for example. A centipoise is one one-hundredth of a poise; one poise is equal to 0.100 kg·m⁻¹·s⁻¹. Viscosity can be reported as "intrinsic viscosity" (IV, η, units of dL/g) in some aspects; this term refers to a measure of the contribution of a glucan polymer to the viscosity of a liquid (e.g., solution) comprising the glucan polymer. IV measurements herein can be obtained, for example, using any suitable method such as disclosed in U.S. Pat. Appl. Publ. Nos. 2017/0002335, 2017/0002336, or 2018/0340199, or Weaver et al. (J. Appl. Polym. Sci. 35:1631-1637) or Chun and Park (Macromol. Chem. Phys. 195:701-711). IV can be measured, in part, by dissolving glucan polymer (optionally dissolved at about 100 °C for at least 2, 4, or 8 hours) in DMSO with about 0.9 to 2.5 wt% (e.g., 1, 2, 1-2 wt%) LiCl, for example. IV herein can optionally be used as a relative measure of molecular weight.

A substance herein that is "antimicrobial" or has "antimicrobial activity" or "microbial control activity" (or like terminology) can kill at least one type of microbe or stop/prevent/inhibit/reduce its growth and/or proliferation. A "microbe", "microorganism" and the like herein can refer to one more bacteria, fungi (e.g., yeast), protists (e.g., algae), or viruses, for example. With the exception of viruses, all these types of microbes can optionally be referred to in terms of one or more microbial cells.

The terms "biofilm", "surface-attached community of microbes" and the like herein refer to a collective/assemblage/population of one or more types of microbial cells (e.g., bacteria) associated with a surface. The cells in a biofilm typically are comprised within a matrix/scaffold of protein and extracellular polymeric substance(s) (EPS) such as polysaccharide material. A biofilm matrix can also comprise, in some aspects, noncellular materials such as mineral crystals, corrosion particles, clay or silt particles, and/or other components. Biofilms typically adhere to surfaces submerged in, or subjected to, aqueous conditions. Biofilms have been described, for example, by Davey and O'Toole (2000, Microbiol. Mol. Biol. Rev. 64:847-867), Donlan (2002, Emerg. Infect. Dis. 8:881-890), Satpathy et al. (2016, Biocatal. Agric. Biotechnol. 7:56-66), Beech and Cheung (1995, Int. Biodeter. Biodegr. 35:59-72), US2018/0187175, US2020/0308592, or US20220306968 (WO2020/247582).

The term "planktonic cells" and like terms herein refer to microbial cells (e.g., bacteria) floating as single cells in a liquid medium. As opposed to biofilm cells, planktonic cells typically live freely and are not associated with other cells in a matrix. A single type of bacteria can exist either in a planktonic or biofilm state, depending on environmental cues and/or gene expression, for example.

The terms "fiber", "fibers" and the like herein can refer to staple fibers (staple length fibers) or continuous fibers, in some aspects. Fibers herein can comprise alpha-1,3-glucan, natural fiber (e.g., cellulose, cotton, wool, silk), or synthetic fiber (e.g., polyester), or any other type of material disclosed herein that can form a fiber. Fibers can be in a fiber-containing material/article/composition, for example, such as a woven or non-woven product.

The term "woven product" and like terms herein refer to a product formed by weaving, braiding, interlacing, or otherwise intertwining threads or fibers in an organized, consistent, and/or repeating manner.

The terms "non-woven", "non-woven product", "non-woven web" and the like herein refer to a web of individual fibers or filaments that are interlaid, typically in a random or unidentifiable manner. This contrasts with a knitted or woven fabric, which has an identifiable network of fibers or filaments. In some aspects, a non-woven product comprises a non-woven web that is bound or attached to another material such as a substrate or backing. A non-woven in some aspects can further contain a binder or adhesive (strengthening agent) that binds adjacent non-woven fibers together. A non-woven binder or adhesive agent can be applied to the non-woven in the form of a dispersion/latex, solution, or solid, for example, and then the treated non-woven is typically dried.

The terms "fabric", "textile", "cloth" and the like are used interchangeably herein to refer to a woven material having a network of natural and/or artificial fibers. Such fibers can be in the form of thread or yarn, for example. However, in some aspects, a fabric can comprise non-woven fibers.

The term "household care product" and like terms typically refer to products, goods and services relating to the treatment, cleaning, caring and/or conditioning of a home and its contents. The foregoing include, for example, chemicals, compositions, products, or combinations thereof having application in such care.

A "fabric care composition" and like terms refer to any composition suitable for treating fabric in some manner. Examples of such a composition include laundry detergents and fabric softeners, which are examples of laundry care compositions.

A "detergent composition" herein typically comprises at least a surfactant (detergent compound) and/or a builder. A "surfactant" herein refers to a substance that tends to reduce the surface tension of a liquid in which the substance is dissolved. A surfactant may act as a detergent, wetting agent, emulsifier, foaming agent, and/or dispersant, for example.

The terms "heavy duty detergent", "all-purpose detergent" and the like are used interchangeably herein to refer to a detergent useful for regular washing of white and colored textiles at any temperature. The terms "low duty detergent", "fine fabric detergent" and the like are used interchangeably herein to refer to a detergent useful for the care of delicate fabrics such as viscose, wool, silk, microfiber or other fabric requiring special care. "Special care" can include conditions of using excess water, low agitation, and/or no bleach, for example.

The terms "fabric softener", "fabric conditioner" and the like herein refer to compositions, such as in liquid or solid form, that deposit lubricants and/or other surface-modifying ingredients onto fabric to, for example, help maintain softness of the fabric and/or provide other beneficial features to fabric (e.g., lubricity, anti-static, anti-cling, and/or anti-wrinkling). A fabric softener herein typically is applied to fabric following fabric washing with a laundry detergent, usually while rinsing the fabric.

The term "personal care product" and like terms typically refer to products, goods and services relating to the treatment, cleaning, cleansing, caring or conditioning of a person. The foregoing include, for example, chemicals, compositions, products, or combinations thereof having application in such care.

An "oral care composition" herein is any composition suitable for treating a soft or hard surface in the oral cavity such as dental (teeth) and/or gum surfaces.

The term "medical product" and like terms typically refer to products, goods and services relating to the diagnosis, treatment, and/or care of patients.

The term "industrial product" and like terms typically refer to products, goods and services used in industrial and/or institutional settings, but typically not by individual consumers.

The terms "sequence identity", "identity" and the like as used herein with respect to a polypeptide amino acid sequence (e.g., that of a glucosyltransferase) are as defined and determined in U.S. Patent Appl. Publ. No. 2017/0002336.

Various polypeptide amino acid sequences are disclosed herein as features of certain embodiments. Variants of these sequences that are at least about 70-85%, 85-90%, or 90%-95% identical to the sequences disclosed herein can be used or referenced. Alternatively, a variant amino acid sequence can have at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 99.5% identity with a sequence disclosed herein. The variant amino acid sequence has the same function/activity of the disclosed sequence, or at least about 80%, 81%, 82%, 83%, 84%, 85%, 86%, 87%, 88%, 89%, 90%, 91%, 92%, 93%, 94%, 95%, 96%, 97%, 98%, or 99% of the function/activity of the disclosed sequence.

A composition herein that is "dry" or "dried" typically has less than 6, 5, 4, 3, 2, 1, 0.5, or 0.1 wt% water comprised therein.

The terms "percent by volume", "volume percent", "vol %", "v/v %" and the like are used interchangeably herein. The percent by volume of a solute in a solution can be determined using the formula: [(volume of solute)/(volume of solution)] x 100%.

The terms "percent by weight", "weight percentage (wt%)", "weight-weight percentage (% w/w)" and the like are used interchangeably herein. Percent by weight refers to the percentage of a material on a mass basis as it is comprised in a composition, mixture, or solution.

The terms "weight/volume percent", "w/v%" and the like are used interchangeably herein. Weight/volume percent can be calculated as: ((mass [g] of material)/(total volume [mL] of the material plus the liquid in which the material is placed)) x 100%. The material can be insoluble in the liquid (i.e., be a solid phase in a liquid phase, such as with a dispersion), or soluble in the liquid (i.e., be a solute dissolved in the liquid).

The term "isolated" means a substance (or process) in a form or environment that does not occur in nature. A non-limiting example of an isolated substance includes any glucan derivative disclosed herein. It is believed that the embodiments disclosed herein are synthetic/man-made (could not have been made or practiced except for human intervention/involvement), and/or have properties that are not naturally occurring.

The term "increased" as used herein can refer to a quantity or activity that is at least about 1%, 2%, 3%, 4%, 5%, 6%, 7%, 8%, 9%, 10%, 11%, 12%, 13%, 14%, 15%, 16%, 17%, 18%, 19%, 20%, 50%, 100%, or 200% more than the quantity or activity for which the increased quantity or activity is being compared. The terms "increased", "elevated", "enhanced", "greater than", "improved" and the like are used interchangeably herein.

Some aspects of the present disclosure concern a composition that comprises at least:
(a) a derivative of an alpha-glucan (i.e., an alpha-glucan derivative), wherein
   (i) at least about 40% of the glycosidic linkages of the alpha-glucan are alpha-1,6 linkages, and
   (ii) the alpha-glucan has a degree of substitution (DoS) of about 0.001 to about 3.0 with at least one positively charged organic group; and
(b) an insoluble alpha-glucan, wherein at least about 50% of the glycosidic linkages of the insoluble alpha-glucan are alpha-1,3 glycosidic linkages, and the weight-average degree of polymerization (DPw) of the insoluble alpha-glucan is at least 10 (i.e., alpha-1,3-glucan herein).
An alpha-glucan derivative (i.e., dextran derivative) can be any as presently disclosed, such as an alpha-glucan ether (i.e., dextran ether derivative). Such a composition in some aspects, by virtue of the presence of the insoluble alpha-glucan, can allow for enhanced deposition of the alpha-glucan derivative onto/into a fiber-containing material/article. Thus, an insoluble alpha-glucan can optionally be characterized as a deposition aid herein with respect to an alpha-glucan derivative.

Some aspects of the present disclosure concern a composition that comprises an ether derivative of an alpha-glucan (i.e., an alpha-glucan ether), wherein
(i) at least about 40% of the glycosidic linkages of the alpha-glucan are alpha-1,6 linkages,
(ii) the alpha-glucan has a weight-average molecular weight (Mw) of about 10 kDa to about 2000 kDa, and
(iii) the alpha-glucan has a degree of substitution (DoS) of about 0.001 to about 1.0 with at least one positively charged organic group that is ether-linked to the alpha-glucan, wherein the positively charged organic group comprises a C₄ to C₂₀ alkyl group or C₄ to C₂₀ alkylene group;
wherein the ether derivative has antimicrobial activity. According to the invention, the insoluble alpha-glucan as described herein is present in compositions of the invention.
Cationic alpha-glucan ether derivatives/compounds (i.e., dextran ether derivatives) are thus disclosed that have antimicrobial activity.

In some aspects, an alpha-glucan ether comprises about, or at least about, 40%, 50%, 60%, 70%, 80%, 85%, 90%, 91%, 92%, 93%, 94%, 95%, 96%, 97%, 98%, 99%, 99.5%, or 100% alpha-1,6 glycosidic linkages (i.e., the ether is an alpha-1,6-glucan ether, or dextran ether). In some aspects, a substantially linear dextran ether can comprise 5%, 4%, 3%, 2%, 1%, 0.5% or less glycosidic branches (a linear dextran ether has 100% alpha-1,6 linkages). If present, glycosidic branches from a dextran ether are typically short, being one (pendant), two, or three glucose monomers in length. In some aspects, a dextran ether can comprise about, or less than about, 50%, 40%, 30%, 20%, 15%, 10%, 9%, 8%, 7%, 6%, 5%, 4%, 3%, 2%, 1%, 0.5%, or 0% alpha-1,4, alpha-1,3 and/or alpha-1,2 glycosidic linkages. Typically, such linkages exist entirely, or almost entirely, as branch points from alpha-1,6-glucan.

The dextran portion of a dextran ether derivative herein can have alpha-1,2, alpha-1,3, and/or alpha-1,4 branches, for example. In some aspects, about, at least about, or less than about, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 30%, 35%, 40%, 45%, 50%, 2-30%, 2-25%, 2-20%, 2-15%, 2-10%, 3-25%, 3-20%, 3-15%, 3-10%, 5-30%, 5-25%, 5-20%, 5-15%, 5-10%, 7-13%, 8-12%, 9-11%, 10-30%, 10-25%, 10-22%, 10-20%, 10-15%, 12-20%, 12-18%, 14-20%, 14-18%, 15-30%, 15-25%, 15-20%, 15-18%, 15-17%, 17-23%, 18-22%, 19-21%, 35-45%, 37-43%, 38-42%, or 39-41% of all the glycosidic linkages of a branched dextran ether are alpha-1,2, alpha-1,3, and/or alpha-1,4 glycosidic branch linkages. Such branches typically are mostly (>90% or >95%), or all (100%), a single glucose monomer in length. In some aspects, dextran with alpha-1,2-branching can be produced enzymatically according to the procedures in U.S. Patent Appl. Publ. Nos. 2017/0218093 or 2018/0282385 where, for example, an alpha-1,2-branching enzyme such as GTFJ18T1 or GTF9905 can be added during or after the production of the dextran. In some aspects, any other enzyme known to produce alpha-1,2-branching can be used. Dextran with alpha-1,3-branching can be prepared, for example, as disclosed in Vuillemin et al. (2016, J. Biol Chem. 291:7687-7702) or Int. Patent Appl. Publ. No. WO2021/007264.

The dextran portion of a dextran ether derivative herein can have a weight-average molecular weight (Mw) of about, at least about, or less than about, 10, 20, 30, 40, 50, 60, 70, 80, 90, 100, 150, 200, 250, 300, 400, 500, 600, 700, 800, 900, 1000, 1250, 1500, 1750, 2000, 10-500, 10-400, 10-300, 10-200, 20-500, 50-500, 50-400, 50-300, 50-200, 100-500, 100-400, 100-300, 100-200, 150-250, 175-225, 10-70, 20-60, or 30-50 kDa, for example. Yet, in some aspects, the dextran portion of a dextran ether derivative herein can have a DP or DPw of about, at least about, or less than about, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 85, 90, 95, 100, 105, 110, 150, 200, 250, 300, 400, 500, 1000, 1500, 2000, 2500, 3000, 4000, 5000, 6000, 8-20, 8-30, 8-100, 8-500, 3-4, 3-5, 3-6, 3-7, 3-8, 4-5, 4-6, 4-7, 4-8, 5-6, 5-7, 5-8, 6-7, 6-8, 7-8, 90-120, 95-120, 100-120, 105-120, 110-120, 115-120, 90-115, 95-115, 100-115, 105-115, 110-115, 90-110, 95-110, 100-110, 105-110, 90-105, 95-105, 100-105, 90-100, 95-100, 90-95, 85-95, 85-90, 5-100, 5-250, 5-500, 5-1000, 5-1500, 5-2000, 5-2500, 5-3000, 5-4000, 5-5000, 5-6000, 10-100, 10-250, 10-500, 10-1000, 10-1500, 10-2000, 10-2500, 10-3000, 10-4000, 10-5000, 10-6000, 25-100, 25-250, 25-500, 25-1000, 25-1500, 25-2000, 25-2500, 25-3000, 25-4000, 25-5000, 25-6000, 50-100, 50-250, 50-500, 50-1000, 50-1500, 50-2000, 50-2500, 50-3000, 50-4000, 50-5000, 50-6000, 100-100, 100-250, 100-400, 100-500, 100-1000, 100-1500, 100-2000, 100-2500, 100-3000, 100-4000, 100-5000, 100-6000, 250-500, 250-1000, 250-1500, 250-2000, 250-2500, 250-3000, 250-4000, 250-5000, 250-6000, 300-2800, 300-3000, 350-2800, 350-3000, 500-1000, 500-1500, 500-2000, 500-2500, 500-2800, 500-3000, 500-4000, 500-5000, 500-6000, 600-1550, 600-1850, 600-2000, 600-2500, 600-3000, 750-1000, 750-1250, 750-1500, 750-2000, 750-2500, 750-3000, 750-4000, 750-5000, 750-6000, 900-1250, 900-1500, 900-2000, 1000-1250, 1000-1400, 1000-1500, 1000-2000, 1000-2500, 1000-3000, 1000-4000, 1000-5000, 1000-6000, or 1100-1300, for example. The molecular weight of the dextran portion of a dextran ether derivative in some aspects can be about, at least about, or less than about, 0.1, 0.125, 0.15, 0.175, 0.2, 0.24, 0.25, 0.5, 0.75, 1, 2, 3, 4, 5, 6, 7, 8, 9, 10, 20, 30, 40, 50, 60, 70, 80, 90, 100, 110, 120, 130, 140, 150, 160, 170, 180, 190, 200, 0.1-0.2, 0.125-0.175, 0.13-0.17, 0.135-0.165, 0.14-0.16, 0.145-0.155, 10-80, 20-70, 30-60, 40-50, 50-200, 60-200, 70-200, 80-200, 90-200, 100-200, 110-200, 120-200, 50-180, 60-180, 70-180, 80-180, 90-180, 100-180, 110-180, 120-180, 50-160, 60-160, 70-160, 80-160, 90-160, 100-160, 110-160, 120-160, 50-140, 60-140, 70-140, 80-140, 90-140, 100-140, 110-140, 120-140, 50-120, 60-120, 70-120, 80-120, 90-120, 90-110, 100-120, 110-120, 50-110, 60-110, 70-110, 80-110, 90-110, 100-110, 50-100, 60-100, 70-100, 80-100, 90-100, or 95-105 million Daltons. The molecular weight of the dextran portion of a dextran ether derivative in some aspects can be about, at least about, or less than about, 1, 5, 7.5, 15, 10, 20, 30, 40, 50, 60, 70, 80, 90, 100, 150, 200, 250, 300, 400, 500, 600, 700, 800, 900, 1000, 1250, 1500, 1750, 2000, 1-2000, 10-500, 10-400, 10-300, 10-200, 10-100, 10-50, 20-500, 20-400, 20-300, 20-200, 20-100, 20-50, 30-500, 30-400, 30-300, 30-200, 30-100, 30-50, 40-500, 40-400, 40-300, 40-200, 40-100, 40-50, 50-500, 50-400, 50-300, 50-200, 100-500, 100-400, 100-300, 100-200, 200-500, 200-400, 200-300, 7.5-10, 7.5-15, 7.5-20, 10-30, 15-25, 40-60, 45-55, 190-210, or 290-310 kDa, for example. The molecular weight of a dextran ether herein can be calculated, for example, based on any of the foregoing dextran DPw/kDa values, further taking into account the ether's DoS and type of ether group(s); such a molecular weight can be about, at least about, or less than about, any of the above kDa values or ranges (or those calculated from an above DP/DPw value or range), for example.

The dextran portion of a dextran ether derivative herein can be as disclosed (e.g., molecular weight, linkage/branching profile, production method), for example, in U.S. Patent Appl. Publ. Nos. 2016/0122445, 2017/0218093, 2018/0282385, 2020/0165360, or 2019/0185893. In some aspects, a dextran for ether derivatization can be one produced in a suitable reaction comprising glucosyltransferase (GTF) 0768 (SEQ ID NO:1 or 2 of US2016/0122445), GTF 8117, GTF 6831, or GTF 5604 (these latter three GTF enzymes are SEQ ID NOs:30, 32 and 33, respectively, of US2018/0282385), or a GTF comprising an amino acid sequence that is at least 90%, 91%, 92%, 93%, 94%, 95%, 96%, 97%, 98%, or 99% identical to the amino acid sequence of GTF 0768, GTF 8117, GTF 6831, or GTF 5604.

In some aspects, such as those in which an insoluble alpha-glucan can be used as a deposition aid for a dextran derivative, a dextran derivative can have a dextran portion as presently disclosed for a dextran ether. For example, a dextran portion of a dextran derivative can have a molecular weight, glycosidic linkage profile, and/or branching profile as disclosed herein for a dextran ether. Such a dextran derivative can be an ether, ester, or carbamate, for example; any positively charged organic group as disclosed herein can be linked via an ether, ester or carbamate linkage to a dextran derivative in some aspects.

In some aspects, an ether derivative of an alpha-glucan of the present disclosure can have a degree of substitution (DoS) up to about 3.0 (e.g., 0.001 to 3.0) with at least one positively charged (cationic) organic group that is linked (e.g., ether-, ester-, or carbamate-linked) to the alpha-glucan. The DoS can be about, at least about, or up to about, 0.001, 0.0025, 0.005, 0.01, 0.02, 0.025, 0.03, 0.04, 0.05, 0.06, 0.07, 0.075, 0.08, 0.09, 0.1, 0.15, 0.2, 0.25, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, or 3.0 (DoS can optionally be expressed as a range between any two of these values), for example. Some examples of DoS ranges herein include 0.001-3.0, 0.001-2.5, 0.001-2.0, 0.001-1.5, 0.001-1.0, 0.001-0.5, 0.001-0.4, 0.001-0.3, 0.001-0.2, 0.001-0.175, 0.001-0.15, 0.001-0.125, 0.001-0.1, 0.01-3.0, 0.01-2.5, 0.01-2.0, 0.01-1.5, 0.01-1.0, 0.01-0.5, 0.01-0.4, 0.01-0.3, 0.01-0.2, 0.01-0.175, 0.01-0.15, 0.01-0.125, 0.01-0.1, 0.05-3.0, 0.05-2.5, 0.05-2.0, 0.05-1.5, 0.05-1.0, 0.05-0.5, 0.05-0.4, 0.05-0.3, 0.05-0.2, 0.05-0.175, 0.05-0.15, 0.05-0.125, 0.05-0.1, 0.1-3.0, 0.1-2.5, 0.1-2.0, 0.1-1.5, 0.1-1.0, 0.1-0.5, 0.1-0.4, 0.1-0.3, 0.1-0.2, 0.1-0.175, 0.1-0.15 and 0.1-0.125.

Since there are at most three hydroxyl groups in a glucose monomeric unit of an alpha-glucan, the overall DoS of an alpha-glucan derivative can be no higher than 3.0. It would be understood by those skilled in the art that, since an alpha-glucan derivative as presently disclosed has a DoS with at least one type of positively charged organic group in chemical (e.g., ether, ester, or carbamate) linkage (e.g., between about 0.001 to about 3.0), all the substituents of an alpha-glucan derivative cannot only be hydroxyl.

A derivative of an alpha-glucan of the present disclosure can be substituted with at least one positively charged organic group herein that is chemically linked (e.g., ether-, ester-, or carbamate-linked) to the alpha-glucan, such as a positively charged organic group that comprises a C₄ to C₂₀ alkyl or alkylene group. An alpha-glucan derivative as presently disclosed can be derivatized with one, two, or more different types of positively charged organic groups herein, for example; typically, there are no other types of organic groups in the alpha-glucan derivative.

In some aspects, a positively charged organic group can comprise a C₄ to C₂₀ alkyl group. A C₄ to C₂₀ alkyl group can be any one of a C₄, C₅, C₆, C₇, C₈, C₉, C₁₀, C₁₁, C₁₂, C₁₃, C₁₄, C₁₅, C₁₆, C₁₇, C₁₈, C₁₉, or C₂₀ alkyl group, for example. In some aspects, an alkyl group can be a C₁₀ to C₁₄ alkyl group, meaning that the alkyl group can be any one of a C₁₀, C₁₁, C₁₂, C₁₃, or C₁₄ alkyl group. Additional examples include an alkyl group that is a C₆ to C₁₈, C₈ to C₁₈, C₁₀ to C₁₈, C₆ to C₁₆, C₈ to C₁₆, C₁₀ to C₁₆, C₆ to C₁₄, C₈ to C₁₄, C₁₀ to C₁₄, C₆ to C₁₂, C₈ to C₁₂, or C₁₀ to C₁₂ alkyl group. By disclosing a C₁₂ alkyl group, for example, it is meant that the alkyl group is twelve carbons in length and is saturated (i.e., -CH₂CH₂CH₂CH₂CH₂CH₂CH₂CH₂CH₂CH₂CH₂CH₃); this standard meaning applies, accordingly, to other alkyl groups disclosed herein.

In some aspects, a positively charged organic group can comprise a C₄ to C₂₀ alkylene group (e.g., of any length as disclosed herein for an alkyl group). An alkylene group can comprise one, two, three, or more double-bonds, for example. An alkylene group in some aspects can comprise one or more double-bonds spanning carbons (i) 5 and 6, (ii) 6 and 7, (iii) 8 and 9, (iv) 9 and 10, (v) 11 and 12, (vi) 12 and 13, (vii) 14 and 15, and/or (viii) 15 and 16 of the alkylene group, where carbon number is counted starting from the carbon directly linked to the positively charged group (e.g., carbon-1 is linked to the nitrogen of a substituted ammonium group herein). Some combinations of double-bonds of an alkylene group include: (iv) and (vi); (iv), (vi) and (vii); and (i), (iii), (v) and (vii) (with reference to the foregoing list). While a double-bond herein of an alkylene group can be in a *cis* or *trans* orientation, it typically is in the *cis* orientation. An alkylene group can be derived (derivable) from a fatty acid (e.g., caproic acid, caprylic acid, capric acid, lauric acid, myristic acid, palmitic acid, stearic acid, arachidic acid, oleic acid, linoleic acid, arachidonic acid), or an acyl group (e.g., corresponding to any fatty acid herein) of a lipid (e.g., a mono-, di-, or triglyceride).

A positively charged group in some aspects can comprise a substituted ammonium group. Examples of substituted ammonium groups herein are tertiary and quaternary ammonium groups, such as can be represented by Structures I and II (above). In some aspects, a substituted ammonium group is a tertiary ammonium group in which, with reference to Structure I and/or II, R₂ is a hydrogen atom, R₃ is a methyl, ethyl, propyl, or butyl, and R₄ is any C₄ to C₂₀ alkyl or alkylene group as described above. In some aspects, a substituted ammonium group is a quaternary ammonium group in which, with reference to Structure I and/or II, R₂ and R₃ are each independently a methyl, ethyl, propyl, or butyl (e.g., both R₂ and R₃ are methyl, or are both ethyl), and R₄ is any C₄ to C₂₀ alkyl or alkylene group as described above (e.g., a C₁₂ alkyl). A tertiary or quaternary ammonium group in some aspects comprises Structure II, and has any of the foregoing R₂, R₃ and R₄ assignments. An example of a quaternary ammonium group herein comprises dodecyldimethylammonium (i.e., the ammonium nitrogen is linked to a C₁₂ alkyl group and two methyl groups).

One of the groups of a substituted ammonium group herein typically comprises one carbon, or a chain of carbons (e.g., up to 30), that is in linkage (e.g., ether, ester, or carbamate linkage) to an alpha-glucan (i.e., such one carbon or carbon chain links the ammonium group to a glucose monomer of the alpha-glucan derivative). A carbon chain in this context can be linear, for example. Such a carbon or carbon chain can be represented by -CH₂-, -CH₂CH₂-, -CH₂CH₂CH₂-, -CH₂(CH₂)₂CH₂-, -CH₂(CH₂)₃CH₂-, -CH₂(CH₂)₄CH₂-, -CH₂(CH₂)₅CH₂-, -CH₂(CH₂)₆CH₂-, -CH₂(CH₂)₇CH₂-, -CH₂(CH₂)₈CH₂-, -CH₂(CH₂)₉CH₂-, or -CH₂(CH₂)₁₀CH₂-, for example. In some aspects, a carbon chain in this context can be branched, such as by being substituted with one or more alkyl groups (e.g., any as disclosed above such as methyl, ethyl, propyl, or butyl). The point(s) of substitution can be anywhere along the carbon chain. Examples of branched carbon chains include -CH(CH₃)CH₂-, -CH(CH₃)CH₂CH₂-, -CH₂CH(CH₃)CH₂-, -CH(CH₂CH₃)CH₂-, -CH(CH₂CH₃)CH₂CH₂-, -CH₂CH(CH₂CH₃)CH₂-, -CH(CH₂CH₂CH₃)CH₂-, -CH(CH₂CH₂CH₃)CH₂CH₂- and -CH₂CH(CH₂CH₂CH₃)CH₂-; longer branched carbon chains can also be used, if desired. In some aspects, a chain of one or more carbons (e.g., any of the above linear or branched chains) is further substituted with one or more hydroxyl groups. Examples of hydroxy- or dihydroxy (diol)-substituted chains include -CH(OH)-, -CH(OH)CH₂-, -C(OH)₂CH₂-, -CH₂CH(OH)CH₂-, -CH(OH)CH₂CH₂-, -CH(OH)CH(OH)CH₂-, -CH₂CH₂CH(OH)CH₂-, -CH₂CH(OH)CH₂CH₂-, -CH(OH)CH₂CH₂CH₂-, -CH₂CH(OH)CH(OH)CH₂-, -CH(OH)CH(OH)CH₂CH₂- and -CH(OH)CH₂CH(OH)CH₂-. In each of the foregoing examples, the first carbon atom of the chain is linked (e.g., ether-, ester-, or carbamate-linked) to a glucose monomer of the alpha-glucan, and the last carbon atom of the chain is linked to a positively charged group (e.g., a substituted ammonium group as disclosed herein). One or more positively charged organic groups in some aspects can be dodecyldimethylammonium hydroxypropyl groups (i.e., Structure II, where R₂ is a C₁₂ alkyl group, and R₃ and R₄ are each a methyl group).

A counter ion for a positively charged organic group herein can be any suitable anion, such as an acetate, borate, bromate, bromide, carbonate, chlorate, chloride, chlorite, dihydrogen phosphate, fluoride, hydrogen carbonate, hydrogen phosphate, hydrogen sulfate, hydrogen sulfide, hydrogen sulfite, hydroxide, hypochlorite, iodate, iodide, nitrate, nitride, nitrite, oxalate, oxide, perchlorate, permanganate, phosphate, phosphide, phosphite, silicate, stannate, stannite, sulfate, sulfide, sulfite, tartrate, or thiocyanate anion.

An alpha-glucan derivative compound in some aspects can contain one type of chemically linked positively charged organic group. Examples of such a positively charged organic group are as disclosed herein. Optionally, an alpha-glucan ether, ester, or carbamate compound having a single type of chemically linked positively charge organic group can be characterized, respectively, as a monoether, monoester, or monocarbamate.

An alpha-glucan derivative compound in some aspects can contain two or more different types of chemically linked positively charge organic groups (i.e., a mixed derivative). A combination of different positively charged organic groups can individually comprise substituted ammonium groups that individually comprise at least: (i) C₁₂ and C₁₄ alkyl and/or alkylene groups; (ii) C₁₀, C₁₂, C₁₄ and C₁₆ alkyl and/or alkylene groups; (iii) C₈, C₁₀, C₁₂, C₁₄, C₁₆ and C₁₈ alkyl and/or alkylene groups; or (iv) C₆, C₈, C₁₀, C₁₂, C₁₄, C₁₆, C₁₈ and C₂₀ alkyl and/or alkylene groups, for example. The content of these different groups (any foregoing combination) in an alpha-glucan mixed derivative herein, based on DoS, can reflect the relative content of these groups as listed and represented by fatty acids in in Table A (or within ±5% thereof).

In some aspects, an alpha-glucan ether compound has one or more different types of etherified positively charged organic groups as presently disclosed, but has no other types of organic groups derivatized to the alpha-glucan (e.g., a hydrophobic group that, for example, is ether- or ester-linked to the alpha-glucan).

An alpha-glucan ether derivative in some aspects can have antimicrobial activity (e.g., anti-bacterial, anti-fungal/yeast, or anti-viral activity). Thus, a composition comprising such an ether derivative can have antimicrobial properties. An alpha-glucan ether derivative herein that is antimicrobial can kill at least one type of microbe (microbe cell) and/or stop/prevent/inhibit its growth (e.g., achieving mature cell/virus size) and/or proliferation (cell/virus division/replication). A microbe herein can be a bacterial, fungal (e.g., yeast), protozoan (e.g., algal), or enveloped viral species, for example. A microbe in some aspects can be harmful (e.g., pathogenic, food-spoiling ability) or present a nuisance (e.g., odor-causing, biofilm-forming) to humans, animals (e.g., pets, livestock, foodstock), or systems/enterprises/operations, for example.

While not being held to any particular theory, it is believed that in some aspects an alpha-glucan ether derivative can derive its antimicrobial activity, at least in part, to an ability to interact with (e.g., embed in) microbial lipid membranes (typically cell membrane or enveloped virus membrane). This interaction with a membrane of a microbe can result in permeabilization and/or destabilization of a microbial membrane, which can lead to impairment of membrane protein function and/or leakage of cell/virus content, thereby impairing cell/virus growth or division/replication. Thus, in some aspects, a composition can comprise an alpha-glucan ether derivative herein that is associated with a microbial membrane (microbial lipid membrane). Such an association can be that the alpha-glucan ether is embedded in the membrane, for instance. A microbial membrane herein typically is a lipid bilayer. A microbial membrane can be a cytoplasmic membrane, for example. With respect to bacteria, a membrane herein can be an outer cell membrane (e.g., that of a Gram-negative species) or a cytoplasmic membrane (either of a Gram-positive or -negative species). A C₄-C₂₀ alkyl group herein of an alpha-glucan ether is embedded in a membrane in some aspects. A membrane herein can be that of any microbe disclosed herein, for example. A microbial membrane in which an alpha-glucan ether is embedded can be (i) that of a still-living microbe (e.g., a metabolically active cell, such as one that is able to produce or metabolize ATP) (e.g., but which is now impaired in its growth and/or replication potential), (ii) that of a killed microbe that is generally still intact, or (iii) free from an intact cell/virus (e.g., a membrane fragment from a killed cell/virus), for example.

Yet, in some aspects, it is believed that an alpha-glucan ether derivative herein might derive its antimicrobial activity, at least in part, to an ability to interact with (i) a microbial cell wall, such as that of a bacteria (e.g., Gram-negative bacteria), fungus/yeast, or protist/algae, and/or (ii) a bacterial glycocalyx. Thus, in some aspects, a composition can comprise an alpha-glucan ether derivative herein that is associated with a microbial cell wall or bacterial glycocalyx. A cell wall herein can be that of any microbe disclosed herein, for example, that has a cell wall. A glycocalyx herein can be that of any bacteria disclosed herein, for example, that has a glycocalyx.

A microbe in some aspects is a bacteria, such as a Gram-negative or Gram-positive bacteria. A bacteria can be spherical (coccus), rod (bacillus), or spiral (spirochete) shaped, for example. A bacteria can be aerobic or anaerobic, for example, and/or spore-forming. A bacteria in some aspects is a species of *Acinetobacter* (e.g., *A. baumannii*)*, Actinomyces* (e.g., *A. israelii*)*, Aeromicrobium, Bacillus* (e.g., *B. anthracis, B. cereus), Bacteroides* (e.g., *B. fragilis), Bartonella* (e.g., B. *henselae, B*. *quintana), Bordetella* (e.g., *B. pertussis), Borrelia* (e.g., *B. burgdorferi, B. garinii, B. afzelii, B. recurrentis), Brevundimonas* (e.g., *B*. *vesicularis, B. diminuta), Burkholderia* (e.g., *B*. *cepacia, B. pseudomallei*)*, Brucella* (e.g., *B*. *abortus, B. canis, B. melitensis, B. suis), Campylobacter* (e.g., *C. jejuni*)*, Chlamydia* (e.g., *C. pneumoniae, C. trachomatis), Clostridium* (e.g., *C. botulinum, C. difficile, C. perfringens, C. tetani*)*, Corynebacterium* (e.g., *C. diphtheriae*), *Desulfatitalea* (e.g., *D. tepidiphila*), *Desulfobacter* (e.g., *D. postgatei*)*, Desulfovibrio* (e.g., *D. vulgaris*), *Desulfotomaculum* (e.g., *D. australicum), Desulfomicrobium* (e.g., *D. escambiense*), *Desulfobulbus, Desulfobacula, Desulfotignum* (e.g., *D. toluenicum*), *Desulfobacterium* (e.g., *D. cetonicum*), *Desulfococcus* (e.g., *D. multivorans*), *Desulfosporosinus* (e.g., *D. lacus*), *Desulfotalea* (e.g., *D*. *psychrophila*), *Enterobacter* (e.g., *E. gergoviae*), *Desulfohalobium* (e.g., *D. retbaense*), *Enterobacter, Enterococcus* (e.g., *E. faecalis, E. faecium*), *Escherichia* (e.g., *E. coli*)*, Francisella* (e.g., *F. tularensis*), *Haemophilus* (e.g., *H. influenzae*), *Helicobacter* (e.g., *H*. *pylori*)*, Klebsiella* (e.g., *K. pneumoniae*), *Lawsonia* (e.g., *L. intracellularis*), *Legionella* (e.g., *L. pneumophila*), *Leptospira* (e.g., *L. interrogans, L. santarosai, L. weilii, L. noguchii*)*, Listeria* (e.g., *L. monocytogenes*), *Microbacterium* (e.g., *M. lacticum, M. laevaniformans*), *Micrococcus* (e.g., *M. luteus*), *Mycobacterium* (e.g., *M. leprae, M. tuberculosis, M. ulcerans*), *Mycoplasma* (e.g. *M. pneumoniae*), *Neisseria* (e.g., *N. gonorrhoeae, N. meningitidis*), *Nitrospira* (e.g., *N. moscoviensis, N. marina), Propionibacterium* (e.g., *P. acnes*), *Pseudomonas* (e.g., *P. aeruginosa, P. putida, P. alcaliphila, P. fluorescens), Rickettsia* (e.g., *R. rickettsii*)*, Salmonella* (e.g., *S. typhi, S. typhimurium, S. enterica), Shigella* (e.g., S. *sonnei, S. flexneri, S. dysenteriae), Staphylococcus* (e.g., *S*. *aureus, S*. *epidermidis, S. saprophyticus), Stenotrophomonas* (e.g., *S. maltophilia), Streptococcus* (e.g., *S*. *agalactiae, S. pneumoniae, S*. *pyogenes, S. mutans, S. salivarius, S. bovis), Syntrophobacter* (e.g., *S. fumaroxidans), Thermodesulfobacterium* (e.g., *T. commune), Thermodesulfovibrio* (e.g., *T. aggregans), Thermodesulfatator* (e.g., *T. autotrophicus), Treponema* (e.g., *T. pallidum), Ureaplasma* (e.g., *U. urealyticum), Vibrio* (e.g., *V. cholerae),* or *Yersinia* (e.g., *Y. pestis, Y. enterocolitica, Y. pseudotuberculosis).* A bacteria in some aspects is a sulfate-reducing bacteria (e.g., any of the taxonomic orders *Desulfovibrionales, Desulfobacterales, Syntrophobacterales, Nitrospirales, Clostridiales, Selenomonadales, Thermodesulfobacteriales, Desulfurellales,* and/or *Thermoanaerobacterales).* Still additional examples of bacterial species herein are any of those disclosed in U.S. Patent Nos. 9192598 or 9675736, U.S. Patent Appl. Publ. Nos. 2020/0308592, 2018/0187175, or 2022/0306968 (WO2020/247582), or Davey and O'Toole (2000, Microbiol. Mol. Biol. Rev. 64:847-867), Donlan (2002, Emerg. Infect. Dis. 8:881-890), or Satpathy et al. (2016, Biocatal. Agric. Biotechnol. 7:56-66).

A microbe in some aspects is a fungus such as a yeast. A fungus or yeast in some aspects is a species of *Aspergillus* (e.g., *A. brasiliensis, A. fumigatus, A. flavus), Candida* (e.g., *C*. *albicans, C. tropicalis, C. glabrata, C*. *parapsilosis*), *Cryptococcus* (e.g., *C*. *neoformans, C. gattii*)*, Histoplasma* (e.g., *H. capsulatum), Malassezia* (e.g., *M*. *globosa), Pneumocystis* (e.g., *P. jirovecii, P. carinii*)*,* or *Stachybotrys* (e.g., *S. chartarum).*

A microbe in some aspects is an enveloped virus (i.e., a virus having a lipid bilayer as its outermost layer). An enveloped virus can be a DNA virus (e.g., Herpesvirus, Poxvirus, Hepadnavirus, Asfarvirus), RNA virus (e.g., Flavivirus, Alphavirus, Togavirus, Coronavirus such as a SARS coronavirus or a COVID-19 virus, Hepatitis D, Orthomyxovirus such as an influenza virus, Paramyxovirus, Rhabdovirus, Bunyavirus, Filovirus), or retrovirus (e.g., human immunodeficiency virus [HIV], human T-lymphotropic virus type-1 or -2), for instance.

An alpha-glucan ether derivative in some aspects can exhibit a minimum inhibitory concentration (MIC) toward a microbe such as a bacteria or yeast. An MIC can be, for instance, the lowest concentration of an alpha-glucan ether that will inhibit the visible growth (e.g., colony formation or development of broth turbidity) of a microbe, typically under otherwise suitable growth conditions (e.g., temperature, media) and incubation period (e.g., 12-15, 12-24, 18-24, or 18-30 hours). An MIC of an alpha-glucan ether derivative in some aspects can be about, or less than about, 1000, 750, 500, 400, 300, 250, 200, 100, 90, 80, 75, 60, 50, 40, 30, 25, 20, 15, 10, 10-1000, 10-500, 10-250, 10-100, 10-50, 20-1000, 20-500, 20-250, 20-100, 20-50, 40-1000, 40-500, 40-250, or 40-100 ppm.

An alpha-glucan ether derivative comprised in (e.g., adsorbed/deposited onto) a fiber-containing material/article (and further including an insoluble alpha-glucan herein) can exhibit enhanced durability in terms of maintaining its antimicrobial activity over time and/or washing/laundry cycles. For example, a fiber-containing material/article, such as one comprising a fabric, that has been treated with an alpha-glucan ether derivative herein (optionally included with an insoluble alpha-glucan herein) can retain about, or at least about, 40%, 50%, 60%, 70%, 80%, 90%, 40%-90%, 50%-90%, 60%-90%, 70%-90%, 80%-90%, 40%-80%, 50%-80%, or 60%-80% of its antimicrobial activity (i) over about, or at least about, 0.5, 1, 2, 3, 4, 5, or 6 years, and/or (ii) over about, or at least about, 10, 15, 20, 25, 30, 35, 40, 45, 50, 55, 60, 70, 80, 90, 100, 30-100, 30-90, 30-80, 30-70, 30-60, 40-100, 40-90, 40-80, 40-70, or 40-60 wash/laundry cycles, as compared to its antimicrobial activity at the time the treated fabric was produced and/or before it was ever subjected to a wash/laundry cycle. Antimicrobial activity of a fiber-containing material/article can be measured using any suitable methodology, such as any procedure disclosed in the below Examples. For example, antimicrobial activity can be measured herein by following American Society for Testing and Materials (ASTM) method E3160-18 (*Quantitative Evaluation of the Antibacterial Properties of Porous Antibacterial Treated Articles)* (e.g., Procedure 2A below; e.g., at a use level ≤5000 ppm) or ASTM method AATCC 100 *(Quantitative Evaluation of the Antibacterial Properties of Porous Antibacterial Treated Articles*) (e.g., e.g., Procedure 2B below; at a use level ≤5000 ppm). A wash/laundry cycle can be performed using any suitable methodology, such as disclosed in the below Examples. For example, a wash/laundry cycle can be performed using ASTM E3162-18 *(Measuring the Durability of Antibacterial Agents Applied to Textiles under Simulated Home Laundering Conditions*) (e.g., Procedure 3 below).

An insoluble alpha-glucan herein typically is an insoluble alpha-1,3-glucan. In compositions of the invention which contain an insoluble alpha-glucan, at least about 50% of the glycosidic linkages of the insoluble alpha-glucan are alpha-1,3 glycosidic linkages, and the weight-average degree of polymerization (DPw) of the insoluble alpha-glucan is at least 10. In some aspects, about, or at least about, 60%, 70%, 80%, 85%, 90%, 91%, 92%, 93%, 94%, 95%, 96%, 97%, 98%, 99%, 99.5%, or 100% of the glycosidic linkages of an insoluble alpha-glucan are alpha-1,3 glycosidic linkages. Typically, the glycosidic linkages that are not alpha-1,3 are mostly or entirely alpha-1,6. It should be understood that the higher the percentage of alpha-1,3 linkages present in an insoluble alpha-glucan, the greater the probability that the glucan is linear, since there are lower occurrences of certain linkages that might be part of branch points. In some aspects, insoluble alpha-glucan has no branch points or less than about 5%, 4%, 3%, 2%, or 1% branch points as a percent of the glycosidic linkages in the alpha-glucan.

Insoluble alpha-glucan for use in compositions of the invention have a DPw of at least about 10. In some aspects, the DPw, DPn, or DP of insoluble alpha-glucan can be about, less than about, or at least about, 10, 15, 20, 25, 30, 35, 36, 37, 38, 39, 40, 41, 42, 43, 44, 45, 46, 47, 48, 49, 50, 51, 52, 53, 54, 55, 56, 57, 58, 59, 60, 65, 70, 75, 80, 85, 90, 95, 100, 110, 125, 150, 200, 300, 400, 500, 600, 700, 800, 900, 1000, 1100, 1200, 1300, 1400, 1500, 1600, 1700, 1800, 1900, 2000, 2500, 3000, 3500, or 4000 (but the DPw is at least 10 in insoluble alpha-glucans in compositions of the invention). DPw, DPn, or DP can optionally be expressed as a range between any two of these values. Merely as examples, the DPw, DPn, or DP of insoluble alpha-glucan herein can be about 10-1600, 50-1600, 100-1600, 200-1600, 300-1600, 400-1600, 500-1600, 600-1600, 700-1600, 10-1250, 50-1250, 100-1250, 200-1250, 300-1250, 400-1250, 500-1250, 600-1250, 700-1250, 10-1000, 50-1000, 100-1000, 200-1000, 300-1000, 400-1000, 500-1000, 600-1000, 700-1000, 10-900, 50-900, 100-900, 200-900, 300-900, 400-900, 500-900, 600-900, 700-900, 600-800, or 600-750. Merely as further examples, the DPw, DPn, or DP of insoluble alpha-glucan herein can be about 10-100, 25-100, 35-100, 10-80, 25-80, 35-80, 10-60, 25-60, 35-60, 10-55, 25-55, 35-55, 10-50, 25-50, 35-50, 35-45, 35-40, 40-100, 40-80, 40-60, 40-55, 40-50, 45-60, 45-55, 45-50, 10-35, 20-35, 15-30, or 20-30. In some aspects, an insoluble alpha-glucan can have a high molecular weight as reflected by high intrinsic viscosity (IV); e.g., IV can be about, or at least about 6, 7, 8, 9, 10, 11, 12, 13, 14, 15, 16, 17, 18, 19, 20, 21, 22, 6-8, 6-7, 6-22, 6-20, 6-17, 6-15, 6-12, 10-22, 10-20, 10-17, 10-15, 10-12, 12-22, 12-20, 12-17, or 12-15 dL/g. For comparison purposes, note that the IV of alpha-glucan with at least 90% (e.g., about 99% or 100%) alpha-1,3 linkages and a DPw of about 800 has an IV of about 2-2.5 dL/g. IV herein can be as measured with alpha-glucan polymer dissolved in DMSO with about 0.9 to 2.5 wt% (e.g., 1, 2, 1-2 wt%) LiCl, for example.

Insoluble alpha-glucan herein can be as disclosed (e.g., molecular weight, linkage profile, and/or production method), for example, in U.S. Patent Nos. 7000000, 8871474, 10301604, or 10260053, or U.S. Patent Appl. Publ. Nos. 2019/0112456, 2019/0078062, 2019/0078063, 2018/0340199, 2018/0021238, 2018/0273731, 2017/0002335, 2015/0232819, 2015/0064748, 2020/0165360, 2020/0131281, or 2019/0185893. Insoluble alpha-glucan can be produced, for example, by an enzymatic reaction comprising at least water, sucrose and a glucosyltransferase enzyme that synthesizes the insoluble alpha-glucan. Glucosyltransferases, reaction conditions, and/or processes contemplated to be useful for producing insoluble alpha-glucan can be as disclosed in any of the foregoing references.

In some aspects, a glucosyltransferase enzyme for producing an insoluble alpha-glucan can comprise an amino acid sequence that is 100% identical to, or at least 90%, 91%, 92%, 93%, 94%, 95%, 96%, 97%, 98%, 98.5%, 99%, or 99.5% identical to, SEQ ID NO:2, 4, 6, 8, 10, 12, 14, 16, 18, 20, 26, 28, 30, 34, or 59, or amino acid residues 55-960 of SEQ ID NO:4, residues 54-957 of SEQ ID NO:65, residues 55-960 of SEQ ID NO:30, residues 55-960 of SEQ ID NO:28, or residues 55-960 of SEQ ID NO:20, and have glucosyltransferase activity; these amino acid sequences are disclosed in U.S. Patent Appl. Publ. No. 2019/0078063. It is noted that a glucosyltransferase enzyme comprising SEQ ID NO:2, 4, 8, 10, 14, 20, 26, 28, 30, 34, or amino acid residues 55-960 of SEQ ID NO:4, residues 54-957 of SEQ ID NO:65, residues 55-960 of SEQ ID NO:30, residues 55-960 of SEQ ID NO:28, or residues 55-960 of SEQ ID NO:20, can synthesize insoluble alpha-glucan comprising at least about 90% (~100%) alpha-1,3 linkages.

In some aspects, insoluble alpha-glucan can be in the form of an insoluble graft copolymer such as disclosed in Int. Patent Appl. Publ. No. WO2021247810, or U.S. Patent Appl. Publ. Nos. 2020/0165360, 2019/0185893, or 2020/0131281. A graft copolymer can comprise dextran (as backbone) and alpha-1,3-glucan (as one or more side chains), where the latter component has been grafted onto the former component; typically, this graft copolymer is produced by using dextran or alpha-1,2- and/or alpha-1,3-branched dextran as a primer for alpha-1,3-glucan synthesis by an alpha-1,3-glucan-producing glucosyltransferase as described above. Alpha-1,3-glucan side chain(s) of an alpha-glucan graft copolymer herein can be alpha-1,3-glucan as presently disclosed. The dextran backbone of an alpha-glucan graft copolymer herein can be as presently disclosed for an alpha-glucan derivative, for example; e.g., the dextran backbone can have a linkage, branching, and/or molecular weight profile as disclosed for an alpha-glucan derivative herein. The dextran backbone of an alpha-glucan graft copolymer herein can be as disclosed (e.g., molecular weight, linkage/branching profile, production method), for example, in U.S. Patent Appl. Publ. Nos. 2016/0122445, 2017/0218093, 2018/0282385, 2020/0165360, or 2019/0185893.

Insoluble alpha-glucan for use in preparing a composition of the present disclosure can be in the form of particles in some aspects. As comprised in an aqueous composition such as a dispersion, about 40-60%, 40-55%, 45-60%, 45-55%, 47-53%, 48-52%, 49-51%, or 50% by weight of such insoluble alpha-glucan particles have a diameter (i.e., D₅₀) of about, less than about, or at least about, 1, 2, 3, 4, 5, 6, 7, 8, 9, 10, 11, 12, 13, 14, 15, 16, 17, 18, 19, 20, 21, 22, 23, 24, 25, 1-25, 1-22, 1-20, 1-18, 5-25, 5-22, 5-20, 5-18, 15-22, 15-20, 15-18, 16-22, 16-20, or 16-18 microns, for example. In some aspects, about, or at least about, 60%, 65%, 70%, 75%, 60-75%, 60-70%, 60-65%, 65-75%, or 65-70% by weight of the insoluble alpha-glucan particles of an aqueous dispersion have a diameter of about, or less than about, 25, 30, 35, 40, 25-40, 30-40, 35-40, 25-35, or 30-35 micrometers. In some aspects, at least about 90%, 95%, 96%, 97%, 98%, 99%, or 100% by weight of the insoluble alpha-glucan particles of an aqueous dispersion have a diameter of less than about 30, 35, 40, 45, or 50 micrometers. Particle size herein can be as measured, for example, by a process comprising light scattering or electrical impedance change (e.g., using a Coulter Counter), as described in any of U.S. Patent Nos. 6091492, 6741350 and 9297737.

Insoluble alpha-glucan herein typically does not have any chemical derivatization (e.g., etherification, esterification, phosphorylation, sulfation, oxidation, carbamation) (e.g., no substitution of hydrogens of glucan hydroxyl groups with a non-sugar chemical group). However, in some aspects, insoluble alpha-glucan can be a charged (e.g., cationic or anionic) derivative of an alpha-glucan as disclosed herein. The DoS of such a derivative typically is less than about 0.3, 0.25, 0.2, 0.15, 0.1, or 0.05. The type of derivative can be any of the derivatives disclosed herein (e.g., ether, ester), or as disclosed in Int. Patent Appl. Publ. No. WO2021247810, for example. Typically, insoluble alpha-glucan herein is enzymatically derived in an inert vessel (typically under cell-free conditions) and is not derived from a cell wall (e.g., fungal cell wall).

Also disclosed herein, but not claimed, is a method of inhibiting one or more microbes. Such a method can comprise at least:
(a) providing at least one alpha-glucan ether derivative as disclosed in some aspects, and
(b) contacting/treating one or more microbes with (exposing one or more microbes to) the at least one alpha-glucan ether derivative or a composition as disclosed herein comprising the at least one ether derivative, wherein this contacting (i) inhibits the growth and/or proliferation of the one or more microbes, and/or (ii) inhibits the colonization of the one or more microbes on a surface. A microbe in this method can be any microbe as disclosed herein, for example. Such a method can optionally be characterized as a microbial control, antimicrobial, or disinfection method (or other like terms).

An alpha-glucan ether derivative typically can be characterized to inhibit a microbe (i.e., exhibit antimicrobial activity toward the microbe) in a microbial control method herein by killing it and/or stopping/preventing/inhibiting its growth (e.g., achieving mature cell/virus size) and/or proliferation (cell/virus division/replication). In some aspects, one or more types of such an inhibition effect can affect at least about 30%, 40%, 50%, 60%, 70%, 80%, 85%, 90%, 91%, 92%, 93%, 94%, 95%, 96%, 97%, 98%, 99%, 99.5%, 99.8%, 99.9%, 99.95%, 99.98%, 99.99%, 99.995%, 99.998%, 99.999%, or 100% of (i) microbe cells of a microbe population contacted/treated by the alpha-glucan ether derivative, or (ii) enveloped virus particles of a population thereof contacted by the alpha-glucan ether derivative. Such a level of inhibition of a microbe can be with respect to one, two, three, four, or more, or all, species of microbe(s) that may be present in a microbe population being treated.

An alpha-glucan ether derivative that is used in a microbial control method may be dissolved in an aqueous composition (e.g., any as disclosed herein, such as water). The concentration of alpha-glucan ether derivative in a composition applied to one or more microbes in a microbial control method can be any concentration disclosed herein, for example, such as an MIC. The temperature and/or pH in which an alpha-glucan ether is applied can be any temperature and/or pH disclosed herein for a composition, for example. The amount of time for which one or more microbes are exposed to at least one alpha-glucan ether herein can be for about, at least about, or less than about, 0.06, 0.07, 0.08, 0.09, 0.1, 0.1, 0.3, 0.4, 0.5, 0.6, 0.7, 0.8, 0.9, 1, 2, 3, 4, 5, 6, 10, 12, 15, 18, 20, 24, 30, 36, 42, 48, 54, 60, 72, 84, 96, 108, 12-60, 12-54, 12-48, 12-42, 12-36, 12-30, 12-24, 18-60, 18-54, 18-48, 18-42, 18-36, 18-30, 18-24, or 36-60 hours, for example. A particular level of inhibition (above, e.g., at least ~95% or ~99.9% of microbial cells or enveloped virus particles are inhibited) may be achieved by one of these time amounts.

An ether derivative as provided in step (a) can be comprised in or on any composition disclosed herein. For example, a composition can be a fiber-containing material/article. An ether derivative as provided in step (a) may be adsorbed to the surface of a composition. An ether derivative as provided in step (a) may be adsorbed to the surface of a fiber-containing material/article that also has an insoluble alpha-glucan herein (alpha-1,3-glucan) adsorbed to its surface.

Application of a composition comprising an alpha-glucan ether to one or more microbes in a microbial control method can be for the purpose of inhibiting microbes that are known to exist, or possibly exist, in/on a composition/article being treated. A composition/article herein can be modified to comprise an alpha-glucan ether to inhibit one or more microbes that are already present in/on the composition/article. Yet, a composition/article herein (e.g., a surface, a fiber-containing composition/article) may have been previously modified to comprise an alpha-glucan ether to inhibit one or more microbes from ever living/growing in/on the composition/article (e.g., to act as a preservative, and/or to prevent colonization).

A surface to which one or more alpha-glucan ether derivatives can be applied can be the surface of any material, composition, article, device, or system disclosed herein. A surface can be associated with: a water management system (e.g., water treatment and/or purification, water storage/transportation, industrial water, water desalination) or any other water-bearing system (e.g., piping/conduits, heat exchangers, condensers, filters/filtration systems, storage tanks, water cooling towers, pasteurizers, boilers, sprayers, nozzles, ship hull, ballast water), agriculture (e.g., grain, fruits/vegetables, fishing, aquaculture, dairy, animal farming, timber, plants, soil conditioning), pharmaceutical setting, food processing/manufacturing, paper making, transportation (e.g., shipping, train or truck container), a medical/dental/healthcare setting (e.g., hospital, clinic, examination room, nursing home), medical/health devices (e.g., contact lenses, intravenous catheters and connectors, endotracheal tubes, intrauterine devices, mechanical heart valves, pacemakers, peritoneal dialysis catheters, prosthetic joints, tympanostomy tubes, urinary catheters, voice prostheses, instruments), food service setting (e.g., restaurant, commissary kitchen, cafeteria), retail setting (e.g., grocery, soft drink machine/dispenser), hospitality/travel setting (e.g., hotel/motel), sports/recreational setting (e.g., aquatics/tubs, spa), or office/home setting (e.g., bathroom, tub/shower, kitchen, appliances [e.g., laundry machine, automatic dishwashing machine, fridge, freezer], sprinkler system, home/building water piping, water storage tank, water heater). A surface herein can be abiotic, inert, and/or biotic (of life or derived from life), for example. An abiotic or inert surface can comprise a metal (e.g., iron, copper, nickel, zinc, titanium, molybdenum, chromium), for example, and optionally be a metal alloy (e.g., steel, stainless steel). An abiotic or inert surface can comprise plastic (polyethylene terephthalate [PET], high-density polyethylene [HDPE], polyvinyl chloride [PVC], low-density polyethylene [LDPE], polypropylene, polystyrene), rubber, porcelain/ceramic, silica/glass, and/or mineral material (e.g., stone/rock/concrete). A biotic surface can be that of wood/pulp, teeth, skin/nails/wounds, nasal passage, oral cavity, genitourinary tract, conjunctiva, or ear canal. Further examples of surfaces herein that can be treated with an alpha-glucan ether derivative include any of those of a system as disclosed in any of U.S. Patent Appl. Publ. Nos. 2013/0029884, 2005/0238729, 2010/0298275, 2016/0152495, 2013/0052250, 2015/009891, 2016/0152495, 2017/0044468, 2012/0207699, or 2020/0308592, or U.S. Patent Nos. 4552591, 4925582, 6478972, 6514458, 6395189, 7927496, or 8784659.

In performing step (b) of a microbial control method herein, (i) the growth and/or proliferation of one or more microbes is inhibited, and/or (ii) the colonization of one or more microbes on a surface is inhibited. Such inhibition can be measured using any suitable methodology, such as any procedure disclosed in the below Examples. For example, antimicrobial activity can be measured herein by following ASTM method E3160-18 (*ibid*.) (e.g., Procedure 2A below; e.g., at a use level ≤5000 ppm), ASTM method AATCC 100 (*ibid*.) (e.g., Procedure 2B below; e.g. at a use level ≤5000 ppm), an MIC test (e.g., Procedure 5 below), or EPA SOP No. MB-27-03 (*Standard Operating Procedure for Germicidal and Detergent Sanitizing Action of Disinfectants Tests)* (e.g., Procedure 6).

In some examples of performing step (b) of a microbial control method, the colonization of one or more microbes on a surface is inhibited. Such activity can be, for example, preventing one or more microbes (e.g., bacteria) from forming a biofilm on a surface (or any other form of surface-colonization), or reducing this ability (e.g., by about, or at least about, 30%, 40%, 50%, 60%, 70%, 80%, 85%, 90%, 91%, 92%, 93%, 94%, 95%, 96%, 97%, 98%, 99%, 99.5%, 99.9%, 100%, 50%-99.9%, 50%-99%, 50%-95%, 50%-90%, 70%-99.9%, 70%-99%, 70%-95%, or 70%-90%), as compared to what would occur if step (b) was not performed (under otherwise same conditions). This reduction can be augmented by about, or at least about, 30%, 40%, 50%, 60%, 70%, 80%, 85%, 90%, 91%, 92%, 93%, 94%, 95%, 96%, 97%, 98%, or 99%, for example, by including one or more nucleases herein (typically a DNAse) with an alpha-glucan ether. A nuclease can be included at any enzyme concentration herein; in some aspects the nuclease concentration can be about, or at least about 5, 10, 20, 25, 30, 40, 50, 60, 70, 75, 80, 90, 100, 125, 150, 175, 200, 250, 300, 350, 400, 450, 500, 5-100, 10-100, 20-100, 30-100, 40-100, 50-100, 50-500, 100-500, 150-500, or 200-500 ppm. Inhibition of biofilm formation can be measured using any suitable methodology, such as any procedure disclosed in the below Examples (e.g., Example 21). Inhibiting biofilm formation or any other form of surface colonization by a microbe can be achieved by targeting microbial cells that are planktonic and/or that have settled on a surface. A biofilm that has already formed can be treated in step (b); such treatment can cause the biofilm to be inhibited as disclosed herein, and/or to dispersed.

A biofilm can comprise one or more microbes (typically bacteria) disclosed herein, for example. In some aspects, a biofilm can be one that forms on a hard surface such as in a laundry machine or automatic dishwasher, or on a fabric or related fiber-containing material herein (e.g., as a result of being in a laundry machine and/or laundry cleaning setting). A bacteria that can form a biofilm on a fabric and/or a hard surface in a laundry machine setting can be, for example, a species of *Acinetobacter, Aeromicrobium, Brevundimonas, Microbacterium, Micrococcus* (e.g., *M*. *luteus*), *Pseudomonas* (e.g., *P*. *alcaliphila, P. fluorescens*), *Staphylococcus* (e.g., *S. epidermidis*), or *Stenotrophomonas.*

In some examples of performing step (b) of a microbial control method, the production of malodor (unpleasant odor) by one or more microbes can be controlled (e.g., prevented or reduced). Thus, a microbial control method can optionally be characterized as an odor control method. A reduction in malodor herein (as produced and/or detected) can be by about, or at least about, 30%, 40%, 50%, 60%, 70%, 80%, 85%, 90%, 91%, 92%, 93%, 94%, 95%, 96%, 97%, 98%, 99%, 99.5%, 99.9%, 100%, 50%-99.9%, 50%-99%, 50%-95%, 50%-90%, 70%-99.9%, 70%-99%, 70%-95%, or 70%-90%, as compared to the level of malodor that would be produced and/or detected if step (b) was not performed (under otherwise same conditions). Malodor detection can be measured using any suitable methodology (e.g., measure subjectively using human sensory odor panels), such as by following Procedure 4 below or Test Method IACM 0710.

A composition in some aspects comprising at least one alpha-glucan derivative (e.g., ether, ester, or carbamate) and at least one insoluble alpha-glucan (alpha-1,3-glucan), can be an aqueous composition (e.g., a dispersion such as colloidal dispersion, where the alpha-glucan derivative is dissolved in the aqueous phase of the dispersion; e.g., where such a dispersion is used in a finishing method herein) or a dry composition, for example. In some aspects, a composition can comprise about, at least about, or less than about, 0.001, 0.0025, 0.005, 0.0075, 0.01, 0.025, 0.05, 0.075, 0.1, 0.125, 0.15, 0.2, 0.25, 0.3, 0.4, 0.5, 0.6, 0.7, 0.75, 0.8, 0.9, 1.0, 1.2, 1.25, 1.4, 1.5, 1.6, 1.75, 1.8, 2.0, 2.25, 2.5, 3.0, 3.5, 4.0, 4.5, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 99.5 wt% or w/v% of an alpha-glucan derivative herein. A composition can comprise a range between any two of these wt% or w/v% values of alpha-glucan derivative (e.g., 0.01-0.2, 0.01-0.15, 0.01-0.125, 0.01-0.1, 0.01-0.075, 0.025-0.2, 0.025-0.15, 0.025-0.125, 0.025-0.1, 0.025-0.075, 0.05-0.2, 0.05-0.15, 0.05-0.125, 0.05-0.1, 0.05-0.075, 0.075-0.2, 0.075-0.15, 0.075-0.125, or 0.075-0.1 wt% or w/v%), for example. Any of the foregoing wt% or w/v% values/ranges can apply, for example, to the content of an insoluble alpha-glucan in a composition herein. Any of the foregoing concentration values can be expressed in terms of its respective parts-per-million (ppm) value, if desired. In some aspects, the ratio of an alpha-glucan derivative to an insoluble alpha-glucan on a weight-to-weight basis can be about 3:1, 2.5:1, 2:1, 1.5:1, 1.25:1, 1:1, 0.75:1, 0.5:1, or 0.25:1. The liquid component of an aqueous composition can be an aqueous fluid such as water or aqueous solution, for instance. The solvent of an aqueous solution typically is water, or can comprise about, or at least about, 10, 20, 30, 40, 50, 60, 70, 80, 90, 95, 98, or 99 wt% water, for example. In some aspects, a composition herein can comprise, or be in the form of, a solution, dispersion (e.g., emulsion), mixture, dry powder, or extrusion. Since an alpha-glucan derivative herein typically is aqueous-soluble, its presence in a dispersion or mixture, for example, would be understood as being dissolved in an aqueous solution that is part of the dispersion or mixture.

The aqueous solution component of an aqueous composition in some aspects has no (detectable) dissolved sugars, or about 0.1-1.5, 0.1-1.25, 0.1-1.0, 0.1-.75, 0.1-0.5, 0.2-0.6, 0.3-0.5, 0.2, 0.3, 0.4, 0.5, or 0.6 wt% dissolved sugars. Such dissolved sugars can include sucrose, fructose, leucrose, and/or soluble gluco-oligosaccharides, for example. The aqueous solution component of an aqueous composition in some aspects can have one or more salts/buffers (e.g., Na⁺, Cl⁻, NaCl, phosphate, tris, citrate) (e.g., ≤ 0.1, 0.5, 1.0, 2.0, or 3.0 wt%), and/or a pH of about 4.0, 4.5, 5.0, 5.5, 6.0, 6.5, 7.0, 7.5, 8.0, 8.5, 9.0, 9.5, 10.0, 10.5, 4.0-10.0, 4.0-9.0, 4.0-8.0, 5.0-10.0, 5.0-9.0, 5.0-8.0, 6.0-10.0, 6.0-9.0, or 6.0-8.0, for example.

The temperature of a composition herein can be about, at least about, or less than about, 0, 5, 10, 15, 20, 25, 30, 35, 37, 40, 42, 45, 50, 55, 60, 65, 70, 75, 80, 85, 90, 95, 100, 105, 110, 115, 120, 125, 130, 10-30, 10-25, 15-30, 15-25, 20-40, 20-35, 20-30, 20-25, 25-30, 30-50, 30-45, 30-40, 30-35, 35-40, 35-50, 40-45, 50-60, 110-130, 110-125, 110-120, 115-130, or 115-125 °C, for example.

A composition herein can in some aspects be non-aqueous (e.g., a dry composition). Examples of such embodiments include powders, granules, microcapsules, flakes, or any other form of particulate matter. Other examples include larger compositions such as pellets, bars, kernels, beads, tablets, sticks, or other agglomerates. A non-aqueous or dry composition typically has about, or no more than about, 5, 4, 3, 2, 1.5, 1.0, 0.5, 0.25, 0.10, 0.05, or 0.01 wt% water comprised therein. In some aspects (e.g., those directed to a laundry or dish washing products), a dry composition herein can be provided in a sachet or pouch.

A composition can in some aspects be a detergent composition. Examples of such compositions are disclosed herein as detergents for dishwashing and detergents for fabric care.

A composition herein can in some aspects comprise one or more salts such as a sodium salt (e.g., NaCl, Na₂SO₄). Other non-limiting examples of salts include those having (i) an aluminum, ammonium, barium, calcium, chromium (II or III), copper (I or II), iron (II or III), hydrogen, lead (II), lithium, magnesium, manganese (II or III), mercury (I or II), potassium, silver, sodium strontium, tin (II or IV), or zinc cation, and (ii) an acetate, borate, bromate, bromide, carbonate, chlorate, chloride, chlorite, chromate, cyanamide, cyanide, dichromate, dihydrogen phosphate, ferricyanide, ferrocyanide, fluoride, hydrogen carbonate, hydrogen phosphate, hydrogen sulfate, hydrogen sulfide, hydrogen sulfite, hydride, hydroxide, hypochlorite, iodate, iodide, nitrate, nitride, nitrite, oxalate, oxide, perchlorate, permanganate, peroxide, phosphate, phosphide, phosphite, silicate, stannate, stannite, sulfate, sulfide, sulfite, tartrate, or thiocyanate anion. Thus, any salt having a cation from (i) above and an anion from (ii) above can be in a composition, for example. A salt can be present in an aqueous composition herein at a wt% of about, or at least about, .01, .025, .05, .075, .1, .25, .5, .75, 1.0, 1.25, 1.5, 1.75, 2.0, 2.5, 3.0, 3.5, .01-3.5, .5-3.5, .5-2.5, or .5-1.5 wt% (such wt% values typically refer to the total concentration of one or more salts), for example.

A composition herein can optionally contain one or more enzymes (active enzymes). Examples of suitable enzymes include proteases, cellulases, hemicellulases, peroxidases, lipolytic enzymes (e.g., metallolipolytic enzymes), xylanases, lipases, phospholipases, esterases (e.g., arylesterase, polyesterase), perhydrolases, cutinases, pectinases, pectate lyases, mannanases, keratinases, reductases, oxidases (e.g., choline oxidase), phenoloxidases, lipoxygenases, ligninases, pullulanases, tannases, pentosanases, malanases, beta-glucanases, arabinosidases, hyaluronidases, chondroitinases, laccases, metalloproteinases (e.g., thermolysin), amadoriases, glucoamylases, arabinofuranosidases, phytases, isomerases, transferases, nucleases, and amylases. If an enzyme(s) is included, it may be comprised in a composition herein at about 0.0001-0.1 wt% (e.g., 0.01-0.03 wt%) active enzyme (e.g., calculated as pure enzyme protein), for example. In fabric care or automatic dishwashing applications, an enzyme herein (e.g., any of the above such as cellulase, protease, amylase, lipase, and/or nuclease) can be present in an aqueous composition in which a fabric or dish is treated (e.g., wash liquor, grey water) at a concentration that is minimally about 0.01-0.1 ppm total enzyme protein, or about 0.1-10 ppb total enzyme protein (e.g., less than 1 ppm), to maximally about 100, 200, 500, 1000, 2000, 3000, 4000, or 5000 ppm total enzyme protein, for example.

As described above, a composition herein can optionally contain a nuclease. A nuclease can be a deoxyribonuclease (DNase) or ribonuclease (RNase), for example. A DNase herein can catalyze the hydrolytic cleavage of one or more phosphodiester linkages in the backbone of a deoxyribonucleic acid (DNA) molecule, thereby degrading (partly or totally) the DNA molecule; this definition similarly applies to an RNase with respect to a ribonucleic acid (RNA) molecule . A nuclease in some aspects can be an endonuclease (e.g., a nuclease that cleaves anywhere along the chain of a DNA or RNA molecule (e.g., endodeoxyribonuclease) or exonuclease (e.g., a nuclease that cleaves at one or both ends of a DNA or RNA molecule) (e.g., exodeoxyribonuclease). A nuclease in some aspects, but less typically, can be a site-specific endonuclease. Examples of suitable nucleases are disclosed in U.S. Patent Appl. Publ. Nos. 2017/0081617, 2020/0248106, 2020/0032171, 2019/0048291, 2018/0187175, 2020/0199498, 2020/0024584, 2020/0109381, 2020/003217, 2020/0190437, 2020/0032169, 2020/0190436, 2019/0127665, 2020/032546, 2020/0190440, 2020/0190438, 2021/0137075, and 2019/0256831. A nuclease in some aspects can be (derivable from) a bacterial, fungal (yeast), algal, plant, or animal nuclease, for example. A nuclease in some aspects can be from (derivable from) a species of *Bacillus* (e.g., sp-62738, sp-62490, sp-13390, sp-62599, sp-62520, sp-16840, sp-62668, sp-13395, sp-11238, sp-62451, sp-18318, sp. SA2-6, *B. subtilis, B. licheniformis, B. pumilus, B. horikoshii, B. akibai, B. algicola, B. cibi [B. indicus], B. horneckiae, B. idriensis, B. vietnamensis, B. hwajinpoensis, B. marisflavi, B. luciferensis), Paenibacillus* (e.g., sp-62212, sp-62605, *P. xylanexedens, P. mucilaginosus), Escherichia* (e.g., *E. coli*)*, Jeotgalibacillus* (e.g., sp-13376), *Serratia* (e.g., *S*. *marcescens), Staphylococcus* (e.g., *S*. *aureus), Streptomyces* (e.g., sp-63712, *S. iakyrus, S. thermoalcalitolerans), Streptococcus* (e.g., *S. infantis), Fictibacillus* (e.g., sp-62719), *Exiguobacterium* (e.g., sp. NG55), *Saccharothrix* (e.g., *S. australiensis), Kutzneria* (e.g., *K. albida), Pholiota* (e.g., *P. squarrosa), Marasmius* (e.g., *M*. *oreades), Cercospora* (e.g., *C. fusimaculans), Deconica* (e.g., *D. coprophila*)*, Physisporinus* (e.g., *P. sanguinolentus), Stropharia* (e.g., *S*. *semiglobata), Cladosporium* (e.g., *C. cladosporioides), Irpex* (e.g., *I. lacteus), Phlebia* (e.g., *P. subochracea), Rhizoctonia* (e.g., *R*. *solani*)*, Ascobolus* (e.g., sp. ZY179, *A. stictoideus), Urnula* (e.g., sp-56769), *Morchella* (e.g., *M. costata), Trichobolus* (e.g., *T. zukalii*)*, Trichophaea* (e.g., *T. saccata, T. minuta, T. abundans), Pseudoplectania* (e.g., *P. nigrella), Gyromitra* (e.g., *G. esculenta), Morchella* (e.g., *M. esculenta, M. crassipes), Disciotis* (e.g., *D. venosa), Aspergillus* (e.g., *A. oryzae, A. niger, A. nidulans), Mortierella* (e.g., *M. humilis), Penicillium* (e.g., *P. citrinum), Bos taurus* (e.g., pancreatic nuclease). In some aspects, a nuclease can be used along with any other enzyme(s) disclosed herein, such as a metalloproteinase (e.g., thermolysin).

In some aspects, a composition can comprise at least one other antimicrobial agent in addition to an alpha-glucan ether herein that has antimicrobial activity. Examples of another antimicrobial agent include antibacterial agents, phenolic compounds (e.g., 4-allylcatechol; p-hydroxybenzoic acid esters such as benzylparaben, butylparaben, ethylparaben, methylparaben and propylparaben; 2-benzylphenol; butylated hydroxyanisole; butylated hydroxytoluene; capsaicin; carvacrol; creosol; eugenol; guaiacol; halogenated bisphenolics such as hexachlorophene and bromochlorophene; 4-hexylresorcinol; 8-hydroxyquinoline and salts thereof; salicylic acid esters such as menthyl salicylate, methyl salicylate and phenyl salicylate; phenol; pyrocatechol; salicylanilide; thymol; lactic acid; halogenated diphenylether compounds such as triclosan and triclosan monophosphate), copper (II) compounds (e.g., copper (II) chloride, fluoride, sulfate and hydroxide), zinc ion sources (e.g., zinc acetate, citrate, gluconate, glycinate, oxide, and sulfate), phthalic acid and salts thereof (e.g., magnesium monopotassium phthalate), hexetidine, octenidine, sanguinarine, benzalkonium chloride, domiphen bromide, alkylpyridinium chlorides (e.g. cetylpyridinium chloride, tetradecylpyridinium chloride, N-tetradecyl-4-ethylpyridinium chloride), iodine, sulfonamides, bisbiguanides (e.g., alexidine, chlorhexidine, chlorhexidine digluconate), piperidino derivatives (e.g., delmopinol, octapinol), magnolia extract, grapeseed extract, rosemary extract, menthol, geraniol, citral, eucalyptol, antibiotics (e.g., augmentin, amoxicillin, tetracycline, doxycycline, minocycline, metronidazole, neomycin, kanamycin, clindamycin), and/or any antibacterial agents disclosed in U.S. Patent No. 5776435. One or more additional antimicrobial agents can optionally be present at about 0.01-10 wt% (e.g., 0.1-3 wt%), for example, in a composition herein.

A composition can comprise one, two, three, four or more different alpha-glucan derivatives herein and, optionally, at least one non-derivatized alpha-glucan (e.g., as disclosed herein, such as an insoluble alpha-glucan). For example, a composition can comprise at least one type of alpha-glucan ether derivative and at least one type of alpha-glucan; in some aspects, the latter can be a precursor compound of the former. A non-derivatized alpha-glucan (e.g., precursor compound) in some aspects is not present and/or not detectable (e.g., is not detectable down to a level of about, or less than about, 0.01, 0.005, 0.001, or 0.0005 wt%), for example, in a composition.

A composition in some aspects comprising at least one alpha-glucan derivative (e.g., an antimicrobial ether derivative) and at least one insoluble alpha-glucan herein can be a fiber-containing material/article. An alpha-glucan derivative and insoluble alpha-glucan can be adsorbed to the fiber-containing material/article (i.e., the alpha-glucan derivative and insoluble glucan are adsorbed to the surface[s] of the material/article), for example. In some aspects, a fiber-containing material/article comprises fiber that has previously been treated to adsorb an alpha-glucan derivative and insoluble alpha-glucan (prior to using the fiber to produce the material/article). A fiber-containing material/article herein can have antimicrobial activity by virtue of containing one or more antimicrobial alpha-glucan ethers as presently disclosed. A fiber-containing material/article comprising at least one alpha-glucan derivative in some aspects can be as produced by a method of treating a fiber-containing material/article as presently disclosed. A fiber-containing material/article can be any fiber-containing material/article as disclosed herein, for example.

Fiber of a fiber-containing material/article herein can comprise natural fibers, synthetic fibers, semi-synthetic fibers, or any combination thereof, for example. Examples of natural fibers herein include cellulosic fibers (e.g., cotton; pulp such as wood pulp or other type of plant pulp; long vegetable fibers such as jute, flax, ramie, coir, kapok, sisal, henequen, abaca, hemp and sunn) and proteinaceous fibers (e.g., wool and related mammalian fibers, silk). Examples of synthetic fibers include polyester, acrylic, nylon and spandex. A semi-synthetic fiber herein typically is produced using naturally occurring material that has been chemically derivatized or chemically processed, an example of which is rayon (e.g., viscose, modal, lyocell).

A fiber-containing material/article herein can be a yarn or fabric, for example. A fabric typically can be any of a woven fabric/product, knitted fabric/product, or non-woven fabric/product. Examples of fabric types herein include broadcloth, canvas, chambray, chenille, chintz, corduroy, cotton, cretonne, damask, denim, flannel, gingham, jacquard, knit, matelassé, oxford, percale, poplin, plissé, sateen, seersucker, sheers, terry cloth, twill, velvet, rayon, linen, Tencel^{®}; silk, wool, polyester (e.g., Sorona^{®}), acrylic, nylon, spandex (e.g., LYCRA), jute, flax, ramie, coir, kapok, sisal, henequen, abaca, hemp and sunn. Fabric comprising a combination of fiber types herein (e.g., natural and synthetic) include those with both cotton fiber and polyester, for example. Materials/articles containing one or more fabrics herein include, for example, clothing (e.g., exercise/fitness/sport clothing, pants, shirts, underwear/undergarments, socks, hats, belts, shoes/footwear, neckwear), curtains, shower curtains, drapes, upholstery, carpeting, bed linens, mattresses, bath linens, towels, tablecloths, sleeping bags, tents, car interiors, laundry containers, roping/netting, etc.

A non-woven product or fabric herein can be, for example, air-laid, dry-laid, wet-laid, carded, electrospun, spun-lace, spun-bond, or melt-blown. In some aspects, a non-woven product can be an abrasive or scouring sheet, agricultural covering, agricultural seed strip, apparel lining, automobile headliner or upholstery, bib, cheese wrap, civil engineering fabric, coffee filter, cosmetic remover or applicator, detergent pouch/sachet, fabric softener sheet, envelope, face mask, filter, garment bag, heat or electricity conductive fabric, household care wipe (e.g., for floor care, hard surface cleaning, pet care etc.), house wrap, hygiene product (e.g., sanitary pad/napkin, underpad), insulation, label, laundry aid, medical care or personal injury care product (e.g., bandage, cast padding or cover, dressing, pack, sterile overwrap, sterile packaging, surgical drape, surgical gown, swab), mop, napkin or paper towel, paper, tissue paper, personal wipe or baby wipe, reusable bag, roofing undercovering, table linen, tag, tea or coffee bag, upholstery, vacuum cleaning bag, or wallcovering. The fiber of a non-woven product can comprise cellulose and/or alpha-1,3-glucan in some aspects, or can comprise one or more other materials disclosed herein that can be used to form a fiber. Examples of non-woven products herein, non-woven product materials, and/or methods of production of non-woven products and materials, can be as disclosed in U.S. Pat. Appl. Publ. Nos. 2020/0370216, 2018/0282918, 2017/0167063, 2018/0320291, or 2010/0291213.

A fiber-containing material/article as presently disclosed can be a paper/packaging composition or cellulose fiber-containing composition. Examples of such compositions can be any type of paper/packaging or cellulose fiber-containing composition disclosed herein, such as paper (e.g., writing paper, office paper, copying paper, crafting paper), cardboard, paperboard, corrugated board, tissue paper, napkin/paper towel, wipe, or non-woven fabric. Formulations and/or components (in addition to an alpha-glucan derivative and insoluble alpha-glucan herein) of a paper/packaging composition or cellulose fiber-containing composition herein, and well as forms of these compositions, can be as described in, for example, U.S. Patent Appl. Publ. Nos. 2018/0119357, 2019/0330802, 2020/0062929, 2020/0308371, or 2020/0370216.

In one embodiment, the present disclosure concerns a composition comprising at least: (a) a derivative of an alpha-glucan, wherein (i) at least about 40% of the glycosidic linkages of the alpha-glucan are alpha-1,6 linkages, and (ii) the alpha-glucan has a degree of substitution (DoS) of about 0.001 to about 3.0 with at least one positively charged organic group; and (b) an insoluble alpha-glucan, wherein at least about 50% of the glycosidic linkages of the insoluble alpha-glucan are alpha-1,3 glycosidic linkages, and the weight-average degree of polymerization (DPw) of the insoluble alpha-glucan is at least 10.

In another embodiment, the present disclosure concerns a method of treating a fiber-containing material/article, the method comprising: (a) providing a composition herein comprising a positively charged alpha-glucan derivative and an insoluble alpha-glucan, and (b) contacting the composition with a fiber-containing material/article, wherein at least the alpha-glucan derivative and the insoluble alpha-glucan are adsorbed (or otherwise incorporated into/onto) to the fiber-containing material/article.

Some aspects herein concern a method of treating a fiber-containing material/article, the method comprising:
(a) providing a composition herein comprising (a) a derivative of an alpha-glucan, wherein (i) at least about 40% of the glycosidic linkages of the alpha-glucan are alpha-1,6 linkages, and (ii) the alpha-glucan has a degree of substitution (DoS) of about 0.001 to about 3.0 with at least one positively charged organic group; and (b) an insoluble alpha-glucan, wherein at least about 50% of the glycosidic linkages of the insoluble alpha-glucan are alpha-1,3 glycosidic linkages, and the weight-average degree of polymerization (DPw) of the insoluble alpha-glucan is at least 10, and
(b) contacting the composition with a fiber-containing material/article (or treating a fiber-containing material/article with the composition), wherein at least the alpha-glucan derivative and the insoluble alpha-glucan are adsorbed to the fiber-containing material/article. Such a method can optionally be characterized herein as a finishing method (e.g., a textile finishing method). The fiber-containing material/article can acquire antimicrobial activity from the contacting step when using an alpha-glucan derivative having antimicrobial activity, for example.

Any alpha-glucan derivative, insoluble alpha-glucan, and/or fiber-containing material/article as disclosed herein, for example, can be used in a method of treating a fiber-containing material/article. Step (a) of a finishing method typically can comprise providing at least one alpha-glucan derivative and at least one insoluble alpha-glucan in an aqueous composition, which can be any as disclosed herein, for example. In some aspects, an aqueous composition is water comprising one or more dissolved alpha-glucan derivatives and one or more dispersed insoluble alpha-glucans. Any of the aqueous compositions, or features thereof, as described below for conducting a finishing method can be considered a composition of the present disclosure. Any product of performing such a finishing method can be considered a composition of the present disclosure.

The respective/individual concentration of one or more alpha-glucan derivatives and one or more insoluble alpha-glucans in an aqueous composition for treating a fiber-containing material/article in a finishing method can be any concentration disclosed herein for an alpha-glucan derivative and insoluble alpha-glucan in an aqueous composition, for example.

An aqueous composition for a finishing method herein can optionally comprise a non-ionic surfactant such as a non-ionic alcohol ethoxylate-based surfactant. The concentration of such a surfactant in an aqueous composition can be about, or less than about, 0.1, 0.09, 0.08, 0.07. 0.06, 0.05, 0.05-0.1, 0.05-0.09, or 0.05-0.08 wt%, for example. Suitable examples of non-ionic surfactants herein include secondary alcohol ethoxylate (e.g., with hydrophilic-lipophilic balance of ~10.5) (e.g., TERGITOL^{™} 15-S-5 [CAS No. 84133-50-6], Dow Inc.) and alcohol ethoxylate (e.g., with hydrophilic-lipophilic balance of ~12.5) (e.g., Ecosurf^{™} EH9, Dow Inc. [CAS No. 64366-70-7]).

An aqueous composition for a finishing method herein can optionally comprise an uncharged polymer such as a polyurethane (e.g., a polyether polyurethane), for example. A polyurethane can be non-ionic (and optionally also hydrophilic and/or aliphatic), for example. In some aspects, a polyurethane can initially be provided as a PUD (polyurethane dispersion), in which case the PUD is used for adding polyurethane to an aqueous composition. A PUD can comprise about 25-50 wt% (e.g., ~30-40 wt%, ~35 wt%) polyurethane solids in water and have a pH of about 4.5-7.5 (e.g., pH 5.0-7.0), for example. A PUD can optionally have a density of about 7-10 pounds/gallon (e.g., ~8-9 pounds/gallon), a viscosity of less than about 150 centipoise (cps) (e.g., <100 cps), a percent elongation of about 500-700% (e.g., about 600%), a 100%-modulus of about 30-50 psi (e.g., ~40 psi), and/or a Sward hardness of less than about 5 (e.g., about <4). An example of a PUD product useful herein is Bondthane^{™} UD-410 (Bond Polymers Int.).

The concentration of one or more uncharged polymers (e.g., alpha-1,3-glucan or polyurethane) in an aqueous composition herein can be about, or less than about, 0.1, 0.09, 0.08, 0.07, 0.06, 0.05, 0.05-0.1, 0.05-0.09, or 0.05-0.08 wt% in some aspects.

In some aspects of a finishing method, including one or more non-ionic surfactants (e.g., alcohol ethoxylate or secondary alcohol ethoxylate) and/or one or more uncharged polymers (e.g., polyurethane, or an insoluble alpha-glucan herein such as alpha-1,3-glucan) can enhance the uptake of an alpha-glucan derivative herein onto a fiber-containing material/article. Such an enhancement can be an increase of about, or at least about, 25%, 50%, 75%, 100%, 150%, or 200%, for example, as compared to applying an aqueous composition that only differs by lacking the aforementioned uptake enhancer(s) (under otherwise same conditions). In some aspects, a fiber-containing material/article produced by a finishing method can comprise, in addition to an alpha-glucan derivative herein, a non-ionic surfactant and/or an uncharged polymer (e.g., an insoluble alpha-glucan herein such as alpha-1,3-glucan).

An aqueous composition for a finishing method in some aspects comprises less than about 5, 4, 3, 2, 1, 0.5, 0.1, 0.05, 0.01, 0.005, or 0.001 wt% of an anionic compound(s), or no anionic compound(s), or an anionic compound is undetectable, optionally wherein the anionic compound is a polymer or surfactant/detergent. Typically, an anionic compound in these aspects does not refer to an anion of a salt (e.g., a halogen anion).

In some aspects of a finishing method, an aqueous composition can be in the form of, or comprise, fabric softener (liquid fabric softener). An example of such an aqueous composition is a rinse used in laundering a fabric-comprising material/article herein typically following cleaning of the fabric-comprising material/article with a laundry detergent composition (e.g., laundry rinse such as used in a laundry rinse cycle in a washing machine). The concentration of an alpha-glucan derivative herein in an aqueous composition comprising fabric softener (e.g., a rinse) can be about, or at least about, 20, 30, 40, 50, 60, 70, 80, 20-80, 20-70, 20-60, 30-80, 30-70, 30-60, 40-80, 40-70, or 40-60 ppm, for example. The concentration of a fabric softener in the aqueous composition (e.g., a rinse) can be about, or at least about, 50, 75, 100, 150, 200, 300, 400, 500, 600, 50-600, 50-500, 50-400, 50-300, 50-200, 100-600, 100-500, 100-400, 100-300, 100-200, 10-600, 50-500, 50-400, 50-300, 50-200, 200-600, 200-500, 200-400, or 200-300 ppm, for example. The fabric softener concentration can be based on the total fabric softener composition added (not necessarily based on an individual component of the fabric softener), or based on one or more fabric softening agents(s) in the fabric softener formulation. A fabric softener herein can comprise at least water and, for example, one or more of a fabric softening agent (e.g., diethyl ester dimethyl ammonium chloride), anti-static agent, perfume, wetting agent, viscosity modifier (e.g., calcium chloride), pH buffer/buffering agent (e.g., formic acid), antimicrobial agent (optionally in addition to an alpha-glucan ether herein), anti-oxidant, radical scavenger (e.g., ammonium chloride), chelant/builder (e.g., diethylenetriamine pentaacetate), anti-foaming agent/lubricant (e.g., polydimethylsiloxane), preservative (e.g., benzisothiazolinone) and colorant. In some aspects, a fabric softener can comprise at least water and one or more of a fabric softening agent, viscosity modifier, pH buffer/buffering agent, radical scavenger, chelant/builder and anti-foaming agent/lubricant. A fabric softener can be perfume-free and/or dye-free, or have less than about 0.1 wt% of a perfume and/or dye in some aspects. In some aspects, a fabric softener that can be adapted for use herein can be as disclosed in any of U.S. Patent Appl. Publ. Nos. 2014/0366282, 2001/0018410, 2006/0058214, 2021/0317384, or 2006/0014655, or Int. Patent Appl. Publ. Nos. WO2007/078782, WO1998/016538, WO1998/012293, WO1998007920, WO2000/070004, WO2009/146981, WO2000/70005, or WO2013087366. Some brands of fabric softeners that can be adapted for use herein, if desired, include DOWNY, DOWNY ULTRA, DOWNY INFUSIONS, ALL, SNUGGLE, LENOR and GAIN. A liquid fabric softener product (e.g., as it exists before being used in a laundry rinse cycle) can be formulated to include one or more alpha-glucan derivatives and insoluble alpha-glucans in some aspects. A fabric softener in some aspects can be in a unit dose, such as disclosed herein for a detergent.

Step (b) of a finishing method herein can comprise at least contacting a composition as presently disclosed comprising at least one alpha-glucan derivative and at least one insoluble alpha-glucan with a fiber-containing material/article, wherein at least the alpha-glucan derivative and insoluble alpha-glucan typically are adsorbed to the fiber-containing material/article. This step can be conducted under any temperature and/or pH as disclosed for a composition herein, for example. In some aspects, the contacting step can be conducted for about, or at least about, (i) 2, 3, 4, 5, 6, 7, 8, 9, 10, 12.5, 15, 20, 30, 45, 60, 90, or 120 minutes, or (ii) 3, 4, 5, 6, 9, 12, 15, 18, 21, 24, 30, 36, 42, or 48 hours. Contacting in some aspects can be performed using any suitable methodology, such as any procedure disclosed in the below Examples (e.g., an exhaustion process or padding process as described in Procedure 1A or 1B, respectively). In some aspects, contacting can be performed in the context of exposing a fiber-containing article to a liquid fabric softener (e.g., as diluted in water, such as what is typically used in a laundry rinse cycle) (e.g., as described below in Procedure 7).

Step (b) of a finishing method herein can optionally further comprise a drying step following the contacting step. A drying step can be performed directly after the contacting step, or following one or more additional steps (e.g., washing and/or rinsing [e.g., rinsing in water) that might follow the contacting step. Drying can be performed by any suitable means, such as air drying (e.g., ~20-25 °C), or at a temperature of at least about 30, 40, 50, 60, 70, 80, 90, 100, 120, 140, 160, 170, 175, 180, or 200 °C, for example. A fiber-containing material or article has that been dried herein typically has less than 3, 2, 1, 0.5, or 0.1 wt% water comprised therein.

A finishing method in some aspects can further comprise a step (c) of subjecting the fiber-containing article, following step (b), to at least one cycle of washing and/or rinsing. Washing and/or rinsing can be performed immediately following step (b), or after an optional rinsing and/or drying step(s) that would come between steps (b) and (c). Washing and/or rinsing (optionally followed by a drying step) can be performed about, at least about, or up to about, 10, 20, 30, 40, 50, 60, 70, 80, 90, 100, 1250, 150, 175, or 200 times/cycles, for example. Each cycle of washing and/or rinsing can optionally be followed by a drying step (e.g., as above). Rinsing can be done with water, for example, and can optionally be conducted for a time and/or at a temperature as listed herein for a washing step. A washing step can optionally be conducted following a previous washing step without any intervening rinsing and/or drying steps, if desired; for example, grey water formed during a washing can be removed from the washed material/article (e.g., by spinning in a laundry machine) followed by initiation of another washing. A rinsing step can optionally be conducted following a previous rinsing step without any intervening washing and/or drying steps, if desired; for example, rinse water formed during a rinsing can be removed from the rinsed material/article (e.g., by spinning in a laundry machine) followed by initiation of another rinsing.

Washing (laundering) can be performed, for example, using any relevant conditions (e.g., time, temperature, wash/rinse volumes) for conducting a fabric care method or laundry method as disclosed in WO1997/003161 and U.S. Patent Nos. 4794661, 4580421 and 5945394. In some aspects, washing can be done as disclosed in the below Examples; for example, washing can be performed using ASTM E3162-18 (*Measuring the Durability of Antibacterial Agents Applied to Textiles under Simulated Home Laundering Conditions)* (e.g., Procedure 3 below). In some aspects, a washing step can be conducted: (i) for at least about 5, 10, 20, 30, 40, 50, 60, 70, 80, 90, 100, 110, or 120 minutes; (ii) at a temperature of at least about 10, 15, 20, 25, 30, 35, 40, 45, 50, 55, 60, 65, 70, 75, 80, 85, 90, or 95 °C (e.g., for laundry wash or rinse: a "cold" temperature of about 15-30 °C, a "warm" temperature of about 30-50 °C, a "hot" temperature of about 50-95 °C); (iii) at a pH of about 2, 3, 4, 5, 6, 7, 8, 9, 10, 11, or 12 (e.g., pH range of about 2-12, or about 3-11); (iv) at a salt (e.g., NaCl) concentration of at least about 0.5, 1.0, 1.5, 2.0, 2.5, 3.0, 3.5, or 4.0 wt%; or any combination of (i)-(iv). Washing typically can be done in an aqueous composition in which a laundry detergent (e.g., heavy duty laundry detergent) is mixed (e.g., in laundry bath/tub water); a laundry detergent implemented here can be any as disclosed herein, for example.

A fiber-containing material/article in some aspects of a finishing method, after having been subjected to at least one cycle of washing and/or rinsing as presently disclosed (e.g., step [c] of finishing method], retains about, or at least about, 50%, 55%, 60%, 65%, 70%, 75%, 80%, 85%, 90%, 95%, 96%, 97%, 98%, or 99% by weight of the alpha-glucan derivative that was adsorbed to the fiber-containing material/article from step (b).

In some aspects in which an alpha-glucan derivative herein with antimicrobial activity is used in a finishing method, the fiber-containing material/article (e.g., the surface thereof) acquires antimicrobial activity from step (b). This acquired antimicrobial activity, or at least about 50%, 55%, 60%, 65%, 70%, 75%, 80%, 85%, 90%, 95%, 96%, 97%, 98%, or 99% of the acquired antimicrobial activity, is retained by the fiber-containing material/article after having been subjected to at least one cycle of washing and/or rinsing as presently disclosed (e.g., step [c] of finishing method).

A composition as presently disclosed, for example one comprising at least one alpha-glucan derivative and at least one insoluble alpha-glucan, can be in the form of a household care product, personal care product, industrial product, medical product, or pharmaceutical product, for example, such as described in any of U.S. Patent Appl. Publ. Nos. 2018/0022834, 2018/0237816, 2018/0230241, 20180079832, 2016/0311935, 2016/0304629, 2015/0232785, 2015/0368594, 2015/0368595, 2016/0122445, 2019/0202942, or 2019/0309096, or Int. Patent Appl. Publ. No. WO2016/133734. In some aspects, a composition can comprise at least one component/ingredient of a household care product, personal care product, industrial product, or medical product as disclosed in any of the foregoing publications and/or as presently disclosed.

Personal care products herein are not particularly limited and include, for example, skin care compositions, cosmetic compositions, antifungal compositions, and antibacterial compositions. Personal care products herein may be in the form of, for example, lotions, creams, pastes, balms, ointments, pomades, gels, liquids, combinations of these and the like. The personal care products disclosed herein can include at least one active ingredient, if desired. An active ingredient is generally recognized as an ingredient that causes an intended pharmacological effect.

A skin care product typically may include at least one active ingredient for the treatment or prevention of skin ailments, providing a cosmetic effect, or for providing a moisturizing benefit to skin, such as zinc oxide, petrolatum, white petrolatum, mineral oil, cod liver oil, lanolin, dimethicone, hard fat, vitamin A, allantoin, calamine, kaolin, glycerin, or colloidal oatmeal, and combinations of these. A skin care product may include one or more natural moisturizing factors such as ceramides, hyaluronic acid, glycerin, squalane, amino acids, cholesterol, fatty acids, triglycerides, phospholipids, glycosphingolipids, urea, linoleic acid, glycosaminoglycans, mucopolysaccharide, sodium lactate, or sodium pyrrolidone carboxylate, for example. Other ingredients that may be included in a skin care product include, without limitation, glycerides, apricot kernel oil, canola oil, squalane, squalene, coconut oil, corn oil, jojoba oil, jojoba wax, lecithin, olive oil, safflower oil, sesame oil, shea butter, soybean oil, sweet almond oil, sunflower oil, tea tree oil, shea butter, palm oil, cholesterol, cholesterol esters, wax esters, fatty acids, and orange oil. A skin care product can be an ointment, lotion, or sanitizer (e.g., hand sanitizer) in some aspects.

A personal care product herein can also be in the form of makeup, lipstick, mascara, rouge, foundation, blush, eyeliner, lip liner, lip gloss, other cosmetics, sunscreen, sun block, nail polish, nail conditioner, bath gel, shower gel, body wash, face wash, lip balm, skin conditioner, cold cream, moisturizer, body spray, soap, body scrub, exfoliant, astringent, scruffing lotion, depilatory, permanent waving solution, antidandruff formulation, antiperspirant composition, deodorant, shaving product, pre-shaving product, after-shaving product, cleanser, skin gel, rinse, dentifrice composition, toothpaste, or mouthwash, for example. An example of a personal care product (e.g., a cleanser, soap, scrub, cosmetic) comprises a carrier or exfoliation agent (e.g., jojoba beads [jojoba ester beads]) (e.g., about 1-10, 3-7, 4-6, or 5 wt%); such an agent may optionally be dispersed within the product.

A personal care product in some aspects can be a hair care product. Examples of hair care products herein include shampoo, hair conditioner (leave-in or rinse-out), cream rinse, hair dye, hair coloring product, hair shine product, hair serum, hair anti-frizz product, hair split-end repair product, mousse (e.g., hair styling mousse), hair spray (e.g., hair styling spray), and styling gel (e.g., hair styling gel). A hair care product can be in the form of a liquid, paste, gel, solid, or powder in some embodiments. A hair care product as presently disclosed typically comprises one or more of the following ingredients, which are generally used to formulate hair care products: anionic surfactants such as polyoxyethylenelauryl ether sodium sulfate; cationic surfactants such as stearyltrimethylammonium chloride and/or distearyltrimethylammonium chloride; nonionic surfactants such as glyceryl monostearate, sorbitan monopalmitate and/or polyoxyethylenecetyl ether; wetting agents such as propylene glycol, 1,3-butylene glycol, glycerin, sorbitol, pyroglutamic acid salts, amino acids and/or trimethylglycine; hydrocarbons such as liquid paraffins, petrolatum, solid paraffins, squalane and/or olefin oligomers; higher alcohols such as stearyl alcohol and/or cetyl alcohol; superfatting agents; antidandruff agents; disinfectants; anti-inflammatory agents; crude drugs; water-soluble polymers such as methyl cellulose, hydroxycellulose and/or partially deacetylated chitin; antiseptics such as paraben; ultra-violet light absorbers; pearling agents; pH adjustors; perfumes; and pigments.

A composition in some aspects can be a hair care composition such as a hair styling or hair setting composition (e.g., hair spray, hair gel or lotion, hair mousse/foam) (e.g., aerosol hair spray, non-aerosol pump-spray, spritze, foam, crème, paste, non-runny gel, mousse, pomade, lacquer, hair wax). A hair styling/setting composition/formulation that can be adapted to include at least one alpha-glucan derivative and insoluble alpha-glucan herein can be as disclosed in, for example, US20090074697, WO1999048462, US20130068849, JPH0454116A, US5304368, AU667246B2, US5413775, US5441728, US5939058, JP2001302458A, US6346234, US20020085988, US7169380, US20090060858, US20090326151, US20160008257, WO2020164769, or US20110217256. A hair care composition such as a hair styling/setting composition can comprise one or more ingredients/additives as disclosed in any of the foregoing references, and/or one or more of a fragrance/perfume, aroma therapy essence, herb, infusion, antimicrobial, stimulant (e.g., caffeine), essential oil, hair coloring, dying or tinting agent, anti-gray agent, anti-foam agent, sunscreen/UV-blocker (e.g., benzophenone-4), vitamin, antioxidant, surfactant or other wetting agent, mica, silica, metal flakes or other glitter-effect material, conditioning agent (e.g., a volatile or non-volatile silicone fluid), anti-static agent, opacifier, detackifying agent, penetrant, preservative (e.g., phenoxyethanol, ethylhexylglycerin, benzoate, diazolidinyl urea, iodopropynyl butylcarbamate), emollient (e.g., panthenol, isopropyl myristate), rheology-modifying or thickening polymer (e.g., acrylates/methacrylamide copolymer, polyacrylic acid [e.g., CARBOMER]), emulsified oil phase, petrolatum, fatty alcohols, diols and polyols, emulsifier (e.g., PEG-40 hydrogenated castor oil, Oleth-20), humectant (e.g., glycerin, caprylyl glycol), silicone derivative, protein, amino acid (e.g., isoleucine), conditioner, chelant (e.g., EDTA), solvent (e.g., see below), monosaccharide (e.g., dextrose), disaccharide, oligosaccharide, pH-stabilizing compound (e.g., aminomethyl propanol), film former (e.g., acrylates/hydroxyester acrylate copolymer, polyvinylpyrrolidone/vinyl acetate copolymer, triethyl acetate), aerosol propellant (e.g., C₃-C₅ alkane such as propane, isobutane, or n-butane, monoalkyl ether, dialkyl ether such as di(C₁-C₄ alkyl) ether [e.g., dimethyl ether]), and/or any other suitable material herein.

The total content of one or more alpha-glucan derivatives in a hair care composition such as a hair styling/setting composition herein can be about, at least about, or less than about, 0.5, 1, 2, 3, 4, 5, 6, 7, 8, 9, 10, 11, 12, 13, 14, 15, 0.5-15, 0.5-10, 0.5-5, 0.5-2, 1-15, 1-10, 1-5, 1-2, 2.5-7.5, 3-7, or 4-6 wt%, for example. A hair styling/setting composition can comprise a solvent comprising water and optionally a water-miscible (typically polar) organic compound (e.g., liquid or gas) such as an alcohol (e.g., ethanol, propanol, isopropanol, n-butanol, iso-butanol, tert-butanol), an alkylene glycol alkyl ether, and/or a monoalkyl or dialkyl ether (e.g., dimethyl ether), for example. If an organic compound is included, it can constitute about 10%, 20%, 30%, 40%, 50%, or 60% by weight or volume of the solvent (balance is water), for example. The amount of solvent in a hair styling/setting composition herein can be about 50-90, 60-90, 70-90, 80-90, 50-95, 60-95, 70-95, 80-95, or 90-95 wt%, for example.

An example of a hair styling gel formulation herein can comprise about 90-95 wt% (e.g., ~92 wt%) solvent (e.g., water), 0.3-1.0 wt% (e.g., ~0.5 wt%) thickener (e.g., polyacrylic acid), 0.1-0.3 wt% (e.g., ~0.2 wt%) chelant (e.g., EDTA) (optional), 0.2-1.0 wt% (e.g., ~0.5 wt%) humectant (e.g., glycerin), 0.01-0.05 wt% (e.g., ~0.02 wt%) UV-blocker (e.g., benzophenone-4) (optional), 0.05-0.3 wt% (e.g., ~0.1 wt%) preservative (e.g., diazolidinyl urea) (optional), 0.5-1.2 wt% (e.g., ~0.8 wt%) emulsifier (e.g., Oleth-20), 0.1-0.3 wt% (e.g., ~0.2 wt%) fragrance/perfume (optional), 0.2-1.0 wt% (e.g., ~0.5 wt%) pH-stabilizing compound (e.g., aminomethyl propanol), and a suitable amount of alpha-glucan derivative herein for providing antimicrobial activity.

An example of a hair styling spray formulation herein can comprise about 0.2-1.0 wt% (e.g., ~0.5 wt%) pH-stabilizing compound (e.g., aminomethyl propanol), 0.1-0.3 wt% (e.g., ~0.2 wt%) fragrance/perfume (optional), 0.05-0.12 wt% (e.g., ~0.08 wt%) surfactant (e.g., ethoxylated dimethicone polyol), 0.05-0.12 wt% (e.g., ~0.08 wt%) conditioner (e.g., cyclomethicone) (optional), 0.05-0.3 wt% (e.g., ~0.2 wt%) preservative (e.g., sodium benzoate) (optional), 15-20 wt% (e.g., ~17 wt%) water, 30-40 wt% (e.g., ~65 wt%) alcohol (e.g., ethanol), 40-60 wt% (e.g., ~45 wt%) propellant (e.g., dimethyl ether, or a ~2:1 mix of dimethyl ether to C₃-C₅ alkane [e.g., mix of propane and isobutane]), and a suitable amount of alpha-glucan derivative herein for providing antimicrobial activity.

A composition in some aspects can be a hair care composition such as a hair conditioner. Suitable examples of ingredients that can be in a hair conditioner, in addition to at least an alpha-glucan derivative herein, include cationic polymers (e.g., cationized guar gum, diallyl quaternary ammonium salt/acrylamide copolymers, quaternized polyvinylpyrrolidone and derivatives thereof, various polyquaternium-compounds); cationic surfactants (e.g., stearalkonium chloride, centrimonium chloride, sapamin hydrochloride); fatty alcohols (e.g., behenyl alcohol); fatty amines (e.g., stearyl amine); waxes; esters; nonionic polymers (e.g., polyvinylpyrrolidone, polyvinyl alcohol, polyethylene glycol); silicones; siloxanes (e.g., decamethylcyclopentasiloxane); polymer emulsions (e.g., amodimethicone); and nanoparticles (e.g., silica nanoparticles, polymer nanoparticles). A hair conditioner composition/formulation that can be adapted to include at least one alpha-glucan derivative herein can be, for example, as disclosed in any of U.S. Patent Appl. Publ. Nos. 2003/0125224, 2005/0180941, 2008/0112912, 20080311067, 2011/0150810, or 2019/0125645, U.S. Patent Nos. 5876705 or 6221817, or Int. Patent Appl. Publ. No. WO2006/010441.

Various examples of personal care formulations comprising at least one alpha-glucan derivative as presently disclosed are disclosed below (1-3).
(1) A hair conditioner composition comprising: cetyl alcohol (1-3%), isopropyl myristate (1-3%), hydroxyethyl cellulose (Natrosol^{®} 250 HHR, 0.1-1%), alpha-glucan derivative (0.1-2%), potassium salt (0.1-0.5%), Germaben^{®} II preservative (0.5%, available from International Specialty Products), and the balance being water.
(2) A hair shampoo composition comprising: 5-20% sodium laureth sulfate (SLES), 1-2 wt% cocamidopropyl betaine, 1-2 wt% sodium chloride, 0.1-2% alpha-glucan derivative, preservative (0.1-0.5%), and the balance being water.
(3) A skin lotion composition comprising: 1-5% glycerin, 1-5% glycol stearate, 1-5% stearic acid, 1-5% mineral oil, 0.5-1% acetylated lanolin (Lipolan^{®} 98), 0.1-0.5 cetyl alcohol, 0.2-1% triethanolamine, 0.1-1 wt% Germaben^{®} II preservative, 0.5-2 wt% alpha-glucan derivative, and the balance being water.
A suitable content of insoluble alpha-glucan as disclosed herein can be used in any of formulations of (1)-(3), for example and are present in aspects of the invention.

Some aspects of the present disclosure regard hair that has been treated with a hair care composition herein (e.g., hair styling/setting composition, shampoo, conditioner). For example, hair can comprise an alpha-glucan derivative and insoluble alpha-glucan on its surface (e.g., adsorbed or otherwise deposited on hair surface); optionally, one or more other ingredients of a hair care composition herein can also be present.

A pharmaceutical product herein can be in the form of an emulsion, liquid, elixir, gel, suspension, solution, cream, or ointment, for example. Also, a pharmaceutical product herein can be in the form of any of the personal care products disclosed herein, such as an antibacterial or antifungal composition. A pharmaceutical product can further comprise one or more pharmaceutically acceptable carriers, diluents, and/or pharmaceutically acceptable salts. A composition herein can also be used in capsules, tablets, tablet coatings, and as an excipients for medicaments and drugs.

A household and/or industrial product herein can be in the form of drywall tape-joint compounds; mortars; grouts; cement plasters; spray plasters; cement stucco; adhesives; pastes; wall/ceiling texturizers; binders and processing aids for tape casting, extrusion forming, injection molding and ceramics; spray adherents and suspending/dispersing aids for pesticides, herbicides, and fertilizers; fabric care products such as fabric softeners and laundry detergents; hard surface cleaners; air fresheners; emulsions (e.g., polymer emulsions); latex; gels such as water-based gels; surfactant solutions; paints such as water-based paints; protective coatings; adhesives; sealants and caulks; inks such as water-based ink; metal-working fluids; films or coatings; or emulsion-based metal cleaning fluids used in electroplating, phosphatizing, galvanizing and/or general metal cleaning operations, for example.

Some aspects of the present disclosure regard products, such as some personal care products, medical care products, industrial products, household care products and other products, that can take advantage of the antimicrobial activity that an alpha-glucan derivative in some aspects can impart to the surface of a material (e.g., a material to which the derivative has been adsorbed). Yet, some aspects herein regard products, such as some personal care products, medical care products, industrial products, household care products and other products, that can take advantage of any other feature/activity that an alpha-glucan derivative herein can impart to the surface of a material (e.g., fabric softening, soil anti-redeposition onto fabric).

Examples of personal care products on which an alpha-glucan derivative and insoluble alpha-glucan herein can be adsorbed or otherwise incorporated (and thereby have antimicrobial properties in some aspects) include absorbent personal hygiene products such as baby diapers, potty training pants/liners, incontinence products (e.g., pads, adult diapers), and feminine hygiene products (e.g., sanitary napkins/pads, tampons, interlabial products, panty liners). Thus, a personal care product in some aspects can be characterized as a personal care absorbent article that can be placed against or near the skin or other tissue to absorb and contain a fluid discharged or emitted from the body. Additional personal care products (regardless of absorbency ability) that can benefit from surface treatment with an alpha-glucan derivatives herein include various woven or non-woven fabric products (e.g., wipes, towelettes, cosmetic wipes/pads, any of the foregoing absorbent products). Examples of personal care products that can be adapted accordingly, or treated accordingly, to take advantage of a composition herein comprising an alpha-glucan derivative and insoluble alpha-glucan (e.g., rendered to have antimicrobial activity) are disclosed in WO1999/037261, U.S. Patent Appl. Publ. Nos. 2004/0167491, 2009/0204091, 2001/0014797, 2013/0281949, 2002/0087138, 2010/0241098, 2011/0137277 and 2007/0287971, and U.S. Patent Nos. 4623339, 2627858, 3585998, 3964486, 6579273, 6183456, 5820619, 4846824, 4397644, 4079739, 8987543, 4781713, 5462539, 8912383, 3749094, 3322123, 4762521 and 5342343.

Examples of medical care products on which an alpha-glucan derivative and insoluble alpha-glucan herein can be adsorbed or otherwise incorporated (and thereby have antimicrobial properties in some aspects) include wound healing dressings (e.g., bandages, surgical pads); swabs and gauze; medical tape or wrap; cast paddings and covers; sponges and wipes; heat/cold packs; sterile packaging; ostomy belt; hospital bed/pillow sheets and mattresses; sanitary towels/pads; controlled drug release devices; cell immobilization islets; bioactive scaffolds for regenerative medicine; stomach bulking devices; personal and/or patient protective equipment (e.g., medical scrubs, gowns, face masks, gloves, shoes, shoe covers, hosiery, caps, drapes); medical shrouds; and medical filters. A medical care product that can benefit from surface treatment with an alpha-glucan derivative herein can comprise woven or non-woven fabric, for example. Examples of medical care products that can be adapted accordingly, or treated accordingly, to take advantage of a composition herein comprising an alpha-glucan derivative and insoluble alpha-glucan (e.g., rendered to have antimicrobial activity) are disclosed in WO1998/046159, U.S. Patent Appl. Publ. Nos. 2005/0256486, 20030070232 and 20040128764, and U.S. Patent Nos. 6191341, 7732657, 4925453, 9161860, 3187747 and 5701617.

Examples of industrial products and other products on which an alpha-glucan derivative and insoluble alpha-glucan herein can be adsorbed or otherwise incorporated (and thereby have antimicrobial properties in some aspects) include cable wrappings (e.g., wrappings for power or telecommunication cables); food pads; paddings and foams; post-mortem shrouds; agricultural and forestry applications such as for retaining water in soil and/or to release water to plant roots; seed strips; filter media (e.g., for HVAC or breathing apparatuses); packaging; wipes (e.g., cleaning wipes); apparel linings; upholstery; house/building wraps and roofing undercoverings; insulation; mops and sponges; dish drying mat; carpet and carpet backing; door rugs and mats; reusable bags; natural and artificial leather; natural and artificial hair/fur; and pet/animal bedding and clothing. Examples of industrial products that can be adapted accordingly, or treated accordingly, to take advantage of a composition herein comprising an alpha-glucan derivative and insoluble alpha-glucan (e.g., rendered to have antimicrobial activity) are disclosed in U.S. Patent Appl. Publ. Nos. 2002/0147483, 2006/0172048, 20050008737, 2008/0199577, 2012/0328723 and 2004/0074271, and U.S. Patent Nos. 5906952, 7567739, 5176930, 6695138, 4865855, 7459501, 5456733, 9089730, 5849210, 7670513, 7670513, 5683813, 5342543, 4840734 and 4894179.

Additional examples of personal care, household care, and other products and ingredients herein can be any as disclosed in U.S. Patent No. 8796196. Examples of personal care, household care, and other products and ingredients herein include perfumes, fragrances, air odor-reducing agents, insect repellents and insecticides, bubble-generating agents such as surfactants, pet deodorizers, pet insecticides, pet shampoos, disinfecting agents, hard surface (e.g., floor, tub/shower, sink, toilet bowl, door handle/panel, glass/window, car/automobile exterior or interior) treatment agents (e.g., cleaning, disinfecting, and/or coating agents), wipes and other non-woven materials, colorants, preservatives, antioxidants, emulsifiers, emollients, oils, medicaments, flavors, and suspending agents.

Some aspects of the present disclosure are drawn to producing an aqueous composition herein that comprises at least one alpha-glucan derivative and at least one insoluble alpha-glucan. For example, an alpha-glucan derivative herein can be dissolved into an aqueous dispersion of an insoluble alpha-glucan. Also for example, an insoluble alpha-glucan can be dispersed into an aqueous solution of an alpha-glucan derivative. As another example, both an alpha-glucan derivative and insoluble alpha-glucan can be introduced into an aqueous milieu at the same time. In some aspects of dispersing an insoluble alpha-glucan, the alpha-glucan can be "never-dried", meaning that the glucan had never been dried since its enzymatic synthesis with glucansucrase (up until the time that it is dispersed into an aqueous milieu); never-dried insoluble alpha-glucan optionally can be in the form of a wet cake, for example. Dry insoluble alpha-glucan (e.g., powder form) can be dispersed in some aspects.

Non-limiting examples of compositions and methods disclosed herein include:
Embodiment 1. A composition comprising at least: (a) a derivative of an alpha-glucan, wherein (i) at least about 40% of the glycosidic linkages of the alpha-glucan are alpha-1,6 linkages, and (ii) the alpha-glucan has a degree of substitution (DoS) of about 0.001 to about 3.0 with at least one positively charged organic group; and (b) an insoluble alpha-glucan, wherein at least about 50% of the glycosidic linkages of the insoluble alpha-glucan are alpha-1,3 glycosidic linkages, and the weight-average degree of polymerization (DPw) of the insoluble alpha-glucan is at least 10.
Embodiment 2. The composition of embodiment 1, wherein at least about 90% of the glycosidic linkages of the alpha-glucan of (a) are alpha-1,6 linkages.
Embodiment 3. The composition of embodiment 1 or 2, wherein the alpha-glucan of (a) comprises at least 1% alpha-1,2 and/or alpha-1,3 branches.
Embodiment 4. The composition of embodiment 1, 2, or 3, wherein the alpha-glucan of (a) has a weight-average molecular weight (Mw) of about 10 kDa to about 2000 kDa (e.g., about 20 kDa to about 500 kDa).
Embodiment 5. The composition of embodiment 1, 2, 3, or 4, wherein said DoS is about 0.001 to about 1.0 (e.g., about 0.05 to about 1.0).
Embodiment 6. The composition of embodiment 1, 2, 3, 4, or 5, wherein the positively charged organic group is ether-linked to the alpha-glucan (i.e., the derivative is an ether derivative).
Embodiment 7. The composition of embodiment 1, 2, 3, 4, 5, or 6, wherein the positively charged organic group comprises a C₄ to C₂₀ alkyl group (e.g., a C₁₀ to C₁₄ alkyl group, or a C₁₂ alkyl group) or C₄ to C₂₀ alkylene group (e.g., a C₁₀ to C₁₄ alkylene group, or a C₁₂ alkylene group).
Embodiment 8. The composition of embodiment 1, 2, 3, 4, 5, 6, or 7, wherein the alpha-glucan derivative has antimicrobial activity.
Embodiment 9. The composition of embodiment 1, 2, 3, 4, 5, 6, 7, or 8, wherein the positively charged organic group comprises a substituted ammonium group (optionally wherein the substituted ammonium group optionally comprises a quaternary ammonium group, further optionally wherein the quaternary ammonium group comprises two methyl groups and a C₄ to C₂₀ alkyl group [e.g., a C₁₀ to C₁₄ alkyl group, or a C₁₂ alkyl group] or a C₄ to C₂₀ alkylene group [e.g., a C₁₀ to C₁₄ alkylene group, or a C₁₂ alkylene group]).
Embodiment 10. The composition of embodiment 1, 2, 3, 4, 5, 6, 7, 8, or 9, wherein at least about 90% of the glycosidic linkages of the insoluble alpha-glucan are alpha-1,3 glycosidic linkages.
Embodiment 11. The composition of embodiment 1, 2, 3, 4, 5, 6, 7, 8, 9, or 10, wherein the DPw of the insoluble alpha-glucan is at least about 200 (e.g., at least about 400).
Embodiment 12. The composition of embodiment 1, 2, 3, 4, 5, 6, 7, 8, 9, 10, or 11, wherein the composition is an aqueous composition (typically wherein the alpha-glucan of [a] is dissolved in the aqueous composition, and the insoluble alpha-glucan is dispersed in the aqueous composition).
Embodiment 13. The composition of embodiment 1, 2, 3, 4, 5, 6, 7, 8, 9, 10, 11, or 12, wherein the composition is a household care product, personal care product, industrial product, medical product, or pharmaceutical product.
Embodiment 14. The composition of embodiment 1, 2, 3, 4, 5, 6, 7, 8, 9, 10, 11, 12, or 13, wherein the composition further comprises a fiber-containing material/article, and the alpha-glucan derivative and the insoluble alpha-glucan are adsorbed to the fiber-containing material/article or otherwise incorporated in the fiber-containing material/article (or otherwise incorporated into/onto the surface of the fiber-containing material/article, or otherwise bound to the surface of the fiber-containing material/article), optionally wherein the fiber-containing material/article is a fabric and/or has antimicrobial activity (optionally wherein the fabric is a synthetic fabric such as polyester).
Embodiment 15. A method of treating a fiber-containing material/article, the method comprising: (a) providing a composition according to embodiment 1, 2, 3, 4, 5, 6, 7, 8, 9, 10, 11, 12, or 13, and (b) contacting the composition with a fiber-containing material/article (or treating a fiber-containing material/article with the composition), wherein at least the alpha-glucan derivative and the insoluble alpha-glucan are adsorbed to the fiber-containing material/article (or otherwise incorporated into/onto the surface of the fiber-containing material/article, or otherwise bound to the surface of the fiber-containing material/article) (optionally wherein rinsing and/or drying of the fiber-containing material/article is performed immediately following step [b]) (optionally wherein the fiber-containing material/article is a synthetic fabric such as polyester).
Embodiment 16. The method of embodiment 15, further comprising: (c) subjecting the fiber-containing material/article, following step (b), to at least one cycle of washing and/or rinsing.
Embodiment 17. The method of embodiment 15 or 16, wherein the fiber-containing material/article retains at least about 50% by weight of the alpha-glucan derivative that was adsorbed to the fiber-containing material/article (or that was otherwise incorporated into/onto the surface of the fiber-containing article, or that was otherwise bound to the surface of the fiber-containing article) from step (b).
Embodiment 18. The method of embodiment 15, 16, or 17, wherein the fiber-containing material/article acquires antimicrobial activity from step (b) (and optionally at least about 50% of this antimicrobial activity is retained by the fiber-containing article if the article is subjected to at least one cycle of washing and/or rinsing).
Embodiment 19. A method of inhibiting a microbe, the method comprising: (a) providing a composition according to embodiment 14, and (b) contacting the microbe with the composition (e.g., such that the alpha-glucan derivative contacts the microbe), wherein said contacting (i) inhibits the growth and/or proliferation of the microbe, and/or (ii) inhibits the colonization of the microbe on a surface (e.g., the surface of the fiber-containing material/article).
Embodiment 20. The method of embodiment 19, wherein the microbe is selected from a bacteria, fungus/yeast, protist/algae, or enveloped virus. Embodiments 19 and 20 do not form aspects of the invention.

### EXAMPLES

The present disclosure is further exemplified in the following Examples. It should be understood that these Examples, while indicating certain aspects herein, are given by way of illustration only. From the above discussion and these Examples, one skilled in the art can ascertain the essential characteristics of the disclosed embodiments, and can make various changes and modifications to adapt the disclosed embodiments to various uses and conditions. The scope of the invention is as set forth in the appended claims.

### Materials/Methods

All fabrics were purchased from TestFabrics, Inc. (314: Double Knitted Texturized Nylon, 730: Texturized Polyester Interlock, 460-60: Bleached Cotton Interlock). Chitosan was purchased from Fisher Scientific (Cat. No. 150597). Lauroyl arginine ethyl ester (LAE) was purchased from VEDEQSA (Technical grade, lot no. 01498A). 3-chloro-2-hydroxypropyl dodecyldimethylammonium chloride (QUAB 342), 3-chloro-2-hydroxypropyl cocoalkyldimethylammonium chloride (QUAB 360), and 3-chloro-2-hydroxypropyl trimethylammonium (QUAB 188) were purchased from QUAB Chemicals. Since the alkyl group of QUAB 360 is derived from coconut oil, QUAB 360 is a mixture of chlorohydrin compounds having various alkyl groups as observed in the fatty acids of coconut oil; the relative amount of each alkyl group in QUAB 360 is similar to their relative content in coconut oil, per Table A.

**Table A. Coconut Oil Fatty Acid Content**

| Common name | Fatty acid | Percentage |
|---|---|---|
| Caproic acid | 6:0 | 0.2-0.5 |
| Caprylic acid | 8:0 | 5.4-9.5 |
| Capric acid | 10:0 | 4.5-9.7 |
| Lauric acid | 12:0 | 44.1-51 |
| Myristic acid | 14:0 | 13.1-18.5 |
| Palmitic acid | 16:0 | 7.5-10.5 |
| Stearic acid | 18:0 | 1.0-3.2 |
| Arachidic acid | 20:0 | 0.2-1.5 |
| Oleic acid | 18:1n-9 | 5.0-8.2 |
| Linoleic acid | 18:2n-6 | 1.0-2.6 |

### Representative Preparation of Alpha-1,6-Glucan with Alpha-1,2 Branching

Except as otherwise noted, all alpha-1,6-glucans in the Examples contained a linear backbone with 100% alpha-1,6 glycosidic linkages. Alpha-1,2 branches (i.e., pendant alpha-1,2-linked glucose groups) were added to such backbones. Except as otherwise noted, all reported molecular weights are weight-average and regard the alpha-1,6-glucan backbone prior to branching.

Methods to prepare alpha-1,6-glucan containing various amounts of alpha-1,2 branching are disclosed in U.S. Pat. Appl. Publ. No. 2018/0282385. Reaction parameters such as sucrose concentration, temperature, and pH can be adjusted to provide alpha-1,6-glucan having various levels of alpha-1,2-branching and molecular weight. A representative procedure for the preparation of alpha-1,2-branched alpha-1,6-glucan is provided below (containing 19% alpha-1,2-branching and 81% alpha-1,6 linkages). The 1D ¹H-NMR spectrum was used to quantify glycosidic linkage distribution. Additional samples of alpha-1,6-glucan with alpha-1,2-branching were prepared similarly. For example, one sample contained 32% alpha-1,2-branching and 68% alpha-1,6 linkages, and another contained 10% alpha-1,2-branching and 90% alpha-1,6 linkages.

Soluble alpha-1,6-glucan with about 19% alpha-1,2 branching was prepared using stepwise combination of glucosyltransferase (dextransucrase) GTF8117 and alpha-1,2 branching enzyme GTFJ18T1, according to the following procedure. A reaction mixture (2 L) comprised of sucrose (450 g/L), GTF8117 (9.4 U/mL), and 50 mM sodium acetate was adjusted to pH 5.5 and stirred at 47 °C. Aliquots (0.2-1 mL) were withdrawn at predetermined times and quenched by heating at 90 °C for 15 minutes. The resulting heat-treated aliquots were passed through a 0.45-µm filter. The flow-through was analyzed by HPLC to determine the concentration of sucrose, glucose, fructose, leucrose, oligosaccharides and polysaccharides. After 23.5 hours, the reaction mixture was heated to 90 °C for 30 minutes. An aliquot of the heat-treated reaction mixture was passed through a 0.45-µm filter and the flow-through was analyzed for soluble mono/disaccharides, oligosaccharides, and polysaccharides. A major product was linear dextran with a DPw of 93 (100% alpha-1,6 glycosidic linkages).

A second reaction mixture was prepared by adding 238.2 g of sucrose and 210 mL of alpha-1,2-branching enzyme GTFJ18T1 (5.0 U/mL) to the leftover heat-treated reaction mixture that was obtained from the GTF8117 reaction described immediately above. The mixture was stirred at 30 °C with a volume of ~2.2 L. Aliquots (0.2-1 mL) were withdrawn at predetermined times and quenched by heating at 90 °C for 15 minutes. The resulting heat-treated aliquots were passed through a 0.45-µm filter. The flow-through was analyzed by HPLC to determine the concentration of sucrose, glucose, fructose, leucrose, oligosaccharides and polysaccharides. After 95 hours, the reaction mixture was heated to 90 °C for 30 minutes. An aliquot of the heat-treated reaction mixture was passed through a 0.45-µm filter and the flow-through was analyzed for soluble mono/disaccharides, oligosaccharides, and polysaccharides. Leftover heat-treated mixture was centrifuged using 1-L centrifugation bottles. The supernatant was collected and cleaned more than 200-fold using an ultrafiltration system with 1- or 5-kDa MWCO cassettes and deionized water. The cleaned oligo/polysaccharide product solution was dried. Dry sample was then analyzed by ¹H-NMR spectroscopy to determine the anomeric linkages of the oligosaccharides and polysaccharides.

### Representative Preparation of Alpha-1,6-Glucan Cationic Ether Compounds

Cationic alpha-1,6-glucan ether compounds were prepared. In particular, (A) trimethylammonium hydroxypropyl alpha-1,2-branched alpha-1,6-glucan ("Glucan C1 Quat"), (B) dodecyldimethylammonium hydroxypropyl alpha-1,2-branched alpha-1,6-glucan ("Glucan C12 Quat"), and (C) cocoalkyldimethylammonium hydroxypropyl poly alpha-1,6-glucan ("Glucan C6-C18 Quat", or "Glucan C8-C18 Quat" as there could be few or no C6 alkyl groups represented in the cocoalkyl portion of this heterogeneous compound; see Table A) were prepared as disclosed below:
A. Polysaccharide solution (43% solids, 73 g; alpha-1,6-glucan with 20% alpha-1,2-branching and 80% alpha 1,6 linkages, Mw 60 kDa) was charged into a 22-L reactor equipped with an overhead stirrer. To the stirring solution was added 27.2 g of 50% NaOH solution. The preparation was heated to 50 °C, after which 76 g of a 65% solution of 3-chloro-2-hydroxypropyltrimethylammonium chloride (QUAB 188) was added with an additional funnel over 2 hours and 45 minutes. The resulting reaction was then kept at 58 °C for 3 hours. The reaction was then diluted with water (500 mL) and neutralized with 18 wt% HCl. The product was purified by ultrafiltration (10-kDa membrane) and freeze-dried. The degree of substitution (DoS) of the product with trimethylammonium hydroxypropyl groups (Glucan C1 Quat) was determined to be 0.3 by ¹H NMR.
B. A 4-neck, 500-mL reactor equipped with a mechanical stir rod, thermocouple, and addition funnel was charged with 80 g of a preparation containing 32 g of 3-chloro-2-hydroxypropyl dodecyldimethylammonium chloride (QUAB 342) and 48 g water. A glucan solution (alpha-1,6-glucan with 20% alpha-1,2-branching and 80% alpha 1,6 linkages, Mw 60 kDa, 21 g glucan in 60 mL water) was charged to a separate 500-mL reactor and heated to 55 °C. 3-chloro-2-hydroxypropyl dodecyldimethylammonium chloride from the other flask was slowly added to the reactor with the glucan solution over 10 minutes. Sodium hydroxide (10 g, 50 wt%) was then added over a 10-minute period. Water (10 mL) was then added. The reaction preparation was heated to 60 °C (10 min) and stirred at 58-60 °C for 3.5 hours. After being cooled to 35 °C, the reaction was poured into water to a total volume of about 2 L. The pH of the preparation was adjusted to about 7 by addition of 18.5 wt% HCl. The preparation was filtered and no solid was observed in the filter. The product was purified from the filtrate by ultra-filtration (10-kDa membrane), and then freeze-dried. The DoS of the product with dodecyldimethylammonium hydroxypropyl groups (Glucan C12 Quat) was determined to be 0.1 by ¹H NMR.
C. A 4-neck, 500-mL reactor equipped with a mechanical stir rod, thermocouple, and addition funnel was charged with 142 g of a preparation containing 56.8 g of 3-chloro-2-hydroxypropyl cocoalkyldimethylammonium chloride (QUAB 360) and 85.2 g water. A glucan solution (alpha-1,6-glucan with 20% alpha-1,2-branching and 80% alpha 1,6 linkages, Mw 40 kDa, 21 g of glucan in 60 mL water) was charged to a separate 500-mL reactor and heated to 55 °C and heated to 55 °C. 3-chloro-2-hydroxypropyl cocoalkyldimethylammonium chloride from the other flask was slowly added to the reactor with the glucan solution over 10 minutes. Sodium hydroxide (15.8 g, 50 wt%) was then added over a 10-minute period. Water (10 mL) was then added. The reaction preparation was heated to 60 °C (10 min) and stirred at 58-60 °C for 3.5 hours. After being cooled to 35 °C, the reaction was poured into water to a total volume of about 2 L. The pH of the preparation was adjusted to about 7 by addition of 18.5 wt% HCl. The preparation was filtered and no solid was observed in the filter. The product was purified from the filtrate by ultra-filtration (10-kDa membrane), and then freeze-dried. The DoS of the product with cocoalkyldimethylammonium hydroxypropyl groups (Glucan C8-C18 Quat) was determined to be 0.16 by ¹H NMR.

### Procedure 1. Treating Fabric with Functionalized Alpha-1,6-Glucan

(Procedure 1A) Exhaust Method: Alpha-1,6-glucan ether compounds (e.g., as prepared in Materials/Methods) were exhaust-applied using Werner Mathis AG BFA-12 exhaust equipment. Individual formulation baths comprising desired concentrations of a glucan ether polymer in water were prepared. Each fabric sample (20 g) was loosely folded horizontally and placed into a test canister, followed by addition of 200 mL of a bath solution. The canisters were then closed and secured to the rotating wheel of the equipment. The rotation speed of the wheel was set to 35 rpm, switching from clockwise to counterclockwise modes at 1-minute intervals. Samples were exhausted using a program with preset parameters, such as temperature and duration as given in Table 1. In each case, the instrument, with the samples already loaded, would first heat up at a selected rate of heating until the target temperature was reached, maintaining the temperature for preset durations, before cooling down to initial temperature. Once the exhaustion process was complete, all the fabric samples were removed from the exhaust canisters and passed through padding equipment (Fanyuan Instruments, DW2010A) set to a pressure of 8.4 kg to squeeze out the excess water. Lastly, each sample was dried in a MATHIS lab dryer LTE for 5 minutes at 120 °C.

**Table 1. Exhaust Conditions**

| Fabric Type | Application Duration (min) | Application Temperature (°C) |
|---|---|---|
| Cotton | 20 | 30 |
| Polyester | 20 | 50 |
| Nylon | 20 | 50 |

(Procedure 1B) Padding Method: Alpha-1,6-glucan ether compounds (e.g., as prepared in Materials/Methods) were also applied to fabric samples via a padding process using a MATHIS HF or Fanyuan Instruments (DW2010A) padder. Dry fabric was submerged into a bath containing an aqueous solution of glucan ether compound at a desired concentration in water and passed through padder rollers to squeeze out (applied pressure equaled 3.1 bar) excess treatment solution. Each fabric sample was dried and cured in a MATHIS lab dryer LTE for 5 minutes at 120 °C. Each glucan solution was prepared to account for water weight pick-up by each fabric type and their exhaust potential. The antimicrobial performance of padding-treated fabrics herein was the same as that of exhaustion-treated fabrics (data not shown). Thus, the padding process represents an alternative method for treating fabrics with alpha-1,6-glucan ether compounds herein for antimicrobial finishing.

### Procedure 2. Testing the Antibacterial Efficacy of Functionalized Alpha-1,6-Glucan

(Procedure 2A) ASTM E3160-18: In some cases, an alpha-1,6-glucan ether compound herein was considered to be antimicrobially efficient if it imparted a >3 log₁₀ reduction of bacterial growth as tested by the American Society for Testing and Materials (ASTM) method E3160-18 (*Quantitative Evaluation of the Antibacterial Properties of Porous Antibacterial Treated Articles*) at a use level ≤5000 ppm. The ASTM E3160 method was performed as follows: The day before the test, a culture of *Escherichia coli* of the American Type Culture Collection (ATCC) 25922 was inoculated by transferring a single colony from tryptic soy agar (TSA), using a sterile loop, into a tryptic soy broth (TSB). On the day of the experiment (next day), each fabric sample (treated with an alpha-1,6-glucan ether herein or a control compound, e.g., prepared by Procedure 1A above) was cut into three 0.40-g swatches (about 1 x 1 inch). Each fabric swatch was placed into a 50-mL sterile specimen cup. On the same day, bacterial/working inoculum was prepared by diluting the overnight culture of *E. coli* into phosphate buffer supplemented with TSB (1:500) and 0.05% TRITON X-100 to obtain a bacterial concentration that was between 2.5 × 10⁵ colony forming units (CFU)/mL and 1.0 × 10⁶ CFU/mL, with a target concentration of 6 × 10⁵ CFU/mL. All samples were inoculated by applying 0.2 mL of working inoculum in several aliquots directly onto the fabric surface, and were allowed to incubate for 24 hours at 37 °C. Samples were neutralized with 10 mL Dey-Engley (D-E) neutralizing buffer and enumerated in triplicate in 96-well plates containing 180 µL TSB by transferring 20 µL of neutralized sample to the top well of the 96-well plates and serially diluting 20 µL down the plate. Each sample plate was placed in a plastic bin to avoid evaporation and incubated for 24 hour at 37 °C. After incubation, the plates were evaluated for bacterial turbidity. The observations were recorded and calculated by most probable number (MPN) calculation to report log growth.

The treatment bath concentration demand to achieve optimal antimicrobial activity (>3 log₁₀ reduction) was determined by preparing samples with different ladders of bath concentration and testing the antimicrobial efficacy as per the method described above.

(Procedure 2B) ASTM AATCC 100: In some cases, an alpha-1,6-glucan ether compound herein was considered to be antimicrobially efficient if it imparted a >3 log₁₀ reduction of bacterial growth as tested by ASTM method AATCC 100 (*Quantitative Evaluation of the Antibacterial Properties of Porous Antibacterial Treated Articles*) at a use level ≤5000 ppm. The day before the test, cultures of *Staphylococcus aureus* and *Klebsiella pneumoniae* of the American Type Culture Collections (ATCC) 6538 and 4352, respectively, were inoculated by transferring a single colony from TSA, using a sterile loop, into TSB. On the day of the experiment (next day), each fabric sample (treated with an alpha-1,6-glucan ether herein or a control compound, e.g., prepared by Procedure 1A above) was cut into three 1-g swatches (about 2.5 x 2.5 inch). Each fabric swatch was placed into a 50-mL sterile specimen cup. On the same day, bacterial/working inoculum was prepared by diluting the overnight culture of the organism being tested into phosphate buffer supplemented with TSB (1:20) and 0.05% Triton X-100 to obtain a bacterial concentration that was between 1 × 10⁵ CFU/mL and 3 × 10⁵ CFU/mL, with a target concentration of 2 × 10⁵ CFU/mL. All samples were inoculated by applying 1 mL of working inoculum in several aliquots directly onto the fabric surface, and were allowed to incubate for 24 hours at 37 °C. Samples were neutralized with 10 mL D-E neutralizing buffer and enumerated in triplicate in 96-well plates containing 180 µL TSB by transferring 20 µL of neutralized sample to the top well of the 96-well plates and serially diluting 20 µL down the plate. Each sample plate was placed in a plastic bin to avoid evaporation and incubated for 24 hours at 37 °C. After incubation, the plates were evaluated for bacterial turbidity. The observations were recorded and calculated by MPN calculation to report log growth.

The treatment bath concentration demand to achieve optimal antimicrobial activity (>3 log₁₀ reduction) was determined by preparing samples with different ladders of bath concentration and testing the antimicrobial efficacy as per the method described above.

### Procedure 3. Wash Durability Tests for Fabric Treated with Functionalized Alpha-1,6-Glucan

ASTM E3162-18 (*Measuring the Durability of Antibacterial Agents Applied to Textiles under Simulated Home Laundering Conditions*) was performed. A Launder-Ometer^{®} (M228AA, SDL Atlas) was set to 49 °C and a wash solution consisting of 500 mL of tap water and 0.23% (v/v) TIDE detergent and 50 stainless steel balls were added to each of stainless-steel canister. Once the wash solution reached the set temperature, a 10 g swatch of nylon, polyester, or cotton test fabric (e.g., as prepared by Procedure 1) was added to a canister before a wash cycle was initiated. A wash cycle was set at 40 rpm for a duration of 45 minutes. After 45 minutes of washing, the canisters were removed and spent liquid was disposed. Each swatch sample was removed from its canister, hand-wrung and individually placed into a glass beaker, which was then filled with 500 mL of cold tap water for rinsing. Each swatch was swirled and hand-squeezed before the wash water was disposed, and the rinse process was repeated two more times. After the final rinse, each swatch sample was dried in a tumble dryer set for a normal cycle at 71 °C. This washing and drying process was repeated five to ten times, for example. One cycle of washing as described in this Procedure was equivalent to five cycles of washing in a standard washing machine (or "real time" washes/wash cycles).

### Procedure 4. Odor Sensory Testing of Textiles Biofouled with Milk

By controlling or inhibiting the growth of bacteria, the odors they produce can also be controlled. Bacteria metabolize certain proteins, fats, carbohydrates and other nutrients to generate odor, and cows' milk, which is rich in nutrients, is a good surrogate to generate fouling odor by bacteria in textile. Odor associated with bacterial growth found in the fouling of cows' milk, as well as the biofouling of raw milk in the dairy industry, is common and can occur over very short periods of time. This simple form of odor generation has been used in many instances for testing odor control in textiles. Test Method IACM 0710, which was performed in this Procedure, is a qualitative method that measures the generation of odor following the addition of a 20% solution of instant nonfat dry milk (Nestle Carnation) on fabric. In this test, odor is measured subjectively via human sensory odor panels of trained personnel.

The typical protocol for a milk test was as follows: Samples of about 0.5 gram of treated and untreated fabric (e.g., prepare by Procedure 1A) were cut and separately placed flat in sealable containers (20 mL glass vials with screw cap). About 500 µL of 20% solution of instant nonfat dry milk (Nestle Carnation) was added evenly onto each fabric sample making sure that all the milk was absorbed into the fabric without leaving any excess milk in the container. The containers were tightly sealed and placed into an incubator set to 37 °C. After 24-48 hours, the fabric sample containers lids were carefully removed and rated for the presence of odor by smelling (see rating scale below in Table 2). A treated fabric herein can be considered to pass the test if the rating after 24 hours is ≤3, per the guidelines set forth in IACM (International Antimicrobial Council) 0710.

**Table 2. Textile Odor Rating Following Milk Treatment**

| Rating | Odor Presence Description |
|---|---|
| 1 | Faint / No Odor Present - Not Offensive |
| 2 | Moderate Odor Present - Not Offensive |
| 3 | Noticeable Odor Present - Somewhat Offensive |
| 4 | Strong Odor - Offensive |
| 5 | Very Strong Odor - Extremely Offensive |

### Procedure 5. Minimum Inhibitory Concentration (MIC) Test

Antimicrobial activity assessment of alpha-1,6-glucan ether compounds herein and other compounds were carried out using a standard MIC protocol. The MIC test was performed against eleven organisms: nine bacteria, one mold, and one yeast as shown in Table 3. Each test compound was evaluated in two sets, one at 50-5000 ppm (high MIC actives) and the other at 3.125-100 ppm (low MIC actives). All active samples, with the exception of chitosan, were dissolved in DI water at a concentration of 1% w/v for the 50-500 ppm set; chitosan was instead dissolved in water acidified to pH 3.5 with hydrochloric acid. Each test compound was then serially diluted to concentrations of 0.4%, 0.2%, 0.1%, 0.04%, 0.02% and 0.01% (w/v). For the 3.125-100 ppm set, all test compounds were prepared similarly at a concentration 0.1% w/v, and then serially diluted to concentrations of 0.05%, 0.025%, 0.0125%, 0.00625% and 0.003125% (w/v). Bacteria inocula were prepared by diluting overnight cultures 1:20 in trypticase soy broth media (TSB). Yeast and mold were prepared by diluting overnight culture or monthly stock (1×10⁸ cells per mL) 1:20 in potato dextrose broth media (PDB). 96-well plates were used in this test. First, 180 µL of plating media was added to each well. Next, each well received 20 µL water (control) or test compound at a specified concentration according to concentration range to be tested. Each sample was tested in triplicate. Lastly, each well received 20 µL of bacteria or fungi inoculum. Bacterial plates were incubated at 30 °C for 24 hours. Yeast and mold plates were incubated at 25 °C; yeast plates were incubated for 24 hours and mold plates were incubated for 7 days.

**Table 3. Microorganisms Used in MIC Study**

| Microorganism | ATCC No. |
|---|---|
| *Burkholderia cepacia* | 25416 |
| *Enterobacter gergoviae* | 33028 |
| *Escherichia coli* | 8739 |
| *Klebsiella pneumoniae* | 13883 / 4352 |
| *Pseudomonas aeruginosa* | 9027 |
| *Pseudomonas aeruginosa* | 15442 |
| *Pseudomonas putida* | 49128 |
| *Staphylococcus aureus* | 6538 |
| *Staphylococcus epidermidis* | 12228 |
| *Candida albicans* | 10231 |
| *Aspergillus brasiliensis* | 16404 |

### Procedure 6. Modified EPA Method (MB-27-03) for Disinfection Efficacy

EPA SOP No. MB-27-03 (*Standard Operating Procedure for Germicidal and Detergent Sanitizing Action of Disinfectants Tests*)*,* a quantitative method for measuring the bactericidal activity of a compound, was modified to test the disinfectant potential of alpha-1,6-glucan ether compounds against *P*. *aeruginosa* (ATCC 9027) and S. *aureus* (ATCC 6538). The MB-27-03 procedure as adapted here is described below.

Inoculum and Buffer Preparations: Freshly prepared overnight bacterial cultures were plated on tryptic soy agar plates to verify exact inoculation concentrations. About 1 mL of neat culture was serially diluted from 1:10 to 1:10⁷ and the last three serial dilutions (1:10⁵- 1.10⁷) for each bacterial concentration were subsequently plated and the bacterial growth was monitored the next day. For each species, the bacterial culture sample yielding approximately 30-300 colonies on the 1:10⁷ dilution was used for the disinfection test. Dey and Engley (D/E) neutralizing media is known to neutralize a broad spectrum of disinfectants and antimicrobial chemicals. The toxicity of D/E neutralizer against the test organisms was determined by mixing 1 mL of overnight bacterial culture with 9 mL of the neutralizer and monitoring the neutralizer impact. Each mixture was serially diluted, the last three serial dilutions (1:10⁵- 1:10⁷) were subsequently plated, and the bacterial growth was monitored the next day. A neutralizer toxicity control level was determined, which was the amount of neutralizer demonstrated to not largely impact the recovery of the test organism (i.e., ≤1 log₁₀ growth difference between the organism grown with neutralizer and the organism grown without the neutralizer). The Titer Control used in the study for each organism was prepared by adding 1 mL of bacterial culture to 9 mL of phosphate-buffered saline (PBS) solution, and then serially diluting and plating the diluted culture. The last three dilutions that were plated corresponded to 1:10⁵- 1:10⁷ dilutions. The Organism Titer needed to be ≥1×10⁷ CFU/mL. Next, D/E neutralizer buffer effectiveness was tested. About 0.9 mL of the neutralization effectiveness control was added to the highest concentration of alpha-1,6-glucan compound to be tested, vortexed for 10 seconds, 0.1 mL of bacterial culture was added, and then serially diluted and plated. The last three dilutions that were plated corresponded to 1:10⁶- 1:10⁸ dilutions. The neutralized control needed to demonstrate that the neutralizer adequately neutralized the test substance (i.e., ≤1 log₁₀ difference between the neutralized control and the neutralizer toxicity control).

Rate of Kill Evaluation: Each test compound (e.g., alpha-1,6-glucan ethers herein) was diluted to 100 ppm and 1000 ppm in sterile water. Each test sample was then divided into 9 mL aliquots in sterile test tubes. At time zero, 1 mL of test bacteria culture was added to each test tube and the time was noted. At selected timepoints, 1 mL was taken from each sample tube, transferred into a tube containing 9 mL of D/E neutralizer buffer, followed by vortexing for 10 seconds. This corresponded to the 10⁻¹ dilution tube. Next, 1 mL of the neutralized sample was transferred to 9 mL of PBS solution resulting in a 10⁻² dilution, which was then further diluted to a 10⁻³ dilution. Finally, 0.1 mL of each of these diluted samples (10⁻¹, 10⁻², 10⁻³) were plated onto TSA containing D/E neutralizer. To have been considered a test compound, a compound must have demonstrated a ≥1.0×10⁵ CFU kill of both the Gram-positive (*S. aureus*) and Gram-negative (*P*. *aeruginosa)* test organisms at the stated contact times (i.e., a ≥5 log₁₀ reduction of test organism). When TNTC (too numerous to count) values were observed for each dilution tested, a 200 value was substituted for the TNTC value at the highest (most dilute) dilution and scaled up accordingly for the calculations. For a test compound to be considered efficacious, a mean Log₁₀ Reduction ≥5 was required with a contact time of 10 minutes or less.

### Procedure 7. Delivery of Antimicrobial Polymer through Laundry Cycle

The utility of Glucan C12 Quats as potential antimicrobial polymers was tested following the protocol described below. Each Glucan C12 Quat was delivered during the rinse cycle either with a perfume-free fabric softener and/or a fragrance base. It was shown that, at 50 ppm concentration (based on total water in rinse cycle), a Glucan C12 Quat provided antimicrobial protection to the washed fabrics. This alpha-1,6-glucan ether compound worked efficiently both on natural (cotton) and synthetic (polyester) fabric types.

Perfume- and dye-free DOWNY fabric softener was used. About 0.33 g of fabric softener was mixed with 1.4 mL of 3.78% test compound solution; this mix was delivered during rinse cycle. Washing and rinsing were performed at 1-L scale using a Launder-Ometer^{®}. The fabric samples were washed using TIDE detergent and the test compound was delivered along with fabric softener during rinse cycle. Fabric was dried following this washing and rinsing. The fabric softener was tested for any antimicrobial property by the MIC study (Procedure 5). Since fabric softener was used at 300 ppm concentration in rinse, the MIC study maximum concentration was set at 1000 ppm. It was found that the MIC of fabric softener was >1000 ppm; there was no inhibition of Gram-positive or Gram-negative bacteria at the use level (300 ppm). A Glucan C12 Quat can optionally be delivered with a fragrance base (in addition to, or in substitution of, fabric softener) following the same protocol described above.

### RESULTS / EXAMPLES 1-20

### Key: Examples 1-20 and Comparatives 1-13

Example 1: Cotton fabric sample treated with 250 ppm aqueous solution of dodecyldimethylammonium hydroxypropyl alpha-1,6-glucan (glucan portion Mw 200 kDa, 20% alpha-1,2 branching; DoS 0.11).

Example 2: Nylon fabric sample treated with 500 ppm aqueous solution of dodecyldimethylammonium hydroxypropyl alpha-1,6-glucan (glucan portion Mw 200 kDa, 20% alpha-1,2 branching; DoS 0.11).

Example 3: Polyester fabric sample treated with 500 ppm aqueous solution of dodecyldimethylammonium hydroxypropyl alpha-1,6-glucan (glucan portion Mw 200 kDa, 20% alpha-1,2 branching; DoS 0.11).

Example 4: Cotton fabric sample treated with 250 ppm aqueous solution of cocoalkyldimethylammonium hydroxypropyl alpha-1,6-glucan (glucan portion Mw 200 kDa, 20% alpha-1,2 branching; DoS 0.11).

Example 5: Dodecyldimethylammonium hydroxypropyl alpha-1,6-glucan (glucan portion Mw 200 kDa, 20% alpha-1,2 branching; DoS 0.11).

Example 6: Polyester fabric sample treated with 500 ppm aqueous solution of cocoalkyldimethylammonium hydroxypropyl alpha-1,6-glucan (glucan portion Mw 200 kDa, 20% alpha-1,2 branching; DoS 0.11).

Example 7: A 100 ppm aqueous solution of dodecyldimethylammonium hydroxypropyl alpha-1,6-glucan (glucan portion Mw 200 kDa, 20% alpha-1,2 branching; DoS 0.11) in contact with the target bacterial culture for 2.5 minutes before being neutralized with DE buffer.

Example 8: A 100 ppm aqueous solution of dodecyldimethylammonium hydroxypropyl alpha-1,6-glucan (glucan portion Mw 200 kDa, 20% alpha-1,2 branching; DoS 0.11) in contact with the target bacterial culture for 5 minutes before being neutralized with DE buffer.

Example 9: A 100 ppm aqueous solution of dodecyldimethylammonium hydroxypropyl alpha-1,6-glucan (glucan portion Mw 200 kDa, 20% alpha-1,2 branching; DoS 0.11) in contact with the target bacterial culture for 10 minutes before being neutralized with DE buffer.

Example 10: A 1000 ppm aqueous solution of dodecyldimethylammonium hydroxypropyl alpha-1,6-glucan (glucan portion Mw 200 kDa, 20% alpha-1,2 branching; DoS 0.11) in contact with the target bacterial culture for 2.5 minutes before being neutralized with DE buffer.

Example 11: A 1000 ppm aqueous solution of dodecyldimethylammonium hydroxypropyl alpha-1,6-glucan (glucan portion Mw 200 kDa, 20% alpha-1,2 branching; DoS 0.11) in contact with the target bacterial culture for 5 minutes before being neutralized with DE buffer.

Example 12: A 1000 ppm aqueous solution of dodecyldimethylammonium hydroxypropyl alpha-1,6-glucan (glucan portion Mw 200 kDa, 20% alpha-1,2 branching; DoS 0.11) in contact with the target bacterial culture for 10 minutes before being neutralized with DE buffer.

Example 13: Cotton fabric sample treated with 250 ppm aqueous solution of dodecyldimethylammonium hydroxypropyl alpha-1,6-glucan (glucan portion Mw 40 kDa, 40% alpha-1,2 branching; DoS 0.21).

Example 14: Cotton fabric sample treated with 250 ppm aqueous solution of dodecyldimethylammonium hydroxypropyl alpha-1,6-glucan (glucan portion Mw 200 kDa, 20% alpha-1,2 branching; DoS 0.20).

Example 15: Cotton fabric sample treated with 250 ppm aqueous solution of dodecyldimethylammonium hydroxypropyl alpha-1,6-glucan (glucan portion Mw 40 kDa, 40% alpha-1,2 branching; DoS 0.17).

Example 16: Cotton fabric sample treated with 250 ppm aqueous solution of dodecyldimethylammonium hydroxypropyl alpha-1,6-glucan (glucan portion Mw 200 kDa, 20% alpha-1,2 branching; DoS 0.11).

Example 17: Cotton fabric sample washed and then subjected to a rinse containing fabric softener and 50 ppm of dodecyldimethylammonium hydroxypropyl alpha-1,6-glucan (glucan portion Mw 40 kDa, 40% alpha-1,2 branching; DoS 0.21).

Example 18: Cotton fabric sample washed and then subjected to a rinse containing fragrance base and 50 ppm of dodecyldimethylammonium hydroxypropyl alpha-1,6-glucan (glucan portion Mw 40 kDa, 40% alpha-1,2 branching; DoS 0.21).

Example 19: Polyester fabric sample washed and then subjected to a rinse containing fabric softener and 50 ppm of dodecyldimethylammonium hydroxypropyl alpha-1,6-glucan (glucan portion Mw 40 kDa, 40% alpha-1,2 branching; DoS 0.21).

Example 20: Polyester fabric sample washed and then subjected to a rinse containing fragrance base and 50 ppm of dodecyldimethylammonium hydroxypropyl alpha-1,6-glucan (glucan portion Mw 40 kDa, 40% alpha-1,2 branching; DoS 0.21).

Comparative 1: Cotton fabric sample treated with 250 ppm aqueous solution of trimethylammonium hydroxypropyl alpha-1,6-glucan (glucan portion Mw 60 kDa, 20% alpha-1,2 branching; DoS 0.3).

Comparative 2: Nylon fabric sample treated with 250 ppm aqueous solution of trimethylammonium hydroxypropyl alpha-1,6-glucan (glucan portion Mw 60 kDa, 20% alpha-1,2 branching; DoS 0.3).

Comparative 3: Cotton fabric sample treated with 2000 ppm aqueous solution of AEM 5772 (72% active 3-(trihydroxysilyl)propyldimethyl-octadecyl ammonium chloride).

Comparative 4: Cotton fabric sample treated with 500 ppm aqueous solution of chitosan purchased from Fisher Scientific (Cat. No. 150597).

Comparative 5: Cotton fabric sample treated with 500 ppm aqueous solution of LAE that was purchased from VEDEQSA (Technical Grade, Lot No. 01498A).

Comparative 6: Cotton fabric sample treated with 500 ppm aqueous solution of 3-chloro-2-hydroxypropyl dodecyldimethylammonium chloride that was purchased from QUAB chemicals (QUAB 342).

Comparative 7: Cotton fabric sample treated with 500 ppm aqueous solution of dodecyltrimethylammonium chloride that was purchased from Sigma Aldrich (Cat. No. 44242).

Comparative 8: Polyester fabric sample treated with 500 ppm aqueous solution of trimethylammonium hydroxypropyl alpha-1,6-glucan (glucan portion Mw 40 kDa, 20% alpha-1,2 branching; DoS 0.3).

Comparative 9: Alpha-1,6-glucan (MW 40 kDa, 20% alpha-1,2 branching).

Comparative 10: Trimethylammonium hydroxypropyl alpha-1,6-glucan (glucan portion Mw 40 kDa, 20% alpha-1,2 branching; DoS 0.3).

Comparative 11: Dodecyltrimethylammonium chloride that was purchased from Sigma Aldrich (Cat. No. 44242).

Comparative 12: Chitosan purchased from Fisher Scientific (Cat. No. 150597).

Comparative 13: LAE purchased from VEDEQSA (Technical Grade, Lot No. 01498A).

**Table 4. Performance of Glucan C1 and C12 Quat Compounds on Cotton^{a}**

| Example^{b} | Glucan Ether Compound | Performance Parameters | | |
|---|---|---|---|---|
| | | Antimicrobial Efficacy | Wash Durability^{c} | Concentration Demand for Optimal Activity (≥3 log₁₀ reduction) |
| Example 1 | Glucan C12 Quat | >5 log₁₀ reduction | 25 real time washes | 250 ppm |
| Comparative 1 | Glucan C1 Quat | No activity | No activity | >5000 ppm |

| | | | | |
|---|---|---|---|---|
| ^{a} Compounds applied to fabric according to Procedure 1. Antimicrobial activity tested according to Procedure 2A (ASTM-E3160-18). ^{b} See key above for more details regarding Examples and Comparatives. ^{c} Procedure 3 (ASTM-E3162-18). | | | | |

**Table 5. Performance of Glucan C1 and C12 Quat Compounds on Nylon^{a}**

| Example^{b} | Glucan Ether Compound | Performance Parameters | | |
|---|---|---|---|---|
| | | Antimicrobial Efficacy | Wash Durability^{c} | Concentration Demand for Optimal Activity (≥3 log₁₀ reduction) |
| Example 2 | Glucan C12 Quat | ~4 log₁₀ reduction | 25 real time washes | 500 ppm |
| Comparative 1 | Glucan C1 Quat | No activity | No activity | >5000 ppm |

| | | | | |
|---|---|---|---|---|
| ^{a} Compounds applied to fabric according to Procedure 1. Antimicrobial activity tested according to Procedure 2A (ASTM-E3160-18). ^{b} See key above for more details regarding Examples and Comparatives. ^{c} Procedure 3 (ASTM-E3162-18). | | | | |

**Table 6. Durability of Antimicrobial (AM) Activity, on Cotton, of Glucan C12 Quat Versus Other AM Amine Compounds^{a}**

| Example^{b} | Amine Compound | Special Exhaust Condition | Use Level | Initial AM Activity | AM Activity after 25 Real Time Washes^{c} |
|---|---|---|---|---|---|
| Example 1 | Glucan C12 Quat | None | 250 ppm | ~5 log₁₀ reduction | 5 log₁₀ reduction |
| Comparative 3 | Quat Silane | Hydrolysis for 1 hour | 2000 ppm | 5 log₁₀ reduction | <1 log₁₀ reduction |
| Comparative 4 | Chitosan | Low pH Exhaust | 500 ppm | 5 log₁₀ reduction | <1 log₁₀ reduction |
| Comparative 5 | LAE | None | 500 ppm | 5 log₁₀ reduction | <1 log₁₀ reduction |
| Comparative 6 | Derivatization Quat Amine QUAB 342 | None | 500 ppm | 5 log₁₀ reduction | <2 log₁₀ reduction |
| Comparative 7 | Dodecyl trimethylammonium chloride | None | 500 ppm | 5 log₁₀ reduction | <1 log₁₀ reduction |

| | | | | | |
|---|---|---|---|---|---|
| ^{a} Compounds applied to fabric according to Procedure 1. Antimicrobial activity tested according to Procedure 2A (ASTM-E3160-18). ^{b} See key above for more details regarding Examples and Comparatives. ^{c} Procedure 3 (ASTM-E3162-18). | | | | | |

Interestingly, cotton treated with Glucan C12 Quat retained substantially higher antimicrobial (AM) activity after washing compared to cotton treated with the QUAB 342 compound (3-chloro-2-hydroxypropyl dodecyldimethylammonium chloride) (Table 6). The main structural difference between these compounds is the presence of alpha-1,2-branched alpha-1,6-glucan in Glucan C12 Quat. It was also notable that cotton treated with Glucan C12 Quat retained substantially higher antimicrobial activity after washing as compared to cotton treated with another antimicrobial amine compound such as Quat Silane (3-[trihydroxysilyl]propyldimethyl-octadecyl ammonium chloride) or chitosan.

Glucan C12 Quat can be made cost effectively, can be easily formulated in an aqueous media, and does not exhaust or precipitate on fabric in excess of what is desired. Controlling excess exhaustion/precipitation is important for textile applications, as this can lead to a tailing effect (i.e., heterogenous surface application of active compound), which is undesirable. The alpha-glucan portion of Glucan C12 Quat is providing all of the aforementioned desired properties. As comparatives, other glucans such as starch and cellulose, when functionalized with dodecyldimethylammonium hydroxypropyl ether groups (and similar ether groups), are expected to be difficult to formulate in water and cause tailing when applied. Thus, the presently disclosed subject matter is advantaged. The disclosed subject matter is further advantaged by that its practice, which offers both high durability (e.g., low loss from washing treated fabric) and only needs a low input of the substituted ammonium to accomplish antimicrobial protection/odor control of fabric, results in only a low amount of substituted amine release to waste streams. The disclosed technology helps address some of the environmental concerns of using otherwise high concentrations of quaternary amine, such as leaching to waste water. For example, using 500 ppm of Glucan C12 Quat polymer (Mw 200 kDa, 20% alpha-1,2 branching) with DoS 0.2 (this concentration achieved activity on both cotton and synthetic fabrics) only released about 63 ppm of the substituted amine when fully leached to waste water. This release is much lower in comparison to what occurs when using a conventional C12 Quat such as dodecyltrimethylammonium chloride, which leaves a residual of 500 ppm (~8-times higher) of quaternary amine when fully leached.

**Table 7. Odor Control Performance of Glucan Quat Compounds on Natural and Synthetic Fabrics^{a}**

| Example^{b} | Glucan Quat Compound | Fabric Type | Use Level | Initial Odor Control (Milk Test Panel) | Odor Control after 50 (Cotton) or 25 (Nylon) Real Time Washes^{c} |
|---|---|---|---|---|---|
| Example 1 | Glucan C12 Quat | Cotton | 250 ppm | Pass | Pass |
| Example 2 | Glucan C12 Quat | Nylon | 500 ppm | Pass | Pass |
| Example 3 | Glucan C12 Quat | Polyester | 500 ppm | Pass | Pass |
| Example 4 | Glucan C8-C18 Quat | Cotton | 250 ppm | Pass | Pass |
| Comparative 1 | Glucan C1 Quat | Cotton | 250 ppm | Fail | Fail |
| Comparative 2 | Glucan C1 Quat | Nylon | 500 ppm | Fail | Fail |
| Comparative 8 | Glucan C1 Quat | Polyester | 500 ppm | Fail | Fail |

| | | | | | |
|---|---|---|---|---|---|
| ^{a} Compounds applied to fabric according to Procedure 1. Odor control performance tested according to Procedure 4, with the exception that a Pass rating was assigned for an odor rating of 1 or less (Fail rating assigned if odor rating greater than 1). ^{b} See key above for more details regarding Examples and Comparatives. ^{c} Procedure 3 (ASTM-E3162-18). | | | | | |

Interestingly, all three types of fabric tested (cotton, nylon, polyester), when treated with Glucan C12 Quat, exhibited odor control with ratings of less than 1 (Table 7).

**Table 8. Minimum Inhibitory Concentration of Glucan C12 Quat Versus Other Compounds^{a}**

| Microorganism | | Example/Comparative No.^{b} | | | | | |
|---|---|---|---|---|---|---|---|
| | | Comp. 9 | Ex. 5 | Comp. 10 | Comp. 11 | Comp. 12 | Comp. 13 |
| Species | ATCC No. | Non-Deriv. Glucan | Glucan C12 Quat | Glucan-C1 Quat | Dodecyl trimethyl ammonium chloride | Chitosan | LAE |
| *B. cepacia* | 25416 | >5000 ppm | 50 ppm | >5000 ppm | 1000 ppm | 5000 ppm | 100 ppm |
| *E. gergoviae* | 33028 | >5000 ppm | 50 ppm | >5000 ppm | 500 ppm | 5000 ppm | 100 ppm |
| *E. coli* | 8739 | >5000 ppm | 50 ppm | >5000 ppm | 200 ppm | >5000 ppm | 50 ppm |
| *K. pneumoniae* | 13883 | >5000 ppm | 50 ppm | >5000 ppm | 500 ppm | 5000 ppm | 50 ppm |
| *P. aeruginosa* | 9027 | >5000 ppm | 50 ppm | >5000 ppm | 500 ppm | >5000 ppm | 50 ppm |
| *P. aeruginosa* | 15442 | >5000 ppm | 50 ppm | >5000 ppm | 500 ppm | 5000 ppm | 50 ppm |
| *P. putida* | 49128 | >5000 ppm | 25 ppm | >5000 ppm | 500 ppm | 1000 ppm | 25 ppm |
| *S*. *aureus* | 6538 | >5000 ppm | 25 ppm | >5000 ppm | 200 ppm | 5000 ppm | 12.5 ppm |
| *S. epidermidis* | 12228 | >5000 ppm | 12.5 ppm | >5000 ppm | 100 ppm | >5000 ppm | 12.5 ppm |
| *C. albicans* | 10231 | >5000 ppm | 100 ppm | 200 ppm | 200 ppm | 100 ppm | 100 ppm |
| *A. brasiliensis* | 16404 | >5000 ppm | 100 ppm | >5000 ppm | 1000 ppm | >5000 ppm | 100 ppm |

| | | | | | | | |
|---|---|---|---|---|---|---|---|
| ^{a} The minimum inhibitory concentration of each tested compound was determined according to Procedure 5. ^{b} See key above for more details regarding Examples and Comparatives. | | | | | | | |

It is notable that Glucan C12 Quat exhibited low MIC levels against several species of bacteria (several of which are Gram-negative), as well as against two fungal species (Table 8).

**Table 9. Antimicrobial Performance of Glucan Quat Compounds on Cotton and Polyester Measured by AATCC 100 Method^{a}**

| Example^{b} | Fabric | Glucan Quat Compound | Antimicrobial Efficacy | |
|---|---|---|---|---|
| | | | *S*. *aureus* | *K. pneumoniae* |
| Example 1 | Cotton | Glucan C12 Quat | 4 log₁₀ reduction | 4 log₁₀ reduction |
| Example 3 | Polyester | Glucan C12 Quat | >3 log₁₀ reduction | >3 log₁₀ reduction |
| Example 4 | Cotton | Glucan C8-C18 Quat | >3 log₁₀ reduction | >3 log₁₀ reduction |
| Example 6 | Polyester | Glucan C8-C18 Quat | >4 log₁₀ reduction | >4 log₁₀ reduction |
| Comparative 1 | Cotton | Glucan C1 Quat | <1 log₁₀ reduction | <1 log₁₀ reduction |
| Comparative 8 | Polyester | Glucan C1 Quat | <1 log₁₀ reduction | <1 log₁₀ reduction |

| | | | | |
|---|---|---|---|---|
| ^{a} Compounds applied to fabric according to Procedure 1. AATCC 100 Method performed according to Procedure 2B. ^{b} See key above for more details regarding Examples and Comparatives. | | | | |

**Table 10. Rate of Kill Performance of Glucan C12 Quat Measured by Modified EPA Method MB-27-03 for Disinfection Efficacy^{a}**

| Example^{b} | Glucan C12 Quat Conc. | Contact Time | Disinfection Efficacy | |
|---|---|---|---|---|
| | | | *S*. *aureus* | *P. aeruginosa* |
| Example 7 | 100 ppm | 2.5 min. | ~5 log₁₀ reduction | Not measured |
| Example 8 | 100 ppm | 5 min. | Complete kill (~7 log₁₀ reduction) | Complete kill (~7 log₁₀ reduction) |
| Example 9 | 100 ppm | 10 min. | Complete kill (~7 log₁₀ reduction) | Complete kill (~7 log₁₀ reduction) |
| Example 10 | 1000 ppm | 2.5 min. | ~5 log₁₀ reduction | Not measured |
| Example 11 | 1000 ppm | 5 min. | Complete kill (~7 log₁₀ reduction) | Complete kill (~7 log₁₀ reduction) |
| Example 12 | 1000 ppm | 10 min. | Complete kill (~7 log₁₀ reduction) | Complete kill (~7 log₁₀ reduction) |

| | | | | |
|---|---|---|---|---|
| ^{a} Modified EPA Method MB-27-03 performed according to Procedure 6. ^{b} See key above for more details regarding Examples and Comparatives. | | | | |

**Table 11. Antimicrobial Performance on Cotton of Glucan C12 Quat Compounds Having Different Glucan Molecular Weights, Alpha-1,2 Branching Levels, and DoS Values^{a}**

| Example^{b} | Alpha-1,2-Branched Alpha-1,6-Glucan | | DoS | Antimicrobial Efficacy | |
|---|---|---|---|---|---|
| | Mw | % Alpha 1,2 Branching | | *S*. *aureus* | *P. aeruginosa* |
| Example 13 | 40 | 40 | 0.21 | >5 log₁₀ reduction | >5 log₁₀ reduction |
| Example 14 | 200 | 20 | 0.20 | >5 log₁₀ reduction | >5 log₁₀ reduction |
| Example 15 | 40 | 40 | 0.17 | ~5 log₁₀ reduction | ~5 log₁₀ reduction |
| Example 16 | 200 | 20 | 0.11 | >3 log₁₀ reduction | >3 log₁₀ reduction |

| | | | | | |
|---|---|---|---|---|---|
| ^{a} Compounds applied to fabric according to Procedure 1. Antimicrobial activity tested according to Procedure 2A (ASTM-E3160-18). Treated fabric was washed 25 times (real time washes), according to Procedure 3 before antimicrobial activity testing. ^{b} See key above for more details regarding Examples and Comparatives. | | | | | |

**Table 12. Antimicrobial Performance of Glucan C12 Quat Compounds on Cotton and Polyester when Delivered to Fabric through the Rinse of a Laundry Cycle^{a}**

| Example^{b} | Glucan C12 Quat (50 ppm) Delivery Vehicle in Rinse Cycle | Fabric | Log₁₀ Reduction Against: | |
|---|---|---|---|---|
| | | | *K. pneumonia* | *S*. *aureus* |
| Example 17 | Fabric Softener | Cotton | 4.4 | 4 |
| Example 18 | Fragrance Base | Cotton | 4.6 | 4 |
| Example 19 | Fabric Softener | Polyester | 4.3 | 4 |
| Example 20 | Fragrance Base | Polyester | 4.1 | 4 |

| | | | | |
|---|---|---|---|---|
| ^{a} Glucan C12 Quat compound was applied to fabric via laundry rinsing as performed in Procedure 7. Antimicrobial activity of the treated fabric was determined following the AATCC 100 method according to Procedure 2B. ^{b} See key above for more details regarding Examples and Comparatives. | | | | |

### Example 21

### Preventing Biofilm Formation with Cationic Alpha-1,6-Glucan Ether Derivative

Trends toward cold water washing and synthetic athletic wear are driving a need for detergents that eliminate bacteria and other microbes, while at the same time the industry is moving away from laundry powders in which traditional oxygen bleach was feasible. Thus, a need exists for new approaches to remove microorganisms in laundry.

Formation of bacterial biofilms in washing machines and on laundry textiles contributes to malodor through multiple routes such as by spreading harmful and malodorous bacteria, increasing resistance to the laundering process, and providing a reservoir to harbor odorants and substrates for microbial odor production (Bockmuhl, 2017, J. Appl. Microbiol. 122:1124-1133; Gattlen et al., 2010, Biofouling 26:873-882). A laundry detergent ingredient that inhibits the formation of biofilms could prevent the transfer of malodor-causing microorganisms and biofilms to clothes, keeping laundry fresher for a longer period of time.

In this Example, a Glucan Quat C12 was shown to prevent bacterial biofilm formation, thus providing a potential mode for reducing malodor in laundry applications.

### Methods

A biofilm dispersal assay was adapted from the procedure described by Pitts et al. (2003, J. Microbiol. Methods 54:269-276). To produce biofilms in 24-well plates, *Staphylococcus epidermidis* (ATCC 35984) was first grown overnight (18 hours) in a flask of TSB medium at 26 °C with 200 rpm agitation. The culture was then diluted to about 0.1 OD₆₀₀ units in TSB media. Diluted culture (1 mL or 1.1 mL) was added to each well of a 24-well polystyrene plate (Thermo-Fisher cat. no. 150687).

The test alpha-glucan/derivative compounds relevant to this study (irrelevant sample nos. not included) are listed in Table 13.

**Table 13. Alpha-Glucans and Alpha-Glucan Derivatives Tested for Activity Against Biofilm Formation**

| Sample No. | Sample Description | Derivative Functionality |
|---|---|---|
| 2 | Alpha-1,6-glucan, 17 kDa 20% alpha-1,2 branches | NA^{a} |
| 3 | Alpha-1,6-glucan, 40 kDa 20% alpha-1,2 branches | NA |
| 9 | Alpha-1,6-glucan | Benzyl, DoS 0.57 Carboxymethyl, DoS 0.34 |
| 10 | Alpha-1,6-glucan, 187 kDa 5% alpha-1,2 branches | Hydroxypropyl trimethylammonium, DoS 0.4 |
| 11 | Alpha-1,6-glucan, 200 kDa^{b} 20% alpha-1,2 branches | Dodecyldimethylammonium hydroxypropyl, DoS 0.07 |

| | | |
|---|---|---|
| ^{a} NA, not applicable. ^{b} This molecular weight was as measured following alpha-1,2-branching. | | |

Each compound was added to a culture well to a final concentration of 454 ppm, 250 ppm, 100 ppm, or 91 ppm. To achieve these concentrations, the compounds were dissolved at 50 mg/mL in 10% ethanol and then 10 µL, 5 µL, or 2 µL of diluted sample was individually added to each 1 mL or 1.1 mL culture well of the 24-well plate (n.b. sample 11 did not fully dissolve in the 10% ethanol solution). The plate was covered and then incubated for 48 hours at 30 °C without agitation. The liquid in each well was then removed and analyzed for planktonic cell density (OD₆₀₀) (FIG. 2), while the wells were washed three times with phosphate-buffered saline and then allowed to dry.

Biofilms in the wells were detected with crystal violet as follows. A 0.1% solution of crystal violet (1.5 mL) was dispensed into each well. The plates were gently agitated for two minutes, then incubated at room temperature for 30 more minutes. The crystal violet solution from each well was drawn off with a pipette, and the wells were rinsed three times with deionized water. The plate was allowed to dry and then photographed (FIG. 1). The stain that remained bound to biofilm in each well was then removed with a solution of 30% acetic acid (2 mL per well). The absorbance at 590 nm of the destain solution in each well was measured on a spectrophotometer, which provided additional output regarding the biofilm level in each well (data not shown).

### Results

Sample No. 11, a Glucan Quat C12, showed strong inhibition of biofilm formation by *S. epidermidis* (FIG. 1). Impressively, this effect was seen for all the dosage treatments, including the lowest dosage tested (91 ppm). The biofilm inhibition effect by Glucan Quat C12 might not have simply resulted from just killing cells, because the reduction in planktonic cell density observed for this compound (FIG. 2) did not seem to be commensurate with the corresponding biofilm reduction.

### Example 22

### Synergy of Cationic Alpha-1,6-Glucan Ether Derivative and Nuclease in Preventing Biofilm Formation

In this Example, a synergistic effect was observed by a combination of nuclease and Glucan Quat C12 in preventing of biofilm formation.

### Methods

A biofilm dispersal assay was adapted from the procedure described by Pitts et al. (*ibid.*)*.* To produce biofilms in 24-well plates, *S. epidermidis* (ATCC 35984) was first grown overnight (18 hours) in a flask of TSB medium at 26 °C with 200 rpm agitation. The culture was then diluted to about 0.1 OD₆₀₀ units in TSB media. Diluted culture (1 mL) was added to each well of a 24-well polystyrene plate (Thermo-Fisher cat. no. 150687).

The Glucan Quat C12 of Example 21 (Sample No. 11) was added to certain culture wells to a final concentration of 10 ppm, 20 ppm, 40 ppm, or 80 ppm. Nuclease (a *Bacillus cibi* endonuclease) was added to certain culture wells to a final concentration of 5 ppm, 10 ppm, or 20 ppm. After addition of nuclease and/or the alpha-glucan derivative to test wells, the 24-well plate was covered and then incubated for 43 hours at 30 °C without agitation. The liquid in each well was then removed and the wells were washed three times with phosphate-buffered saline, after which the plate was allowed to dry at room temperature.

Biofilms were then detected in each well following the methodology of Example 21. The stain that remained bound to biofilm in each well was then removed and quantitated as in Example 21 to provide additional output regarding the biofilm level in each well.

### Results

Overall, the combination of the nuclease and Glucan Quat C12 inhibited the formation of biofilms more effectively than would have been predicted based on their respective effects on biofilm formation. This synergistic effect was observed through different analyses.

First, as shown in FIG. 3, biofilm formation was substantially prevented by the combination of 10 ppm nuclease and 10 ppm Glucan Quat C12. In contrast, there was a smaller inhibitory effect on biofilm formation upon individual treatment with either 20 ppm nuclease or 20 ppm glucan.

Second, the difference in biofilm signal between samples incubated with 20 ppm Glucan Quat C12 and no glucan derivative was compared under two different conditions: with or without 5 ppm nuclease. The absorbance at 590 nm of destain solution for the sample treated with 20 ppm Glucan Quat C12 was subtracted from that measured for the sample treated with 0 ppm Glucan Quat C12. The difference indicated the amount of biofilm signal prevented by the presence of 20 ppm Glucan Quat C12. This value (difference) was determined for sample sets in the presence or absence of 5 ppm nuclease. The reduction in biofilm signal was more than 3-fold greater in the presence of 5 ppm nuclease than in the absence of nuclease.

Similarly, the difference in biofilm signal between samples incubated with 5 ppm nuclease and no nuclease was compared under two different conditions: with or without 20 ppm Glucan Quat C12. The effect of the 5 ppm nuclease treatment relative to no nuclease was more than 3-fold greater in the presence of 20 ppm Glucan Quat C12 than in the absence of Glucan Quat C12.

### Example 23

### Insoluble Alpha-Glucan with Alpha-1,3 Glycosidic Linkages Enhances the Durability of Cationic Alpha-1,6-Glucan Ether Deposition on Fabric

This Example demonstrates that including insoluble alpha-glucan with a high alpha-1,3 glycosidic linkage content with a cationic alpha-1,6-glucan ether as presently disclosed in fabric treatment can enhance the durability of the ether's deposition on the treated fabric in the face of multiple wash cycles. In particular, dodecyldimethylammonium hydroxypropyl alpha-1,2-branched alpha-1,6-glucan, when provided in a treatment composition with alpha-1,3-glucan (~100% alpha-1,3 glycosidic linkages, DPw ~800, aqueous insoluble), maintained high antimicrobial activity on polyester fabric treated with the composition, even after several washings of the fabric. This antimicrobial activity was higher than the antimicrobial activity observed when using a treatment composition having the cationic alpha-1,6-glucan ether, but not the insoluble alpha-1,3-glucan.

Dodecyldimethylammonium hydroxypropyl alpha-1,2-branched alpha-1,6-glucan (i.e., another form of "Glucan C12 Quat" herein) in this Example was prepared as follows. Cationic ether derivatization was done on alpha-1,6-glucan (100% alpha-1,6 linkages) having 20% alpha-1,2 branches; the total molecular weight of the alpha-1,2-branched alpha-1,6-glucan was 200 kDa. 3-chloro-2-hydroxypropyl-dodecyl-dimethylammonium chloride (QUAB 342) was ordered from QUAB Chemicals. All other ingredients were ordered from Sigma-Aldrich and used as received. The etherification reaction was performed in a 4-neck, 500-mL reactor equipped with a mechanical stir rod, thermocouple, and reflux condenser. Alpha-1,2-branched alpha-1,6-glucan (powder, 21 g) was added to the reactor, after which 90 mL of water was added; this preparation was stirred overnight to fully dissolve the glucan powder. The resulting solution was preheated to ~55 °C, and then 80 g of a QUAB 342 solution (32 g QUAB 342 in 48 g water) was added to the reactor. Aqueous sodium hydroxide (10 g of 50 wt% solution) was then added dropwise to the reactor over a 10-minute period. The reaction preparation was stirred at 64-66 °C for 3.5 hours, after which it was diluted with ~500 mL of water until the contents were fully dissolved and then cooled to 35 °C. Hydrochloric acid (18.5 wt%) was then added to neutralize the preparation to pH ~7. More water was then added to yield a final solution of 2 L, which was entered into an ultrafiltration using a 30 kDa molecular weight cutoff membrane filter. Sufficient filtration was reached when permeate had <200 µS conductivity, while the starting material (pre-filtration) had a conductivity of ~4 mS. The DoS of the Glucan C12 Quat product with dodecyldimethylammonium hydroxypropyl groups was determined by ¹H-NMR to be 0.20.

The Glucan C12 Quat product was then used alone or with insoluble alpha-1,3-glucan (~100% alpha-1,3 glycosidic linkages, DPw ~800) to treat polyester fabric to determine if including alpha-1,3-glucan has any effect on wash-durability of Glucan C12 Quat antimicrobial activity. The results of this analysis are provided in Table 14.

**Table 14. Antimicrobial Activity on Polyester Treated with Glucan C12 Quat and/or Alpha-1,3-Glucan**

| Test | Treatment^{a} Ingredient(s) | | Percent Reduction of Colony Formation^{b} | |
|---|---|---|---|---|
| | Glucan C12 Quat Bath Conc. | Alpha-1,3-Glucan Bath Conc. | Pre-Washing | After 25 Real Washes° |
| Example 23A | 1000 ppm | 500 ppm | >99.8 | >99.6 |
| Example 23B | 1000 ppm | 1000 ppm | >99.9 | >99.6 |
| Comparative A | 1000 ppm | none | >99.9 | >49.3 |
| Comparative B | none | 2000 ppm | 0 | 0 |

| | | | | |
|---|---|---|---|---|
| ^{a} Polyester fabric samples were treated according to Procedure 1B (padding method) with preparations (baths) containing Glucan C12 Quat (dissolved) and/or alpha-1,3-glucan (dispersed) in water. To prepare alpha-1,3-glucan alone or as blended with Glucan C12 Quat, non-preserved alpha-1,3-glucan wet cake (30 wt% solids) was dispersed in water using a homogenizer at 8000 rpm for 20 minutes to form a stock dispersion. For preparing blends, a suitable amount of Glucan C12 Quat was added to the stock dispersion to make a final preparation for fabric treatment. ^{b} Procedure 2B (AATCC 100 Method) was performed on the treated fabric samples using *Klebsiella pneumoniae* for calculating percent reduction of colony formation. ^{c} Wash durability testing was done using Procedure 3 (ASTM E3162-18). | | | | |

Results: The data in Table 14 indicate that insoluble alpha-1,3-glucan acts as a deposition aid to enhance the wash durability of Glucan C12 Quat on polyester fabric. Glucan C12 Quat (Comparative A) when used alone demonstrated good initial antimicrobial efficacy (i.e., % reduction of *K. pneumoniae* colony formation) at 1000 ppm treatment level (fabric treated, but not yet washed). However, its antimicrobial efficacy on the fabric was lower after 25 real washes. When the polyester fabric was treated with preparations containing both Glucan C12 Quat and insoluble alpha-1,3 glucan (Examples 23A-B), the wash durability of Glucan C12 Quat was improved dramatically, as evidenced by the treated fabrics having maintained high antimicrobial activity after 25 real washes. Polyester fabric treated with alpha-1,3-glucan only (Comparative B) did not exhibit any antimicrobial efficacy, either before or after washing the treated fabric. These results altogether indicate that the alpha-1,3-glucan performed as an aid to improve and maintain the deposition of Glucan C12 Quat on the polyester fabric.

## Claims

1. A composition comprising at least:
(a) a derivative of an alpha-glucan, wherein
(i) at least about 40% of the glycosidic linkages of the alpha-glucan are alpha-1,6 linkages, and
(ii) the alpha-glucan has a degree of substitution (DoS) of about 0.001 to about 3.0 with at least one positively charged organic group;
and
(b) an insoluble alpha-glucan, wherein at least about 50% of the glycosidic linkages of the insoluble alpha-glucan are alpha-1,3 glycosidic linkages, and the weight-average degree of polymerization (DPw) of the insoluble alpha-glucan is at least 10.

2. The composition of claim 1, wherein at least about 90% of the glycosidic linkages of the alpha-glucan of (a) are alpha-1,6 linkages.

3. The composition of claim 1 or 2, wherein the alpha-glucan of (a) comprises at least 1% alpha-1,2 and/or alpha-1,3 branches.

4. The composition of any one of claims 1 to 3, wherein the alpha-glucan of (a) has a weight-average molecular weight (Mw) of about 10 kDa to about 2000 kDa.

5. The composition of any one of claims 1 to 4, wherein said DoS is about 0.001 to about 1.0.

6. The composition of any one of claims 1 to 5, wherein the positively charged organic group is ether-linked to the alpha-glucan.

7. The composition of any one of claims 1 to 6, wherein the positively charged organic group comprises a C₄ to C₂₀ alkyl group or C₄ to C₂₀ alkylene group.

8. The composition of any one of claims 1 to 7, wherein the positively charged organic group comprises a substituted ammonium group.

9. The composition of any one of claims 1 to 8, wherein at least about 90% of the glycosidic linkages of the insoluble alpha-glucan are alpha-1,3 glycosidic linkages.

10. The composition of any one of claims 1 to 9, wherein the DPw of the insoluble alpha-glucan is at least about 200.

11. The composition of any one of claims 1 to 10, wherein the composition is an aqueous composition.

12. The composition of any one of claims 1 to 11, wherein the composition further comprises a fiber-containing material/article, and the alpha-glucan derivative and the insoluble alpha-glucan are adsorbed to the fiber-containing material/article or otherwise incorporated in the fiber-containing material/article.

13. The composition of claim 12, wherein the fiber-containing material/article is a fabric.

14. A method of treating a fiber-containing material/article, the method comprising:
(a) providing a composition according to any one of claims 1 to 11, and
(b) contacting the composition with a fiber-containing material/article, wherein at least the alpha-glucan derivative and the insoluble alpha-glucan are adsorbed to the fiber-containing material/article.

15. The method of claim 14, further comprising:
(c) subjecting the fiber-containing material/article, following step (b), to at least one cycle of washing and/or rinsing.

## Patentansprüche

1. Zusammensetzung, umfassend wenigstens:
(a) ein Derivat eines Alpha-Glucans, wobei
(i) es sich bei mindestens etwa 40 % der glycosidischen Bindungen des Alpha-Glucans um alpha-1,6-Bindungen handelt und
(ii) das Alpha-Glucan einen Grad der Substitution (Degree of Substitution, DoS) mit mindestens einer positiv geladenen organischen Gruppe von etwa 0,001 bis etwa 3,0 aufweist;
und
(b) ein unlösliches Alpha-Glucan, wobei es sich bei mindestens etwa 50 % der glycosidischen Bindungen des unlöslichen Alpha-Glucans um alpha-1,3-glycosidische Bindungen handelt und der gewichtsmittlere Polymerisationsgrad (weight-average Degree of Polymerization, DPw) des unlöslichen Alpha-Glucans mindestens 10 beträgt.

2. Zusammensetzung nach Anspruch 1, wobei es sich bei mindestens etwa 90 % der glycosidischen Bindungen des Alpha-Glucans unter (a) um alpha-1,6-Bindungen handelt.

3. Zusammensetzung nach Anspruch 1 oder 2, wobei das Alpha-Glucan unter (a) mindestens 1 % alpha-1,2- und/oder alpha-1,3-Verzweigungen umfasst.

4. Zusammensetzung nach einem der Ansprüche 1 bis 3, wobei das Alpha-Glucan unter (a) ein gewichtsmittleres Molekulargewicht (Mw) von etwa 10 kDa bis etwa 2000 kDa aufweist.

5. Zusammensetzung nach einem der Ansprüche 1 bis 4, wobei der DoS etwa 0,001 bis etwa 1,0 beträgt.

6. Zusammensetzung nach einem der Ansprüche 1 bis 5, wobei die positiv geladene organische Gruppe in Etherverknüpfung mit dem Alpha-Glucan vorliegt.

7. Zusammensetzung nach einem der Ansprüche 1 bis 6, wobei die positiv geladene organische Gruppe eine C₄- bis C₂₀-Alkylgruppe oder C₄- bis C₂₀-Alkylengruppe umfasst.

8. Zusammensetzung nach einem der Ansprüche 1 bis 7, wobei die positiv geladene organische Gruppe eine substituierte Ammoniumgruppe umfasst.

9. Zusammensetzung nach einem der Ansprüche 1 bis 8, wobei es sich bei mindestens etwa 90 % der glycosidischen Bindungen des unlöslichen Alpha-Glucans um alpha-1,3-glycosidische Bindungen handelt.

10. Zusammensetzung nach einem der Ansprüche 1 bis 9, wobei der DPw des unlöslichen Alpha-Glucans mindestens etwa 200 beträgt.

11. Zusammensetzung nach einem der Ansprüche 1 bis 10, wobei es sich bei der Zusammensetzung um eine wässrige Zusammensetzung handelt.

12. Zusammensetzung nach einem der Ansprüche 1 bis 11, wobei die Zusammensetzung ferner einen faserhaltigen Stoff/Gegenstand umfasst und das Alpha-Glucan-Derivat und das unlösliche Alpha-Glucan an den faserhaltigen Stoff/Gegenstand adsorbiert oder anderweitig in den faserhaltigen Stoff/Gegenstand eingebracht sind.

13. Zusammensetzung nach Anspruch 12, wobei es sich bei dem faserhaltigen Stoff/Gegenstand um ein Gewebe handelt.

14. Verfahren zur Behandlung eines faserhaltigen Stoffs/Gegenstands, wobei das Verfahren Folgendes umfasst:
(a) Bereitstellen einer Zusammensetzung nach einem der Ansprüche 1 bis 11 und
(b) Inkontaktbringen der Zusammensetzung mit einem faserhaltigen Stoff/Gegenstand, wobei wenigstens das Alpha-Glucan-Derivat und das unlösliche Alpha-Glucan an den faserhaltigen Stoff/Gegenstand adsorbiert werden.

15. Verfahren nach Anspruch 14, das ferner umfassend:
(c) Durchführen mindestens eines Wasch- und/oder einem Spülzyklus mit dem faserhaltigen Stoff/Gegenstand nach Schritt (b).

## Revendications

1. Composition comprenant au moins :
(a) un dérivé d'un alpha-glucane, dans lequel
(i) au moins environ 40 % des liaisons glycosidiques de l'alpha-glucane sont des liaisons alpha-1,6, et
(ii) l'alpha-glucane a un degré de substitution (DoS) d'environ 0,001 à environ 3,0 par au moins un groupe organique chargé positivement ;
et
(b) un alpha-glucane insoluble, dans lequel au moins environ 50 % des liaisons glycosidiques de l'alpha-glucane insoluble sont des liaisons glycosidiques alpha-1,3, et le degré de polymérisation moyen en poids (DPw) de l'alpha-glucane insoluble est d'au moins 10.

2. Composition selon la revendication 1, dans laquelle au moins environ 90 % des liaisons glycosidiques de l'alpha-glucane de (a) sont des liaisons alpha-1,6.

3. Composition selon la revendication 1 ou 2, dans laquelle l'alpha-glucane de (a) comprend au moins 1 % de ramifications alpha-1,2 et/ou alpha-1,3.

4. Composition selon l'une quelconque des revendications 1 à 3, dans laquelle l'alpha-glucane de (a) a un poids moléculaire moyen en poids (Mw) d'environ 10 kDa à environ 2 000 kDa.

5. Composition selon l'une quelconque des revendications 1 à 4, dans laquelle ledit DoS est d'environ 0,001 à environ 1,0.

6. Composition selon l'une quelconque des revendications 1 à 5, dans laquelle le groupe organique chargé positivement est lié par un éther à l'alpha-glucane.

7. Composition selon l'une quelconque des revendications 1 à 6, dans laquelle le groupe organique chargé positivement comprend un groupe alkyle en C₄ à C₂₀ ou un groupe alkylène en C₄ à C₂₀.

8. Composition selon l'une quelconque des revendications 1 à 7, dans laquelle le groupe organique chargé positivement comprend un groupe ammonium substitué.

9. Composition selon l'une quelconque des revendications 1 à 8, dans laquelle au moins environ 90 % des liaisons glycosidiques de l'alpha-glucane insoluble sont des liaisons glycosidiques alpha-1,3.

10. Composition selon l'une quelconque des revendications 1 à 9, dans laquelle le DPw de l'alpha-glucane insoluble est d'au moins environ 200.

11. Composition selon l'une quelconque des revendications 1 à 10, dans laquelle la composition est une composition aqueuse.

12. Composition selon l'une quelconque des revendications 1 à 11, dans laquelle la composition comprend en outre un matériau/article contenant des fibres, et le dérivé d'alpha-glucane et l'alpha-glucane insoluble sont adsorbés sur le matériau/article contenant des fibres ou autrement incorporés dans le matériau/article contenant des fibres.

13. Composition selon la revendication 12, dans laquelle le matériau/article contenant des fibres est un tissu.

14. Procédé de traitement d'un matériau/article contenant des fibres, le procédé comprenant :
(a) la fourniture d'une composition selon l'une quelconque des revendications 1 à 11, et
(b) la mise en contact de la composition avec un matériau/article contenant des fibres, au moins le dérivé d'alpha-glucane et l'alpha-glucane insoluble étant adsorbés sur le matériau/article contenant des fibres.

15. Procédé selon la revendication 14, comprenant en outre :
(c) la soumission du matériau/article contenant des fibres, suite à l'étape (b), à au moins un cycle de lavage et/ou rinçage.
